(19) Europäisches Patentamt
European Patent Office
Office européen des brevets

(11) Veröffentlichungsnummer: **0 016 900**
**B1**

(12) # EUROPÄISCHE PATENTSCHRIFT

(45) Veröffentlichungstag der Patentschrift: **16.11.83**

(21) Anmeldenummer: **80100097.7**

(22) Anmeldetag: **11.07.78**

(60) Veröffentlichungsnummer der früheren Anmeldung nach Art. 76 EPÜ: **0000500**

(51) Int. Cl.³: **C 07 C 127/19,**
C 07 C 125/065,
C 07 D 307/54,
C 07 D 333/24,
C 07 C 143/86,
C 07 C 143/58
//C07D501/00

(54) **Zwischenprodukte für die Herstellung von Cephalosporinderivaten, Verfahren zu ihrer Herstellung und Verwendung.**

(30) Priorität: **18.07.77 LU 777188**

(43) Veröffentlichungstag der Anmeldung: **15.10.80 Patentblatt 80/21**

(45) Bekanntmachung des Hinweises auf die Patenterteilung: **16.11.83 Patentblatt 83/46**

(84) Benannte Vertragsstaaten: **CH DE FR GB**

(56) Entgegenhaltungen: **DE - A - 2 259 512**

(73) Patentinhaber: **CIBA-GEIGY AG**
**Patentabteilung Postfach**
**CH-4002 Basel (CH)**

(72) Erfinder: **Kocsis, Karoly, Dr.**
**Am Chillweg 121**
**CH-4305 Olsberg (CH)**
Erfinder: **Schneider, Peter, Dr.**
**Rheinfelderstrasse 21 A/1**
**CH-4058 Basel (CH)**
Erfinder: **Fechtig, Bruno, Dr.**
**Hinterlindenweg 1**
**CH-4153 Reinach (CH)**
Erfinder: **Scartazzini, Riccardo, Dr.**
**Conrad Ferdinand Meyer-Strasse 38**
**CH-4059 Basel (CH)**

**EP 0 016 900 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Zwischenprodukte für die Herstellung von Cephalosporinderivaten und Verfahren zu ihrer Herstellung und Verwendung

Die Erfindung betrifft neue Carbonsäuren, geeignet zur Herstellung neuer Acylamido-3-cephem-4-carbonsäure-Verbindungen und ihrer Salze, und Verfahren zu ihrer Herstellung.

Es sind bereits zahlreiche 7ß-Acylamido-3-cephem-4-carbonsäure-Verbindungen bekannt geworden, die sich durch die Substituenten in 3-Stellung des 3-Cephemgerüstes, sowie durch die Acylgruppe an der 7ß-Aminogruppe unterscheiden. Uebersichten über solche Verbindungen, Verfahren zu ihrer Herstellung, sowie auch über ihre antibiotischen Aktivitäten wurden beispielsweise von Edwin H. Flynn, Cephalosporins and Penicillins, Academic Press, New York und London, 1972, J. Cs. Jászberény und T. E. Gunda, Progr. Med. Chem., Vol. 12, *1975*, S. 395—477, und Peter G. Sammes, Chemical Reviews, *1976*, Vol. 76, No. 1, S. 113—155, veröffentlicht.

Das Auftreten von neuen pathogenen Keimen, die gegen die bisher benutzten Antibiotika resistent geworden sind, sowie die bekannten Allergieerscheinungen, wecken das Bedürfnis nach neuen, aktiven Verbindungen, die die genannten Nachteile in geringerem Masse oder überhaupt nicht besitzen.

Der vorliegenden Erfindung liegt die Aufgabe zugrunde, neuartige Carbonsäuren, geeignet zur Herstellung neuer 7ß-Acylamino-3-cephem-4-carbonsäure-Verbindungen herzustellen. Die neuartigen Carbonsäuren sind durch das Vorhandensein einer endständigen $\alpha$-Aminocarbonsäuregruppierung charakterisiert. Die neuen, daraus herstellbaren Cephalosporinverbindungen zeichnen sich durch eine hervorragende Wirkung gegenüber normalen und resistenten Keimen aus.

Die als Ausgangsmaterialien zu benutzenden, neuartigen Carbonsäuren und deren reaktionsfähige funktionelle Derivate, worin funktionelle Gruppen gegebenenfalls geschützt sind, sowie Verfahren zu ihrer Herstellung, sind Gegenstand der vorliegenden Erfindung.

Die Erfindung betrifft Carbonsäure-Verbindungen der Formel

$$HOOC-\underset{\displaystyle NH_2}{\underset{|}{CH}}-(C_nH_{2n})-X-W-NH-(C_mH_{2m})-A-\overset{\displaystyle Y}{\overset{|}{\underset{\displaystyle Z}{\underset{|}{C}}}}-\overset{\displaystyle O}{\overset{\|}{C}}-OH \qquad (III),$$

worin der Index n eine ganze Zahl von 1 bis 4, der Index m 0 oder 1, X Sauerstoff, Schwefel oder die Gruppe —NH—, W eine Gruppe —CO—, —CO—$NHSO_2$— oder —$SO_2NH$—CO—, oder X—W zusammen eine Gruppe —CO— oder —CO—$NHSO_2$—, A gegebenenfalls substituiertes Phenylen, Thienylen oder Furylen, Y Wasserstoff, Hydroxyl, Formyloxy, Amino oder gegebenenfalls in Salzform vorliegendes Sulfo und Z Wasserstoff, oder Y und Z zusammen eine Oxogruppe oder eine Gruppe =N—O—$R^o$, worin $R^o$ Wasserstoff oder gegebenenfalls substituiertes Niederalkyl darstellt, und worin die Aminocarbonsäuregruppierung HOOC—$CH(NH_2)$— und gegebenenfalls in der Gruppierung —A—C(Y) (Z)— vorhandene weitere funktionelle in geschützter Form vorliegen, und ihre zur Acylierung geeigneten reaktionsfähigen Derivate, und Verfahren zu ihrer Herstellung.

Die neuartigen Carbonsäuren sind geeignet zur Herstellung von neuen Acylamido-3-cephem-4-carbonsäure-Verbindungen der Formel

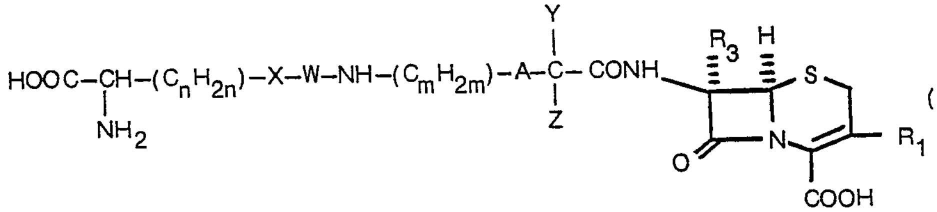

(I)

worin der Index n eine ganze Zahl von 1 bis 4, der Index m 0 oder 1, X Sauerstoff, Schwefel oder die Gruppe —NH—, W eine Gruppe —CO—, —CO—$NHSO_2$— oder —$SO_2NH$—CO—, oder X—W zusammen eine Gruppe —CO— oder —CO—$NHSO_2$—, A gegebenenfalls substituiertes Phenylen, Thienylen oder Furylen, Y Wasserstoff, Hydroxyl, Formyloxy, Amino oder gegebenenfalls in Salzform vorliegendes Sulfo und Z Wasserstoff, oder Y und Z zusammen eine Oxogruppe oder eine Gruppe =N—O—$R^o$, worin $R^o$ Wasserstoff oder gegebenenfalls substituiertes Niederalkyl darstellt, $R^1$ Wasserstoff, Niederalkyl, Niederalkoxy, Halogen oder eine Gruppe der Formel—$CH_2$—$R_2$, worin $R_2$ eine freie, veresterte oder verätherte Hydroxy- oder Mercaptogruppe oder eine quaternäre Ammoniumgruppe darstellt, und $R_3$ Wasserstoff oder Methoxy bedeuten, worin funktionelle Gruppen gegebenenfalls in geschützter Form vorliegen, und Salzen von solchen Verbindungen mit sauren und/oder basischen Gruppen.

In der vorliegenden Beschreibung der Erfindung bedeutet der Ausdruck "Nieder" in Gruppen wie Niederalkyl, Niederalkylen, Niederalkoxy, Niederalkanoyl und dergleichen, dass die entsprechenden

Gruppen, sofern nicht ausdrücklich anders definiert, bis zu 7, bevorzugt bis zu 4 C-Atome enthalten.

Eine Gruppe $—(C_nH_{2n})—$ ist eine verzweigte oder unverzweigte Alkylenkette, und ist insbesondere Methylen, 1,2-Aethylen, 1,3-Propylen oder 1,4-Butylen, ferner beispielsweise 1,1-Aethylen, 1,1-Propylen, 1,2-Propylen, 1,1-Butylen, oder 1,1,-Isobutylen.

Eine gegebenenfalls substituierte Phenylgruppe A ist insbesondere p-, kann aber auch o- oder m-Phenylen sein. Substituenten der Phenylengruppe sind beispielsweise Niederalkyl, wie Methyl, Hydroxy, Niederalkoxy, wie Methoxy, und/oder Halogen, wie Fluor, Chlor oder Brom.

Eine gegebenenfalls substituierte Thienylgruppe A ist insbesondere 2,5-Thienylen, ferner 2,4- oder 2,3-Thienylen.

Eine gegebenenfalls substituierte Furylengruppe A ist insbesondere 2,5-Furylen, ferner 2,4- oder 2,3-Furylen.

Substituenten der Thienylen- und Furylengruppe A sind beispielsweise Niederalkyl, wie Methyl, Niederalkoxy, wie Methoxy, und/oder Halogen, wie Fluor, Chlor oder Brom.

Wenn Z Wasserstoff bedeutet, ist Y bevorzugt Wasserstoff oder Hydroxy, oder auch Amino oder Sulfo.

In einer Gruppe $=N—O—R^o$ die bevorzugt in der Syn-Form vorliegt, enthält $R^o$ als gegebenenfalls substituiertes Niederalkyl 1—4 Kohlenstoffatome, Substituenten einer solchen Niederalkylgruppe $R^o$ sind beispielsweise Niederalkoxy, wie Methoxy, Halogen, wie Fluor, Chlor oder Brom, Hydroxy oder acyliertes Hydroxy, wie Niederalkanoyloxy, z.B. Acetoxy, Sulfo, und insbesondere freies oder auch verestertes Carboxy.

Repräsentative Gruppen $R^o$ sind beispielsweise Methyl, Aethyl, Propyl, Butyl, Methoxymethyl, Methoxyäthyl, wie 2-Methoxyäthyl, 3-Methoxypropyl, 4-Methoxybutyl, 2-Halogen-, wie 2-Chlor-äthyl, 3-Halogen-, wie 3-Chlorpropyl, oder 4-Halogen-, wie 4-Chlorbutyl, 2-Hydroxyäthyl, 3-Hydroxypropyl oder 4-Hydroxybutyl, worin die Hydroxygruppe beispielsweise durch Niederalkanoyl, wie Acetyl, acyliert sein kann, 2-Sulfoäthyl, 3-Sulfopropyl, 2-Carboxyäthyl, 3-Carboxypropyl oder 4-Carboxybutyl, worin die Carboxy gruppe beispielsweise durch Niederalkyl, wie Methyl oder Aethyl verestert sein kann.

Eine Niederalkylgruppe $R_1$ enthält 1—4 C-Atome und ist beispielsweise Aethyl, Propyl, Butyl oder insbesondere Methyl.

Eine Niederalkoxygruppe $R_1$ enthält 1—4 C-Atome und ist beispielsweise Methoxy, Aethoxy, Propoxy oder Butoxy.

$R_1$ als Halogen ist Fluor, Brom, Jod oder bevorzugt Chlor.

Eine veresterte Hydroxy- oder Mercaptogruppe $R_2$ ist durch eine niederaliphatische Carbonsäure oder durch eine gegebenenfalls N-substituierte Carbaminsäure verestert.

Mit niederaliphatischen Carbonsäuren veresterte Hydroxygruppen $R_2$ sind insbesondere Niederalkanoyloxy, insbesondere Acetyloxy, ferner Formyloxy, Propionyloxy, Valeryloxy, Hexanoyloxy, Heptanoyloxy oder Pivaloyloxy.

Mit niederaliphatischen Carbonsäuren veresterte Mercaptogruppen $R_2$ sind Niederalkanoylthio, wie Acetylthio, Formylthio, Propionylthio, Valeroylthio, Hexanoylthio, Heptanoylthio oder Pivaloylthio.

In einer durch eine gegebenenfalls N-substituierte Carbaminsäure veresterten Hydroxy- oder Mercaptogruppe $R_2$ sind N-Substituenten gegebenenfalls durch Halogen, z.B. Chlor, oder durch Niederalkanoyl, z.B. Acetyl oder Propionyl, substituiertes Niederalkyl, z.B. Methyl, Aethyl, 2-Chloräthyl, oder 2-Acetoxyäthyl. In dieser Art veresterte Hydroxygruppen $R_2$ sind z.B. Carbamoyloxy, N-Methylcarbamoyloxy, N-Aethylcarbamoyloxy, N-(2-Chloräthyl)-carbamoyloxy oder N-(2-Acetoxyäthyl)-carbamoyloxy. Entsprechende veresterte Mercaptogruppen $R_2$ sind z.B. Carbamoylthio, N-Methylcarbamoylthio, N-Aethylcarbamoylthio, N-(2-Chloräthyl)-carbamoylthio oder N-(2-Acetoxyäthyl)-carbamoylthio.

Verätherte Hydroxy- und Mercaptogruppen $R_2$ sind beispielsweise mit einem aliphatischen Kohlenwasserstoffrest veräthert, und sind insbesondere Niederalkoxy, insbesondere mit 1—4 C-Atomen, in erster Linie Methoxy, sowie Aethoxy, n-Propyloxy oder Isopropyloxy, ferner geradkettiges oder verzweigtes Butyloxy, oder Niederalkylthio, vorzugsweise mit 1—4 C-Atomen, in erster Linie Methylthio, sowie Aethylthio, n-Propylthio oder Isopropylthio, ferner geradkettiges oder verzweigtes Butylthio.

Verätherte Mercaptogruppen $R_2$ sind insbesondere durch einen gegebenenfalls substituierten, über eine Ringkohlenstoffatom an die Mercaptogruppe gebundenen, heterocyclischen Rest mit 1 bis 4 Ringstickstoffatomen und gegebenenfalls einem weiteren Ringheteroatom der Gruppe Sauerstoff und Schwefel veräthert.

Solche heterocyclische Reste sind in erster Linie gegebenenfalls substituierte, z.B. die unten genannten Substituenten enthaltende, monocyclische, fünfgliedrige diaza-, triaza-, tetraza-, thiaza-, thiadiaza-, thiatriaza, oxaza- oder oxadiazacyclische Reste aromatischen Charakters.

Substituenten solcher Heterocyclylreste sind u.a. Niederalkyl, insbesondere Methyl, sowie Aethyl, n-Propyl, Isopropyl oder geradkettiges oder verzweigtes Butyl, oder durch Hydroxy, verestertes Hydroxy, wie Niederalkanoyloxy, Halogen, wie Chlor, Carboxy, verestertes Carboxy, wie Niederalkoxycarbonyl, Sulfo, amidiertes Sulfo, Amino, Mono- oder Diniederalkylamino, Acylamino, wie Niederalkanoylamino, oder durch substituiertes, wie durch Carboxy oder Halogen substituiertes Niederalkanoylamino substituiertes Niederalkyl, beispielsweise 2-Hydroxyäthyl, 2-Acetoxyäthyl, 2-Chloräthyl, Carboxymethyl, 2-Carboxyäthyl, Aethoxycarbonylmethyl, 2-Aethoxycarbonyläthyl, Sulfomethyl, 2-Sulfoäthyl, Sulfamylmethyl, 2-Sulfamyläthyl, 2-Aminoäthyl, 2-Dimethylaminoäthyl oder 2-Acetylaminoäthyl. Weitere Substituenten des heterocyclischen Restes sind Cycloalkyl, z.B. Cyclopentyl oder Cyclohexyl, Aryl, wie gegebenenfalls durch Halogen, z.B. Chlor, oder Nitro substituiertes Phenyl, Arylniederalkyl, z.B. Benzyl, oder Heterocyclyl, wie Furyl, z.B. 2-Furyl, Thienyl, z.B. 2-Thienyl, oder Oxazolyl, z.B. 2- oder 5-Oxazolyl, oder funktionelle Gruppen, wie Halogen, z.B. Fluor, Chlor oder Brom, gegebenenfalls substituiertes Amino, wie gegebenenfalls durch Niederalkyl mono- oder di-substituiertes Amino, z.B. Amino, Methylamino oder Dimethylamino, Acylamino, wie Niederalkanoylamino oder durch Halogen oder Carboxy substituiertes Niederalkanoylamino, wie Acetylamino, 3-Chlorpropionylamino oder 3-Carboxypropionylamino, Nitro, Hydroxy, Niederalkoxy, z.B. Methoxy, Aethoxy, n-Butyloxy oder 2-Aethylhexyloxy, oder gegebenenfalls funktionell abgewandeltes Carboxy, wie Carboxy, verestertes Carboxy, wie Niederalkoxycarbonyl, z.B. Methoxycarbonyl oder Aethoxycarbonyl, gegebenenfalls substituiertes, wie N-mono- oder N,N-diniederalkyliertes Carbamoyl, z.B. N-Methylcarbamoyl oder N,N-Dimethylcarbamoyl, oder Cyan, sowie Oxo oder Oxido, wobei einer oder mehrere solche Substituenten, die in erster Linie mit Ringkohlenstoffatomen aber auch, insbesondere Niederalkyl und Oxido, mit Ringstickstoffatomen verbunden sind, vorhanden sein können.

Bevorzugte heterocyclisch verätherte Mercaptogruppen $R_2$, worin der heterocyclische Rest einen entsprechenden monocyclischen, fünfgliedrigen Rest darstellt, sind u.a. Imidazolylthio, z.B. 2-Imidazolylthio, gegebenenfalls durch Niederalkyl und/oder Phenyl substituiertes Triazolylthio, z.B. 1H-1,2,3-Triazol-4-ylthio, 1-Methyl-1H-1,2,3-triazol-4-ylthio, 1H-1,2,4-Triazol-3-ylthio, 5-Methyl-1H-1,2,4-triazol-3-ylthio, 3-Methyl-1-phenyl-1H-1,2,4-triazol-5-ylthio, 4,5-Dimethyl-4H-1,2,4-triazol-3-ylthio oder 4-Phenyl-4H-1,2,4-triazol-3-ylthio, insbesondere gegebenenfalls wie angegeben substituiertes Tetrazolylthio, z.B. 1H-Tetrazol-5-ylthio, 1-Methyl-1H-tetrazol-5-ylthio, 1-Carboxymethyl-1H-tetrazol-5-ylthio, 1-(2-Carboxyäthyl)-1H-tetrazol-5-ylthio, 1-Sulfomethyl-1H-tetrazol-5-ylthio, 1-(2-Sulfoäthyl)-1H-tetrazol-5-ylthio, 1-(2-Dimethylaminoäthyl)-1H-tetrazol-5-ylthio, 1-Phenyl-1H-tetrazol-5-ylthio oder 1-(4-Chlorphenyl)-1H-tetrazol-5-ylthio, gegebenenfalls durch Niederalkyl oder Thienyl substituiertes Thiazolylthio oder Isothiazolylthio, z.B. 2-Thiazolylthio, 4-(2-Thienyl)-2-thiazolylthio, 4,5-Dimethyl-2-thiazolylthio, 3-Isothiazolylthio, 4-Isothiazolylthio oder 5-Isothiazolylthio, insbesondere auch gegebenenfalls wie angegeben substituiertes Thiadiazolylthio, z.B. 1,2,3-Thiadiazol-4-ylthio, 1,2,3-Thiadiazol-5-ylthio, 1,3,4-Thiadiazol-2-ylthio, 2-Methyl-1,3,4-thiadiazol-5-ylthio, 2-(3-Carboxypropionylamino)-1,3,4-thiadiazol-5-ylthio, 1,2,4-Thiadiazol-5-ylthio oder 1,2,5-Thiadiazol-3-ylthio, Thiatriazolylthio, z.B. 1,2,3,4-Thiatriazolyl-5-ylthio, gegebenenfalls wie angegeben substituiertes Oxazolylthio oder Isoxazolylthio, z.B. 5-Oxazolylthio, 4-Methyl-5-oxazolylthio, 2-Oxazolylthio, 4,5-Diphenyl-2-oxazolylthio oder 3-Methyl-5-isoxazolylthio, oder gegebenenfalls wie angegeben substituiertes Oxadiazolylthio, z.B. 1,2,4-Oxadiazol-5-ylthio, 2-Methyl-1,3,4-oxadiazol-5-ylthio, 2-Phenyl-1,3,4-oxadiazol-5-ylthio, 5-(4-Nitrophenyl)-1,3,4-oxadiazol-2-ylthio oder 2-(2-Thienyl)-1,3,4-oxadiazol-5-ylthio.

Bevorzugte heterocyclisch verätherte Mercaptogruppen $R_2$, worin der heterocyclische Rest einen entsprechenden monocyclischen, sechsgliedrigen Rest oder einen entsprechenden partiell gesättigten Rest darstellt, sind u.a. gegebenenfalls durch Halogen substituiertes 1-Oxido-pyridylthio, z.B. 1-Oxido-2-pyridylthio oder 4-Chlor-1-oxido-2-pyridylthio, gegebenenfalls durch Hydroxy substituiertes Pyridazinylthio, z.B. 3-Hydroxy-6-pyridazinylthio, gegebenenfalls durch Niederalkyl, Niederalkoxy oder Halogen substituiertes N-Oxido-pyridazinylthio, z.B. 2-Oxido-6-pyridazinylthio, 3-Chlor-1-oxido-6-pyridazinylthio, 3-Methyl-2-oxido-6-pyridazinylthio, 3-Methoxy-1-oxido-6-pyridazinylthio, 3-Aethoxy-1-oxido-6-pyridazinylthio, 3-n-Butyloxy-1-oxido-6-pyridazinylthio oder 3-(2-Aethylhexyloxy)-1-oxido-6-pyridazinylthio, oder gegebenenfalls durch Niederalkyl, Amino, Diniederalkylamino oder Carboxy substituiertes 2-Oxo-1,2-dihydro-pyrimidinylthio, z.B. 2-Oxo-1,2-dihydro-4-pyrimidinylthio, 6-Methyl-2-oxo-1,2-dihydro-4-pyrimidinylthio, 5-Methyl-2-oxo-1,2-dihydro-4-pyrimidinylthio, 6-Amino-2-oxo-1,2-dihydro-4-pyrimidinylthio, 6-Dimethylamino-2-oxo-1,2-dihydro-4-pyrimidinylthio, 5-Carboxy-2-oxo-1,2-dihydro-4-pyrimidinylthio oder 6-Carboxy-2-oxo-1,2-dihydro-4-pyrimidinylthio.

Quaternäre Ammoniumgruppen $R_2$ sind von tertiären organischen Basen, vorzugsweise von entsprechenden aliphatischen Aminen oder in erster Linie von entsprechenden heterocyclischen Stickstoffbasen abgeleitete, über das Stickstoffatom mit dem Methylkohlenstoffatom verbundene, quaternäre Ammoniumgruppen.

In einer quaternären Ammoniumgruppe $R_2$, die von einer tertiären organischen Base abgeleitet wird, ist das Stickstoffatom an das Methylkohlenstoffatom gebunden und liegt demgemäss in quaternisierter, positiv geladener Form vor. Quaternäre Ammoniumgruppen sind u.a. Triniederalkylammonium, z.B. Trimethylammonium, Triäthylammonium, Tripropylammonium oder Tributylammonium, insbesondere aber gegebenenfalls substituierte, z.B. Niederalkyl, wie Methyl, Hydroxyniederalkyl, wie Hydroxymethyl, Amino, substitiuertes Sulfonamido, wie 4-Aminophenylsulfonamido, Hydroxy, Halogen, wie Fluor, Chlor, Brom oder Jod, Halogenniederalkyl, wie Trifluormethyl, Sulfo, gegebenenfalls funktionell abgewandeltes Carboxy, wie Carboxy, Niederalkoxycarbonyl, z.B. Methoxycarbonyl, Cyan, gegebenenfalls durch Niederalkyl, z.B. Methyl oder Aethyl, oder Hydroxyniederalkyl, z.B. Hydroxymethyl, N-mono- oder N,N-disubstituiertes Carbamoyl, z.B. Carbamoyl, N-Methylcarbamoyl oder N,N-Dimethyl-carbamoyl, gegebenenfalls durch Niederalkyl N-substituiertes Hydrazinocarbonyl,

z.B. Hydrazinocarbonyl, Carboxyniederalkyl, wie Carboxymethyl, Niederalkanoyl, wie Acetyl, oder 1-Niederalkyl-pyrrolidinyl, wie 1-Methyl-2-pyrrolidinyl, mono- oder polysubstituierte, monocyclische oder bicyclische azacyclische Ammoniumgruppen aromatischen Charakters, mit 1 oder 2 Ringstickstoff- und gegebenenfalls einem Ringschwefelatom, wie Pyrimidinium, Pyridazinium, Thiazolium, Chinolinium und in erster Linie Pyridinium.

Heterocyclische Ammoniumgruppen $R_2$ sind in erster Linie gegebenenfalls Niederalkyl, Hydroxyniederalkyl, Amino, substituiertes Sulfonamido, Hydroxy, Halogen, Trifluormethyl, Sulfo, Carboxy, Niederalkoxycarbonyl, Cyan, Niederalkanoyl, 1-Niederalkyl-pyrrolidinyl oder gegebenenfalls durch Niederalkyl oder Hydroxyniederalkyl N-substituiertes Carbamoyl enthaltendes Pyridinium, z.B. Pyridinium, 2-, 3- oder 4-Methyl-pyridinium, 3,5-Dimethyl-pyridinium, 2,4,6-Trimethylpyridinium, 2-, 3- oder 4-Aethyl-pyridinium, 2-, 3- oder 4-Propyl-pyridinium oder insbesondere 4-Hydroxymethylpyridinium, ferner 2-Amino- oder 2-Amino-6-methyl-pyridinium, 2-(4-Aminophenylsulfonylamido)-pyridinium, 3-Hydroxy-pyridinium, 3-Fluor-, 3-Chlor-, 3-Jod- oder insbesondere 3-Brom-pyridinium, 4-Trifluormethyl-pyridinium, 3-Sulfopyridinium, 2-, 3- oder 4-Carboxy- oder 2,3- oder 3,4-Dicarboxypyridinium, 4-Methoxycarbonyl-pyridinium, 3- oder 4-Cyan-pyridinium, 3-Carboxymethyl-pyridinium, 3- oder 4-Acetyl-pyridinium, 3-(1-Methyl-2-pyrrolidinyl)-pyridinium, und insbesondere 4-Carbamoyl-, 3-Carbamoyl-, 3,4-Dicarbamoyl-, 3- oder 4-N-Methylcarbamoyl-, 4-N,N-Dimethylcarbamoyl-, 4-N-Aethylcarbamoyl-, 3-N,N-Diäthylcarbamoyl, 4-N-Propylcarbamoyl-, 4-Isopropylcarbamoyl- und 4-Hydroxymethyl-carbamoyl-pyridinium.

Die in Verbindungen der Formeln I und III vorhandenen funktionellen Gruppen, insbesondere Carboxyl- und Amino-, ferner Hydroxy- und Sulfogruppen, sind gegebenenfalls durch Schutzgruppen geschützt, die in der Penicillin-, Cephalosporin- und Peptidchemie verwendet werden.

Solche Schutzgruppen sind leicht, das heisst ohne dass unerwünschte Nebenreaktionen stattfinden, beispielsweise solvolytisch, reduktive, photolytisch oder auch unter physiologischen Bedingungen, abspaltbar.

Schutzgruppen dieser Art, sowie ihre Abspaltung sind beispielsweise beschrieben in "Protective Groups in Organic Chemistry", Plenum Press, London, New York, 1973, ferner in 'The Peptides", Vol. I, Schröder and Lübke, Academic Press, London, New York, 1965.

So sind Carboxylgruppen z.B. üblicherweise in veresterter Form geschützt, wobei solche Estergruppierungen unter schonenden Bedingungen leicht spaltbar sind. Als geeignete geschützte Carboxylgruppen kommen insbesondere Niederalkoxycarbonyl, insbesondere tert.-Niederalkoxycarbonyl, z.B. tert.Butyloxycarbonyl, Polycycloalkoxycarbonyl, z.B. Adamantyloxycarbonyl, Arylmethoxycarbonyl, worin Aryl vorzugsweise einem oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono-oder polysubstituierte Phenylreste darstellt, wie gegebenenfalls, z.B. wie oben erwähnt substituiertes Benzyloxycarbonyl, z.B. 4-Nitro-benzoyloxycarbonyl, oder 4-Methoxybenzyloxycarbonyl, oder z.B. wie oben erwähnt substituiertes Diphenylmethoxycarbonyl, z.B. Benzhydryloxycarbonyl oder Di-(4-methoxyphenyl)-methoxycarbonyl, oder 2-Halogenniederalkoxycarbonyl, z.B. 2,2,2-Trichloräthoxycarbonyl, insbesondere Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, oder Polyhalogenaryloxycarbonyl, wie Pentachlorphenyloxycarbonyl, in Frage. Veresterte Carboxylgruppen sind ebenfalls entsprechende Silyloxycarbonyl-, insbesondere organische Silyloxycarbonylgruppen oder entsprechende Stannyloxycarbonylgruppen. In diesen enthält das Silicium- bzw. Zinnatom vorzugsweise Niederalkyl, insbesondere Methyl, ferner Niederalkoxy, z.B. Methoxy, und/oder Halogen, z.B. Chlor, als Substituenten. Geeignete Silyl- bzw. Stannylschutzgruppen sind erster Linie Triniederalkylsilyl, insbesondere Trimethylsilyl, ferner Dimethy-tert.-butyl-silyl, Niederalkoxy-niederalkylhalogen-silyl, z.B. Methoxy-methyl-chlorsilyl, oder Diniederalkyl-halogen-silyl, z.B. Dimethylchlor-silyl, oder entsprechend substituierte Stannylverbindungen, z.B. Tri-n-butylstannyl.

Bevorzugt als geschützte Carboxylgruppe ist insbesondere tert.-Butyloxycarbonyl, gegebenenfalls, z.B. wie erwähnt substituiertes Benzyloxycarbonyl, z.B. 4-Nitrobenzyloxycarbonyl, oder Diphenylmethoxycarbonyl, z.B. Benzhydryloxycarbonyl.

Eine unter physiologischen Bedingungen spaltbare, veresterte Carboxylgruppe ist in erster Linie eine Acyloxymethoxycarbonylgruppe, worin Acyl z.B. den Rest einer organischen Carbonsäure, in erster Linie einer gegebenenfalls substituierten Niederalkancarbonsäure bedeutet, oder worin Acyloxymethyl den Rest eines Lactons bildet. Solche Gruppen sind Niederalkanoyloxymethoxycarbonyl, z.B. Acetyloxymethylcarbonyl oder Pivaloyloxymethoxycarbonyl, Aminoniederalkanoyloxymethoxycarbonyl, insbesondere $\alpha$-Amino-niederalkanoyloxymethoxycarbonyl, z.B. Glycyloxymethoxycarbonyl, L-Valyloxymethoxycarbonyl oder L-Leucyloxymethoxycarbonyl, ferner Phthalidyloxycarbonyl, z.B. 2-Phthalidyloxycarbonyl, oder Indanyloxycarbonyl, z.B. 5-Indanyloxycarbonyl.

Eine geschützte Aminogruppe kann z.B. in Form einer leicht spaltbaren Acylamino, Triarylmethylamino-, verätherten Mercaptoamino-, 1-Acyl-2-niederalkylidenamino-, Silyl- oder Stannylaminogruppe oder als Azidogruppe vorliegen.

In einer entsprechenden Acylaminogruppe ist Acyl vorzugsweise der Acylrest eines Kohlensäurehalbesters, wie Niederalkoxycarbonyl, insbesondere tert.-Niederalkoxycarbonyl, z.B. tert.-Butyloxycarbonyl, Polycycloalkoxycarbonyl, z.B. Adamantyloxycarbonyl, Arylmethoxycarbonyl, worin Aryl

vorzugsweise einen oder zwei, gegebenenfalls, z.B. durch Niederalkyl, insbesondere tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellt wei gegebenenfalls, z.B. wie oben erwähnt, substituiertes Benzyloxycarbonyl, z.B. 4-Nitro-benzyloxycarbonyl, oder z.B. wie oben erwähnt substituiertes Diphenylmethoxycarbonyl, z.B. Benzhydryloxycarbonyl oder Di-(4-methoxyphenyl)-methoxycarbonyl, oder 2-Halogen-niederalkoxycarbonyl, z.B. 2,2,2-Trichloräthoxycarbonyl, 2-Bromäthoxycarbonyl oder 2-Iodäthoxycarbonyl, oder Acylmethoxycarbonyl, insbesondere Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl. Acyl in einer Acylaminogruppe kann auch den entsprechenden Rest einer organischen Sulfonsäure darstellen; ein solcher Rest ist insbesondere Arylsulfonyl, worin Aryl einen gegebenenfalls, z.B. durch Niederalkyl, wie Methyl, Halogen, wie Brom, oder Nitro, substituierten Phenylrest bedeutet, z.B. 4-Methylphenylsulfonyl.

In einer Triarylmethylaminogruppe sind die Arylrest insbesondere gegebenenfalls substituierte Phenylreste; eine entsprechende Gruppe ist in erster Linie Trityl.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbären 1-Acyl-2-niederalkylidenrest ist Acyl vorzugsweise der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure oder eines Kohlensäurehalbesters, wie eines Kohlensäure-niederalkylhalbesters. Entsprechende Schutzgruppen sind in erster Linie 1-Niederalkanoyl-2-propyliden, z.B. 1-Acetyl-2-propyliden, oder 1-Niederalkoxycarbonyl-2-propyliden, z.B. 1-Aethoxycarbonyl-2-propyliden.

Eine Silyl- oder Stannylaminogruppe ist in erster Linie eine organische Silyl- bzw. Stannylaminogruppe, worin das Silicium- bzw. Zinnatom vorzugsweise Niederalkyl, insbesondere Methyl, ferner Niederalkoxy, z.B. Methoxy, und/oder Halogen, z.B. Chlor, als Substituenten enthält. Entsprechende Silyl- oder Stannylgruppen sind in erster Linie Triniederalkylsilyl, insbesondere Trimethylsilyl, ferner Dimethyl-tert-.-butyl-silyl, Niederalkoxy-niederalkyl-halogen-silyl, z.B. Methoxy-methyl-chlor-silyl, oder Diniederalkyl-halogen-silyl, z.B. Dimethyl-chlor-silyl, oder entsprechend substituiertes Stannyl, z.B. Tri-n-butylstannyl.

Eine Aminogruppe kann auch in protonierter Form geschützt werden; als Anionen kommen in erster Linie diejenigen von starken anorganischen Säuren, wie von Halogenwasserstoffsäuren, z.B. das Chlor- oder Bromanion, oder von Sulfonsäure, wie p-Toluolsulfonsäure, in Frage.

Bevorzugt als Aminoschutzgruppen sind die Acylreste von Kohlensäurehalbestern, insbesondere tert.-Niederalkoxycarbonyl, gegebenenfalls, z.B. wie angegeben substituiertes Benzyloxycarbonyl oder Diphenylmethoxycarbonyl, oder 2-Halogen-niederalkoxycarbonyl, wie 2,2,2-Trichloräthoxycarbonyl.

Hydroxyschutzgruppen sind z.B. Acylreste, wie 2,2-Dichloracetyl oder insbesondere einer der im Zusammenhang mit einer geschützten Aminogruppe genannten Acylreste von Kohlensäurehalbestern, insbesondere 2,2,2-Trichloräthoxycarbonyl, oder organischen Silyl- oder Stannylrest, ferner leicht abspaltbare 2-oxa- oder 2-thia-aliphatische oder cycloaliphatische Kohlenwasserstoffreste, in erster Linie 1-Niederalkoxy-niederalkyl oder 1-Niederalkylthio-niederalk, z.B. 1-Methoxyäthyl, 1-Aethoxyäthyl, 1-Methylthio-äthyl oder 1-Aethylthioäthyl, oder 2-Oxa oder 2-Thiacycloniederalkyl mit 5—7 Ringatomen, z.B. 2-Tetrahydrofuryl oder 2-Tetrahydropyranyl oder entsprechende Thiaanaloge, sowie leicht abspaltbare, gegebenenfalls substituierte $\alpha$-Phenylniederalkylreste, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Eine geschützte Sulfogruppe ist in erster Linie eine mit einem aliphatischen, cycloaliphatischen, cycloaliphatisch aliphatischen, aromatischen oder araliphatischen Alkohol, wie einem Niederalkanol oder mit einem Silyl- oder Stannylrest, wie Triniederalkylsilyl, veresterte Sulfogruppe. In einer Sulfogruppe kann die Hydroxygruppe beispielsweise wie die Hydroxygruppe in einer veresterten Carboxygruppe veräthert sein.

Salze sind insbesondere diejenigen von Verbindungen der Formel I mit einer freien Carboxylgruppe, in erster Linie Metall- oder Ammoniumsalze, wie Alkalimetall-und Erdalkalimetall-, z.B. Natrium-, Kalium, Magnesium- oder Calciumsalze, sowie Ammoniumsalze mit Ammoniak oder geeigneten organischen Aminen, wobei in erster Linie aliphatische, cycloalphatische, cycloaliphatischaliphatische oder araliphatische primäre, sekundäre oder tertiäre Mono-, Di- oder Polyamine, sowie heterocyclische Basen für die Salz bildung in Frage kommen, wie Niederalkylamine, z.b. Triäthylamin, Hydroxyniederalkylamine, z.B. 2-Hydroxyäthylamin, Bis-(2-hydroxyäthyl)-amin oder Tris-(2-hydroxyäthyl)-amin, basische aliphatische Ester von Carbonsäuren, z.B. 4-Aminobenzoesäure-2-diäthylaminoäthylester, Niederalkylenamine, z.B. 1-Aethyl-piperidin, Cycloalkylamine, z.B. Dicyclohexylamin, oder Benzylamine, z.B. N,N'-Dibenzyl-äthylendiamin, ferner Basen vom Pyridintyp, z.B. Pyridin, Collidin oder Chinolin. Verbindungen der Formel I mit einer basischen Gruppe können Säureadditionssalze, z.B. mit anorganischen Säuren, wie Salzsäure, Schwefelsäure, oder Phosphorsäure, oder mit geeigneten organischen Carbon- oder Sulfonsäuren, z.B. Trifluoressigsäure, sowie mit Aminosäuren, wie Arginin und Lysin bilden. Verbindungen der Formel I mit einer freien Carboxylgruppe und freien Aminogruppe können auch in Form von inneren Salzen, d.h. in zwitterionischer Form vorliegen.

Die neuen Carbonsäuren der Formel III enthalten ein oder gegebenenfalls zwei Asymmetriezentren. Das gegebenenfalls vorhandene Asymmetriezentrum nächst zur Carbonylgruppe, nämlich wenn Y Hydroxyl, Amino oder Sulfo und Z Wasserstoff bedeuten, liegt in der R,S- oder bevorzugt in der R-Konfiguration vor. Das Asymmetriezentrum an der endständigen Aminocarbonsäuregruppierung kann die R-, S- oder R,S-Konfiguration besitzen.

Die Verbindungen der Formel I, worin Carboxylgruppen gegebenenfalls in physioligisch spaltbarer Form verestert sind, und ihre pharmazeutisch verwendbaren, nichttoxischen Salze sind wertvolle, antibiotisch wirksame Substanzen, die insbesondere als antibakterielle Antibiotika verwendet werden können. Beispielsweise sind sie *in vitro* gegen gram-positive und gram-negative Mikroorganismen, wie gegen Kokken, inklusive *Niesseria* Arten, und Anaerobier in Minimalkonzentrationen von etwa 0,02 mcg/ml und gegen Enterobacteriaceae in Minimalkonzentrationen von etwa 0,25 mcg/ml, wirksame. *In vivo*, bei subcutaner Application an der Maus, sind sie beispielsweise gegen grampositive Kokken, wie *Staphylococcus aureus* (in Minimaldosen von etwa 3 mg/kg) und *Streptococcus pneumoniae* (in Minimaldosen von etwa 0,15 mg/kg), gegen Enterobakterien, wie *Escherichia coli* (in Minimaldosen von etwa 8 mg/kg), *Klebsiella pneumoniae* und *Proteus mirabilis* (in Minimaldosen von etwa 0,3 mg/kg), und gegen andere gramnegative Bakterien, wie *Pasteurella multocida* (in Minimaldosen von 0,1 mg/kg), wirksam. Die neuen Verbindungen können deshalb entsprechend, z.B. in Form von antibiotisch wirksamen Präparaten zur Behandlung von durch gram-positive oder gram-negative Bakterien und Kokken, insbesondere durch von Enterobakterien, wie *Escherichia coli, Klebsiella pneumoniae* und Proteus mirabilis, verursachten Infektionen Verwendung finden.

Verbindungen der Formel I, worin die funktionellen Gruppen geschützt sind werden als Ausgangsmaterialien zur Herstellung von Verbindungen der Formel I verwendet.

Die vorliegende Erfindung betrifft in erster Linie diejenigen Verbindungen der Formel III, worin die Gruppe $-(C_nH_{2n})-$ unverzweigt ist und die. Indices n und m die angegebene Bedeutung haben, X Sauerstoff oder die Gruppe $-NH-$, W eine Gruppe $-CO-$ oder $-CO-NHSO_2-$, oder X—W zusammen eine Gruppe $-CO-$ oder $-CO-NHSO_2-$ bedeuten, A p- oder m-Phenylen, 2,5-Thienylen oder 2,5-Furylen darstellt, Y Wasserstoff, Hydroxyl, Amino oder Sulfo und Z Wasserstoff, oder Y und Z Wasserstoff, oder Y und Z zusammen eine Gruppe $=N-O-R^o$ bedeuten, worin $R^o$ Wasserstoff oder Methyl ist, sowie die geschützten und reaktionsfähigen Derivate davon.

Besonders hervorzuheben sind Verbindungen der Formel III, worin die Gruppe $-(C_nH_{2n})-$ unverzweigt ist und die Indices n und m die angegebene Bedeutung haben, X Sauerstoff oder die Gruppe $-NH-$ bedeutet, W eine Gruppe $-CO-$ oder $-CONHSO_2-$, oder X—W zusammen eine Gruppe $-CO-$ oder $-CONHSO_2-$ bedeuten, A p- oder m-Phenylen, oder, wenn m 1 ist, auch 2,5-Thienylen oder 2,5-Furylen darstellt, Y Wasserstoffoff, Hydroxyl, Amino oder Sulfo und Z Wasserstoff, oder Y und Z zusammen eine Gruppe $=N-O-R^o$ bedeuten, worin $R^o$ Wasserstoff oder Methyl ist, sowie die geschützten und reaktionsfähigen Derivate davon.

Die Erfindung betrifft insbesondere die in den Beispielen beschriebenen Verbindungen der Formel III, bzw. deren geschützte und reaktionsfähige Dervate.

Die Verbindungen der vorliegenden Erfindung werden nach an sich bekannten Verfahren erhalten.

Verbindungen der Formel I, worin die Carboxylgruppen gegebenenfalls in physiologisch spaltbarer Form verestert sind, sowie ihre Salze, werden hergestellt, indem man in einer der Formel I entsprechenden Ausgangsverbindung, worin mindestens eine der vorhandenen funktionellen Gruppen geschützt ist, die funktionelle(n) Gruppe(n) freisetzt, wenn erwünscht, in einer erhaltenen Verbindung eine Gruppe $R_1$ in eine andere Gruppe $R_1$ überführt, und/oder, wenn erwünscht, eine freie Carboxylgruppe in eine physiologisch spaltbare veresterte Carboxylgruppe überführt, und/oder, wenn erwünscht, ein erhaltenes Isomerengemisch in die einzelnen Isomeren auftrennt, und/oder, wenn erwünscht, eine erhaltene Verbindung in ein Salz oder ein erhaltenes Salz in eine freie Verbindung oder in ein anderes Salz überführt.

In den Ausgangsverbindungen der Formel I, worin funktionelle Gruppen geschützt sind, werden diese, z.B. geschützte Carboxyl-, Amino-, Hydroxy-, Mercapto- und/oder Sulfogruppen, in an sich bekannter Weise, wie mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt.

So kann man z.B. eine tert.-Niederalkoxycarbonyl-, Polycycloalkoxycarbonyl- oder Diphenylmethoxycarbonylgruppe durch Behandeln mit einem geeigneten sauren Mittel, wie Ameisensäure oder Trifluoressigsäure, gegebenenfalls unter Zugabe einer nucleophilen Verbindung, wie Phenol oder Anisol, in die freie Carboxylgruppe überführen. Eine gegebenenfalls substituierte Benzyloxycarbonylgruppe kann z.B. mittels Hydrogenolyse durch Behandeln mit Wasserstoff in Gegenwart eines Hydrierkatalysators, wie eines Palladiumkatalysators freigesetzt werden. Ferner kann man bestimmt substituierte Benzyloxycarbonylgruppen, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoffabgebenden Mittels, das zusammen mit dem Metall nascierenden Wasserstoff zu erzeugen vermag, wie einer Säure, in erster Linie Essig-, sowie Ameisensäure, oder eines Alkohols, wobei man vorzugsweise Wasser zugibt, in die freie Carboxylgruppe überführen. Durch Behandeln mit einem reduzierenden Metall oder Metallsalz, wie oben beschrieben, kann man auch eine 2-Halogen-niederalkoxycarbonylgruppe (gegebenenfalls nach Umwandlung einer 2-Brom-niederalkoxycarbonylgruppe in eine 2-Iodniederalkoxycarbonylgruppe) oder eine Acylmethoxycarbonylgruppe in die freie Carboxylgruppe umwandeln, wobei eine Aroylmethoxycarbonylgruppe ebenfalls durch Behandeln mit einem nucleophilen, vorzugsweise salzbildenden Reagens, wie Natriumthiophenolat oder Natriumjodid gespalten werden kann. Eine Polyhalogenaryloxycarbonylgruppe, wie die Pentachlorphenyloxycarbonylgruppe wird unter milden basischen Bedingungen, wie durch verdünnte Natronlauge oder organische Basen in Gegenwart von Wasser, zur freien Carboxylgruppe verseift. Eine z.B. durch Silylierung oder Stannylierung geschützte Carboxylgruppe kann in üblicher Weise, z.B. durch Behandeln mit Wasser oder einem Alkohol, freigesetzt werden.

Eine geschützte Aminogruppe wird in an sich bekannter und je nach Art der Schutzgruppe in verschiedenartiger Weise, z.B. mittels Solvolyse oder Reduktion, freigesetzt. Eine 2-Halogenniederalkoxycarbonylaminogruppe (gegebenenfalls nach Umwandlung einer 2-Brom-niederalkoxycarbonylgruppe in eine 2-Jod-Niederalkoxycarbonylgruppe), eine Acylmethoxycarbonylaminogruppe oder eine 4-Nitrobenzyloxycarbonylaminogruppe kann z.B. durch Behandeln mit einem geeigneten chemischen Reduktionsmittel, wie Zink in Gegenwart von wässriger Essigsäure, eine Aroylmethoxycarbonylaminogruppe auch durch Behandeln mit einem nucleophilen, vorzugsweise salzbildenden Reagens, wie Natriumthiophenolat, und eine 4-Nitro-benzyloxycarbonylaminogruppe auch durch Behandeln mit einem Alkalimetall-, z.B. Natriumdithionit, eine Diphenylmethoxycarbonylamino-, tert.-Niederalkoxycarbonylamino- oder Polycycloalkoxycarbonylaminogruppe durch Behandeln z.B. mit Ameisen- oder Trifluoressigsäure, eine gegebenenfalls substituierte Benzyloxycarbonylaminogruppe z.B. mittels Hydrogenolyse durch Behandeln mit Wasserstoff in Gegenwart eines Hydrierkatalysators, wie eines Palladiumkatalysators, eine Aryl- oder Arylniederalkylthioaminogruppe z.B. durch Behandeln mit einem nucleophilen Reagens, wie schwefliger Säure, eine Arylsulfonylaminogruppe z.B. mittels elektrolytischer Reduktion, eine 1-Acyl-2-niederalkylidenaminogruppe oder eine Triarylmethylgruppe z.B. durch Behandeln mit wässriger Mineralsäure, und eine mit einer organischen Silyl- oder Stannylgruppe geschützte Aminogruppe z.B. mittels Hydrolyse oder Alkoholys freigesetzt werden.

Eine in Form einer Azidogruppe geschützte Aminogruppe wird in an sich bekannter Weise durch Reduktion, in die freie Aminogruppe übergeführt, beispielsweise durch katalytische Hydrierung mit Wasserstoff und einem Hydrierkatalysator, wie Platinoxid, Palladium, oder auch Raney-Nickel, oder auch durch Zink und Säure, wie Essigsäure. Die katalytische Hydrierung wird bevorzugt in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20 bis 25°, oder auch bei erniedrigter oder erhöhter Temperatur, durchgeführt.

Eine durch eine Acylgruppe, eine Silyl- oder Stannylgruppe oder durch einen gegebenenfalls substituierten $\alpha$-Phenylniederalkylrest geschützte Hydroxygruppe wird wie eine entsprechend geschützte Aminogruppe freigesetzt. Eine durch 2,2-Dichloracetyl geschützte Hydroxygruppe wird durch basische Hydrolyse und eine durch einen 2-oxa- oder 2-thia-aliphatischen oder -cycloaliphatischen Kohlenwasserstoffrest geschützte Hydroxygruppe wird durch Acidolyse freigesetzt.

Eine geschützte Sulfogruppe wird analog einer geschützten Carboxylgruppe freigesetzt.

Bevorzugt werden die Schutzgruppen so gewählt, dass sie alle gleichzeitig abgespalten werden können, beispielsweise acidolytische, wie durch Behandeln mit Trifluoressigsäure oder Ameisensäure, oder reduktiv, wie durch Behandeln mit Zink und Eisessig, oder mit Wasserstoff und einem Hydrierkatalysator, wie einem Palladium/Kohle-Katalysator.

Die beschriebenen Spaltungsreaktionen werden unter an sich bekannten Bedingungen durchgeführt, wenn notwendig unter Kühlen oder Erwärmen, in einem geschlossenen Gefäss und/oder in einer Inertgas-, z.B. Stickstoffatmosphäre.

In einer Verbindung der Formel I, worin eine Aminogruppe, wenn notwendig, geschützt ist, und worin die Carboxylgruppe in 4-Stellung des Cephemringes in freier Form vorliegt, kann man in an sich bekannter Weise eine Gruppe $R_1$ durch einen anderen Rest $R_1$ ersetzen oder in einen anderen Rest $R_1$ unwandeln. So ist es z.B. möglich, in einer Verbindung der Formel I, worin $R_1$ eine Gruppe der Formel $-CH_2-R_2$ bedeutet, und $R_2$ z.B. einen, durch nucleophile Substituenten ersetzbaren Rest darstellt, oder in einem Salz davon, durch Behandeln mit einer entsprechenden Mercaptan- oder mit einer Thiocarbonsäureverbindung einen solchen Rest $R_2$ durch eine verätherte bzw. veresterte Mercaptogruppe $R_2$ zu ersetzen. Ein geeigneter, durch eine verätherte Mercaptogruppe ersetzbarer Rest ist beispielsweise eine durch eine niederaliphatische Carbonsäure veresterte Hydroxygruppe. Solche veresterten Hydroxygruppen sind insbesondere Acetyloxy, ferner Formyloxy.

Die Reaktion einer solchen Verbindung mit einer geeigneten Mercaptanverbindung kann unter neutralen oder schwach basischen Bedingungen in Gegenwart von Wasser und gegebenenfalls einem, mit Wasser mischbaren organischen Lösungsmittel durchgeführt werden. Die basischen Bedingungen können beispielsweise durch Zugabe einer anorganischen Base, wie eines Alkalimetall- oder Erdalkalimetallhydroxids, -carbonats oder -hydrogencarbonats, z.B. von Natrium-, Nalium- oder Calciumhydroxid, -carbonat oder -hydrogencarbonat, eingestellt werden. Als organische Lösungsmittel können

z.B. mit Wasser mischbare Alkohole, z.B. Niederalkanole, wie Methanol oder Aethanol, Ketone, z.B. Niederalkanone, wie Aceton, Amide, z.B. Niederalkancarbonsäureamide, wie Dimethylformamid, oder Nitrile, z.B. Niederalkansäurenitrile, wie Acetonitril, und ähnliche verwendet werden.

Veresterte Hydroxygruppen $R_2$ in einer Verbindung der Formel I, worin $R_1$ die Gruppe $-CH_2-R_2$ bedeutet, wobei $R_2$ für eine, durch den Acylrest eines gegebenenfalls substituierten Halbamids der Kohlensäure veresterte Hydroxygruppe steht, kann man z.B. einführen, indem man eine entsprechende Verbindung der Formel I, worin $R_2$ für freies Hydroxy steht (das man z.B. durch Abspaltung des Acetylrestes aus einer Acetyloxygruppe $R_2$, z.B. durch Hydrolyse in schwach-basischem Medium, wie mit einer wässrigen Natriumhydroxydlösung bei pH 9—10, oder durch Behandeln mit einer geeigneten Esterase, wie einem entsprechenden Enzym aus *Rhizobium tritolii, Rhizobium lupinii, Rhizobium japonicum* oder *Bacillus subtilis*, oder einer geeigneten Citrus-Esterase, z.B. aus Orangenschalen, freisetzen kann), mit einem geeigneten Kohlensäurederivat, insbesondere mit einer Isocyanat- oder Carbaminsäureverbindung, wie einem Silylisocyanat, z.B. Silyltetraisocyanat, einem Sulfonylisocyanat, z.B. Chlorsulfonylisocyanat, oder Carbaminsäurehalogenid, z.B. -chlorid (die zu N-unsubstituierten 3-Aminocarbonyloxymethyl-Verbindungen führen), oder dann mit einer N-substituierten Isocyanat- oder mit einer N-mono- oder N,N-disubstituierten Carbaminsäure-Verbindungen, wie einem entsprechenden Carbaminsäurehalogenid, z.B. -chlorid, umsetzt, wobei man üblicherweise in Gegenwart eines Lösungs- oder Verdünnungsmittels und, wenn notwendig, unter Kühlen oder Erwärmen, in einem geschlossenen Gefäss und/oder in einer Inertgas-, z.B. Stickstoffatomosphäre, arbeitet.

Ferner kann man eine Verbindung der Formel I, worin $R_1$ eine Gruppe $-CH_2-R_2$ bedeutet, wobei $R_2$ z.B. den oben definierten, durch nucleophile Substitution ersetzbaren Rest darstellt, mit einer tertiären organischen Base, insbesondere einem gegebenenfalls substituierten Pyridin, unter neutralen oder schwach sauren Bedingungen, bevorzugt bei einem pH-Wert von etwa 6,5, in Gegenwart von Wasser und gegebenenfalls in einem, mit Wasser mischbaren organischen Lösungsmittel umsetzen und so zu Verbindungen der Formel I gelangen, worin $R_1$ den Rest der Formel $-CH_2-R_2$ darstellt und $R_2$ für eine quaternäre Ammoniumgruppe steht. Die schwach-sauren Bedingungen können durch Zugabe einer geeigneten organischen oder an organischen Säure, beispielsweise Essigsäure, Chlorwasserstoffsäure Phosphorsäure oder auch Schwefelsäure eingestellt werden. Als organische Lösungsmittel können beispielsweise die verstehend genannten, mit Wasser mischbaren Lösungsmittel wendet werden. Zur Erhöhung der Ausbeute können der Reaktionsmischung gewisse Salze zugesetzt werden, beispielsweise Alkalimetall-, wie Natrium- und insbesondere Kaliumsalze, von anorganischen Säuren, wie Halogenwasserstoffsäuren, z.B. Chlorwasserstoff- und insbesondere Jodwasserstoffsäure, sowie der Thiocyansäure, oder organischen Säuren, wie Niederalkancarbonsäuren, z.B. Essigsäure, Vertreter solcher Salze sind beispielsweise Kaliumjodid und Kaliumthiocyanat. Auch Salze von geeigneten Anionenaustauschern, z.B. flüssige Ionenaustauscher in Salzform, wie z.B. Amberlite LA—1 (flüssige sekundäre Amine mit einem Molekulargewicht von 351—393; Oel-löslich und Wasser-unlöslich; mAeq/g = 2,5—2,7 z.B. in Acetatform), mit Säuren, z.B. Essigsäure, können für diesen Zweck verwendet werden.

Quaternäre Ammoniumgruppen $R_2$ können vorteilhafterweise unter Verwendung eines Zwischenprodukts der Formel I, in welchem $R_2$ für eine substituierte, insbesondere für eine aromatisch substituierte Carbonylthiogruppe und in erster Linie für die Benzoylthiogruppe steht, hergestellt werden. Ein solches Zwischenprodukt, das man z.B. durch Umsetzen einer Verbindung der Formel I, worin $R_2$ im Rest $R_1$ eine veresterte Hydroxygruppe, insbesondere eine Niederalkanoyloxy z.B. Acetyloxygruppe bedeutet, mit einem geeigneten Salz, wie einem Alkalimetall-, z.B. Natriumsalz, einer Thiocarbonsäure, wie einer aromatischen Thiocarbonsäure, z.B. Thiobenzoesäure, erhalten kann, wird mit dem tertiären Amin, insbesondere einer tertiären heterocyclischen Base, wie einem gegebenenfalls substituierten Pyridin, umgesetzt, wobei man die quaternäre Ammoniumverbindung erhält. Die Reaktion wird üblicherweise in Gegenwart eines geeigneten Entschewefelungsmittels, insbesondere eines Quecksilbersalzes, z.B. Quecksilber-II-perchlorat, und eines geeigneten Lösungs- oder Verdünnungsmittels oder eines Gemisches, wenn notwendig, unter Kühlen oder Erwärmen, in einem geschlossenen Gefäss und/oder in einer Inertgas-, z.B. Stickstoffatmosphäre, durchgeführt.

Die Ueberführung einer freien Carboxylgruppe in einer erhaltenen Verbindung der Formel I in eine veresterte Carboxylgruppe, die unter physiologischen Bedingungen spaltbar ist, erfolgt nach an sich bekannten Veresterungsmethoden, beispielsweise indem man eine Verbindung der Formel I, worin andere funktionelle Gruppen, wie Amino-, Hydroxy- oder Sulfogruppen, gegebenenfalls in geschützter Form vorliegen, oder ein bezüglich der zu veresternden Carboxylgruppe reaktionsfähiges funktionelles Derivat davon, oder ein Salz davon, mit einem entsprechenden Alkohol oder einem reaktionsfähigen funktionellen Derivat davon, verestert.

Salze von Verbindungen der Formel I können in an sich bekannter Weise hergestellt werden. So kann man Salze von Verbindungen der Formel I mit sauren Gruppen, z.B. durch Behandeln mit Metalllverbindungen, wie Alkalimetallsalzen von geeigneten Carbonsäuren, z.B. dem Natriumsalz der $\alpha$-Aethylcapronsäure oder Natriumcarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche eine freie Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Das Vefahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder während der Reaktion gebildet werden.

Vorzugsweise werden solche Ausgangsstoffe verwendet und die Reaktionsbedingungen so gewählt, dass man zu den vorstehend als besonders bevorzugt aufgeführten Verbindungen gelangt.

Die Ausgangsverbindungen der Formel I, worin mindestens eine der funktionellen Gruppen in geschützter Form vorliegt, können in an sich bekannter Weise hergestellt werden, indem man z.B.

a) in einer Verbindung der Formel

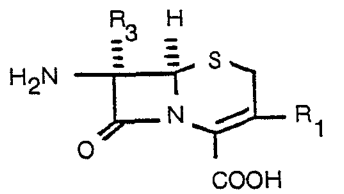

(II),

worin die Aminogruppe gegebenenfalls durch eine die Acylierung erlaubende Gruppe substituiert ist, und worin die 4-Carboxylgruppe und gegebenenfalls im Rest $R_1$ vorhandene weitere funktionelle Gruppen in geschützter Form vorliegen können, die Aminogruppe durch Behandeln mit einer Säure der Formel

$$HOOC-\underset{NH_2}{\underset{|}{CH}}-(C_nH_{2n})-X-W-NH-(C_mH_{2m})-A-\overset{Y}{\overset{|}{\underset{Z}{\underset{|}{C}}}}-\overset{O}{\overset{\|}{C}}-OH \qquad (III),$$

worin die Aminocarbonsäuregruppierung $HOOC-CH(NH_2)-$ und gegebenenfalls in der Gruppierung $-A-C(Y)(Z)-$ vorhandene weitere funktionelle Gruppen in geschützter Form vorliegen, oder mit einem reaktionsfähigen funktionellen Säurederivat oder einem Salz davon acyliert, oder

b) in einer Verbindung der Formel

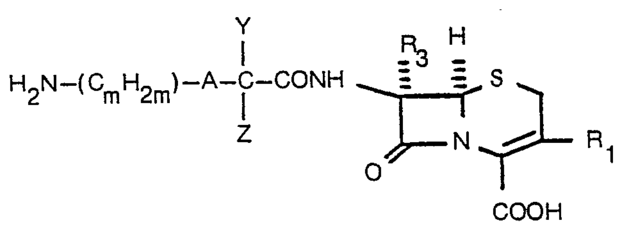

(IV),

worin die Aminogruppe gegebenenfalls durch eine, die Acylierung erlaubende Gruppe substituiert sein kann, und worin die 4-Carboxylgruppe und gegebenenfalls im Rest $R_1$ und in der Gruppierung $-A-C(Y)(Z)-$ vorhandene weitere funktionelle Gruppen in geschützter Form vorliegen können, die Aminogruppe durch Behandeln mit einem reaktionsfähigen funktionellen Derivat einer Säure der Formel

$$HOOC-\underset{NH_2}{\underset{|}{CH}}-(C_nH_{2n})-X-W-OH \qquad (V),$$

worin die Aminocarbonsäuregruppierung $HOOC-CH(NH_2)-$ in geschützter Form vorliegt, oder wenn $X-W$ zusammen eine Gruppe $-CO-$ bedeuten, auch mit einer entsprechenden freien Säure, oder mit einem Salz davon, acyliert, oder

c) in einer Verbindung der Formel

$$HOOC-\underset{\displaystyle NH_2}{\underset{|}{CH}}-(C_nH_{2n})-X-H \qquad (VI),$$

worin X Sauerstoff, Schwefel oder die Gruppe —NH— bedeutet, und worin die Aminocarbonsäuregruppierung HOOC—CH(NH$_2$— in geschützter Form vorliegt, die Gruppe —X—H mit einem reaktionsfähigen funktionellen Derivat einer Verbindung der Formel

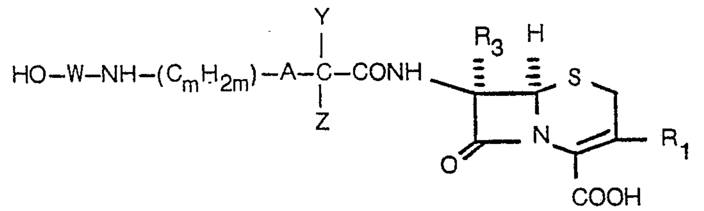

(VII),

worin die 4-Carboxylgruppe und gegebenenfalls im Rest $R_1$ und in der Gruppierung —A—C(Y)(Z)— vorhandene funktionelle Gruppen in geschützter Form vorliegen können, acyliert, und, wenn gewünchst, in einer erhaltenen Verbindung noch nicht geschützte funktionelle Gruppen schützt oder eine Schutzgruppe gegen eine andere austauscht, und/oder, wenn gewünchst, in einem Rest $R_1$ eine Gruppe $R_2$ gegen eine andere Gruppe $R_2$ austauscht, und/oder, wenn gewünscht, eine erhaltene Verbindung, worin $R_3$ Wasserstoff ist, in eine Verbindung überführt, worin $R_3$ Methoxy ist, und/oder wenn notwendig, eine erhaltene 2-Cephemverbindung oder ein erhaltenes Gemisch einer 2-Cephem- und 3-Cephemverbindung, zur 3-Cephemverbindung isomerisiert, und/oder, wenn gewünscht, ein erhaltenes Isomerengemisch in die einzelnen Isomeren auftrennt.

Gegebenenfalls vorhandene, die Aminogruppe substituierende und deren Acylierung erlaubende Reste in einem Ausgangsmaterial der Formel II oder IV sind beispielsweise organische Silyl- oder Stannylgruppen, ferner auch Ylidengruppen, die zusammen mit der Aminogruppe eine Schiff'sche Base bilden. Die genannten organischen Silyl- oder Stannylgruppen sind z.B. die gleichen, die auch mit der 4-Carboxylgruppe am Cephemring eine geschützte Carboxylgruppe zu bilden vermögen. Bei der Silylierung oder Stannylierung einer Carboxylgruppe in einem Ausgangsmaterial der Formel II oder IV, kann, bei Verwendung eines Ueberschusses des Silylierungs- oder Stannylierungsmittels, die Aminogruppe ebenfalls silyliert oder stannyliert werden.

Die genannten Ylidengruppen sind in erster Linie Arylmethylengruppen, worin Aryl insbesondere für einen carbocyclischen, in erster Linie monocyclischen Arylrest, z.B. für gegebenenfalls, wie durch Nitro oder Hydroxy, substituiertes Phenyl steht; solche Arylmethylengruppen sind z.B. Benzyliden, 2-Hydroxybenzyliden oder 4-Nitrobenzyliden, ferner gegebenenfalls, z.B. durch Carboxy substituiertes Oxacycloalkyliden, z.B. 3-Carboxy-2-oxacyclohexyliden.

Die übrigen in den Ausgangsstoffen der Formeln II bis VII vorhandenen funktionellen Gruppen können durch die bereits unter den Verbindungen der Formel I genannten Schutzgruppen geschützt sein. Bevorzugt sind alle nicht an der Acylierungsreaktion teilnehmenden reaktionsfähigen funktionellen Gruppen, insbesondere aber gegebenenfalls vorhandene, acylierbare Amino-, Hydroxy- und Mercaptogruppen, geschützt.

Falls eine freie Säure der Formel III oder V, worin alle funktionellen Gruppen ausser der reagierenden Carboxylgruppe geschützt sind, zur Acylierung eingesetzt wird, verwendet man üblicherweise geeignete Kondensationsmittel, wie Carbodiimide, beispielsweise N,N'-Diäthyl-, N,N'-Dipropyl-, N,N'-Diisopropyl-, N,N'-Dicyclohexyl- oder N-Aethyl-N'-3-dimethylaminopropyl-carbodiimid, geeignete Carbonylverbindungen, beispielsweise Carbonyldiimidazol, oder Isoxazoliniumsalze, beispielsweise N-Aethyl-5-phenyl-isoxazolinium-3'-sulfonat und N-tert.-Butyl-5-methyl-isoxazoliniumperchlorat, oder eine Acylaminoverbindung, z.B. 2-Aethoxy-1-äthoxycarbonyl-1,2-dihydro-chinolin.

Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran, wenn erwünscht oder notwendig, unter Kühlen oder Erwärmen und/oder in einer Inertgasatmosphäre, durchgeführt.

Ein reaktionsfähiges, d.h. Amid-bildendes, bzw. Esterbildendes, funktionelles Derivat einer Säure der Formel III, V oder VII, worin alle funktionellen Gruppen ausser der reagierenden Säuregruppe geschützt sind bzw. sein können, ist in erster Linie ein Anhydrid einer solchen Säure, inklusive und vorzugsweise ein gemischtes Anhydrid, aber auch ein inneres Anhydrid, d.h. ein entsprechendes Keten oder, in der Säure V, wenn W die Gruppe —$SO_2$NH—CO— ist, oder wenn X die Gruppe —NH— und W die Gruppe —CO— oder —CO—$NHSO_2$— ist, ein entsprechendes Isocyanat. Gemischte Anhydride sind z.B. diejenigen mit anorganischen Säuren, wie Halogenwasserstoffsäuren, d.h. die entsprechenden Säurehalogenide, z.B. -chloride oder -bromide, ferner mit Stickstoffwasserstoffsäure, d.h. die entsprechenden Säureazide, mit einer phosphorhaltigen Säure, z.B. Phosphorsäure oder phosphoriger Säure, oder mit einer schwefelhatigen Säure, z.B. Schwefelsäure, oder mit Cyanwasserstoffsäure. Weitere gemischte Anhydride sind z.B. diejenigen mit organischen Carbonsäuren, wie mit gegebenenfalls, z.B. durch Halogen, wie Fluor oder Chlor, substituierten Niederalkancarbonsäuren, z.B. Pivalinsäure oder Trichloressigsäure, oder mit Halbestern, insbesondere Niederalkylhalbestern der Kohlensäure, wie dem Aethyl- oder Isobutylhalbester der Kohlensäure, oder mit organischen, insbesondere aliphatischen oder aromatischen, Sulfonsäuren, z.B. p-Toluolsulfonsäure. Von der Säure III, wenn Z Wasserstoff ist und Y Hydroxy bedeutet, kann auch ein gemischtes inneres Anhydrid mit dem Kohlensäurehalbesters der $\alpha$-Hydroxygruppe verwendet werden.

Weitere, zur Reaktion mit der Amino-, Hydroxy- oder Mercaptogruppe geeignete Säurederivate in einer Säure der Formel III, V, oder VII, worin alle funktionellen Gruppen ausser der reagierenden Carboxylgruppe geschützt sind, bzw. sein können, sind aktivierte Ester, wie Ester mit vinylogen Alkoholen (d.h. Enolen), wie vinylogen Niederalkenolen, oder Arylester, wie 4-Nitrophenyl- oder 2,4-Dinitrophenylester, heteroaromatische Ester, wie Benztriazol-, z.B. 1-Benztriazolester, oder Diacyliminoester, wie Succinylimino- oder Phthalylimioester.

Die Acylierung mit einem Säurederivat, wie einem Anhydrid, insbesondere mit einem Säurehalogenid, kann in Anwesenheit eines säurebindenden Mittels, beispielsweise einer organischen Base, wie eines organischen Amins, z.B. eines tertiären Amins, wie Triniederalkylamin, z.B. Trimethylamin, Triäthylamin oder Aethyl-diisopropylamin, oder N,N-Diniederalkyl-anilin, z.B. N,N-Dimethylanilin, oder eines cyclischen tertiären Amins, wie eines N-niederalkylierten Morpholins, wie N-Methylmorpholin, oder einer Base vom Pyridin-Typ, z.B. Pyridin, einer anorganischen Base, beispielsweise eines Alkalimetall- oder Erdalkalimetallhydroxids, -carbonats oder -hydrogencarbonats, z.B. Natrium-, Kalium- oder Calciumhydroxid, -carbonat oder -hydrogencarbonat, oder eines Oxirans, beispielsweise eines niederen 1,2-Alkylenoxids, wie Aethylenoxid oder Propylenoxid, durchgeführt werden.

Die obigen Acylierungen können in einem inerten, vorzugsweise wasserfreien Lösungsmittel oder Lösungsmittelgemisch vorgenommen werden, beispielsweise in einem Carbonsäureamid, wie einem Formamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem Ester, z.B. Essigsäureäthylester, oder einem Nitril, z.B. Acetonitril, oder Mischungen davon, und, wenn notwendig, bei erniedrigter oder erhöhter Temperatur, etwa bei —40° bis etwa 100°, bevorzugt bei —10° bis +40°, und/oder in einer Inertgas-, z.B. Stickstoffatmosphäre.

In einer acylierenden Säure der Formel III, V oder VII oder in einem Säurederivat davon kann eine geschützte Aminogruppe auch in ionischer Form vorliegen, d.h. das Ausgangsmaterial der Formel III, V oder VII kann in Form eines Säureadditionssalzes, vorzugsweise mit einer starken anorganischen Säure, wie einer Halogenwasserstoffsäure, z.B. Salzsäure, oder Schwefelsäure verwendet werden.

Ferner kann ein Säurederivat, wenn erwünscht, *in situ* gebildet werden. So erhält man z.B. ein gemischtes Anhydrid durch Behandeln einer Säure der Formel III, oder einer Säure der Formel V, worin X—W die Gruppe —CO— bedeutet, mit entsprechend geschützten funktionellen Gruppen, oder eines geeigneten Salzes davon, wie eines Ammoniumsalzes, z.B. mit einem organischen Amin, wie 4-Methylmorpholin, oder eines Metall-, z.B. Alkalimetallsalzes, mit einem geeigneten Säurederivat, wie einem entsprechenden Säurehalogenid einer gegebenenfalls substituierten Niederalkancarbonsäure, z.B. Trichloracetylchlorid, oder mit einem Halbester eines Kohlensäurehalbhalogenids, z.B. Chlorameisensäureäthylester oder -isobutylester, und verwendet das so erhältliche gemischte Anhydrid ohne Isolierung.

Ein Säurechlorid einer Säure der Formel V, worin X—W eine Gruppierung —O—CO—, —S—CO— oder —NH—CO— ist und worin die Aminocarbonsäuregruppierung HOOC—CH($NH_2$)— in geschützter Form vorliegt, kann, z.B. *in situ*, gebildet werden, indem man eine Aminocarbonsäure der Formel VI, worin X Sauerstoff, Schwefel oder die Gruppe —NH— bedeutet, und die Gruppierung HOOC—CH($NH_2$)— in geschützter Form vorliegt, in Gegenwart eines Salzsäureakzeptors in einem inerten organischen Lösungsmittel oder Lösungsmittelgemisch mit Phosgen behandelt. Die Salzsäureakzeptoren, Lösungsmittel und Reaktionsbedingungen sind die gleichen, die für die Acylierung von Verbindungen der Formel II oder IV genannt wurden; beispielsweise kann die Reaktion in Gegenwart von Pyridin in Methylenchlorid und Toluol bei etwa 0° bis etwa +10° stattfinden.

Auf die gleiche Weise kann, z.B. *in situ*, ein Säurechlorid einer Säure der Formel VII, worin W die Gruppe —CO— bedeutet, und worin die 4-Carboxylgruppe und in den Gruppen —A—C(Y)(Z)— und $R_1$ gegebenenfalls vorhandene weitere funktionelle Gruppen geschützt sind, aus einer entsprechend geschützten Verbindung der Formel IV, durch Behandlung mit Chlorsulfonylisocyanat hergestellt werden.

In einem erhaltenen Zwischenprodukt der Formel I, worin mindestens eine der funktionellen Gruppen in geschützter Form vorliegt, können auf übliche, an sich bekannte Weise, noch nicht geschützte funktionelle Gruppen geschützt oder vorhandene Schutzgruppen gegen andere Schutzgruppen ausgetauscht werden, z.B. durch Abspalten der vorhandenen Schutzgruppe und Einführen der gewünschten anderen Schutzgruppe.

Verbindungen der Formel III, worin die Aminocarbonsäuregruppierung HOOC—CH($NH_2$)— und gegebenenfalls in der Gruppierung —A—C(Y)(Z)— vorhandene weitere funktionelle Gruppen in geschützter Form vorliegen, werden beispielsweise hergestellt, indem man eine Verbindung der Formel

$$H_2N—(C_mH_{2m})—A—\underset{\underset{Z}{|}}{\overset{\overset{Y}{|}}{C}}—\overset{\overset{O}{\|}}{C}—OH \qquad (VIII),$$

worin die Aminogruppe gegebenenfalls durch eine die Acylierung erlaubende Gruppe substituiert sein kann, und gegebenenfalls in der Gruppierung —A—C(Y)(Z)— vorhandene funktionelle Gruppen geschützt sind, unter intermediärem Schutz der Carboxylgruppe, mit einem reaktionsfähigen funktionellen Derivat einer Säure der Formel (V)

$$HOOC—\underset{\underset{NH_2}{|}}{CH}—(C_nH_{2n})—X—W—OH \qquad (V),$$

worin die Aminocarbonsäuregruppierung HOOC—CH($NH_2$)— in geschützter Form vorliegt, oder, wenn X—W zusammen eine gruppe —CO— bedeuten, auch mit einer entsprechenden freien Säure oder mit einem Salz davon, acyliert, und wenn erwünscht, eine erhaltene Verbindung in eine andere Verbindung der Formel III mit entsprechend geschützten funktionellen Gruppen überführt.

Die die Acylierung erlaubenden Gruppen, sowie die Schuztgruppen der Aminocarbonsäuregruppierung HOOC—CH($NH_2$)— und der Gruppierung —A—C(Y)(Z)—, sind die gleichen wie die unter den Verbindungen der Formeln II oder IV, sowie I erwähnten. Zum intermediären Schutz der Carboxylgruppe in der Verbindung der Formel VIII können an sich ebenfalls die bereits erwähnten Carboxylschutzgruppen verwendet werden, jedoch müssen sich die zum intermediären Schutz in der vorliegenden Acylierung benützten Carboxylschutzgruppen von den überigen Schutzgruppen, die in den Verbindungen der Formel III notwendigerweise vorhanden bleiben sollen, in der Art ihrer Abspaltung unterscheiden, so dass sie nach der vorliegenden Acylierungsreaktion selektiv abgespalten werden können. Wenn beispielsweise zum intermediären Schutz der Carboxylgruppe eine hydrogenolytisch abspaltbare Schutzgruppe, wie eine der genannten gegebenenfalls substituierten Benzylgruppen, z.B. die Benzyl- oder p-Nitrobenzylgruppe, verwendet wird, dann dürfen die anderen Schutzgruppen hydrogenolytisch nicht abspaltbar sein; sie können beispielsweise die genannten, nur acidolytisch abspaltbaren tert.-Niederalkylgruppen, wie tert.Butyl bzw. tert.-Niederalkoxycarbonylgruppen, wie tert.-Butoxycarbonyl, sein.

Die Acylierung kann im übrigen analog ausgeführt werden wie die Acylierung von Verbindungen der Formel IV mit einer Säure der Formel V, bzw. einem entsprechend geschützten und reaktionsfähigen funktionellen Derivat davon.

In einer erhaltenen Verbindung der Formel III, mit entsprechend geschützten funktionellen Gruppen, kann eine Schutzgruppe, gegebenenfalls selektiv, abgespalten, oder eine, gegebenenfalls bei der Acylierungsreaktion frei gewordene, funktionelle Gruppe geschützt werden. In einer erhaltenen Verbindung der Formel III, worin Z Wasserstoff und Y Hydroxy bedeutet, kann die $\alpha$-Hydroxygruppe oxidativ, wie für die Oxidation von Verbindungen XI zu $\alpha$-Ketosäuren IX angegeben, z.B., unter vorübergehendem Schutz der Carboxylgruppe als Ester, durch Behandeln mit Mangandioxid, in eine $\alpha$-Oxogruppe übergeführt werden und in einer erhältlichen Verbindung der Formel III, worin Z und Y zusammen die Oxogruppe bedeuten, kann diese durch Behandeln mit einem Hydroxylamin der Formel $H_2N—O—R^o$, in die entsprechende Oximinogruppe übergeführt werden, welche Reaktion analog der Ueberführung von $\alpha$-Ketosäuren der Formel IX in Oximinoverbindungen der Formel VIIIa ausgeführt werden kann.

Säuren der Formel V, reaktionsfähige funktionelle Derivate davon, die Vorstufen der Formel

$$HOOC—CH(NH_2)—(C_nH_{2n})—X—H \qquad (VI)$$

und entsprechend geschütze Derivate sind bekannt oder können nach an sich bekannten Methoden, beispielsweise *in situ*, hergestellt werden.

Säuren der Formel VIII und entsprechenden reaktionsfähige funktionelle und geschützte Derivate davon sind zum Teil bekannt oder können nach an sich bekannten Methoden hergestellt werden.

Die unter die Formel VIII fallenden Säuren der Formel

$$H_2N—(C_mH_{2m})—A—\overset{\overset{N—O—R^o}{\|}}{C}—COOH \qquad (VIIIa),$$

worin der Index m den Wert 1 hat, und A und $R^o$ die unter Formel I genannten Bedeutungen haben, sowie entsprechende an der Aminogruppe geschützte, sowie bezüglich der Carboxylgruppe reaktionsfähige funktionelle Derivate davon sind neu und können auf an sich bekannte Weise hergestellt werden, indem man eine $\alpha$-Ketosäure der Formel

$$H_2N—(C_mH_{2m})—A—C(=O)—COOH \quad (IX)$$

worin m und A die unter Formel VIIIa genannten Bedeutungen haben und worin die Aminogruppe in geschützter Form vorliegt, mit einer Hydroxylaminoverbindung der Formel $H_2N—O—R^o$, worin $R^o$ die unter Formel VIIIa genannte Bedeutung hat, behandelt und, wenn gewünscht, in einer erhaltenen Verbindung der Formel VIIIa mit geschützter Aminogruppe, diese in die freie Aminogruppe und/oder, wenn gewünscht, die erhaltene freie Aminogruppe in einer andersartig geschützte Aminogruppe überführt, und/oder, wenn gewünscht, ein erhaltenes Isomerengemisch in die Isomeren auftrennt, und/oder, wenn gewünscht, in einer erhaltenen Verbindung der Formel VIIIa, worin die Aminogruppe geschützt ist, die Carboxylgruppe in ein reaktionsfähiges funktionelles Derivat davon überführt.

In einer $\alpha$-Ketosäure der Formel IX ist A insbesondere eine der unter Formel I genannten bevorzugten Gruppen, in erster Linie 2,5-Furylen, 2,5-Thienylen oder 1,4-Phenylen. Die Aminoschutzgruppe in einer Verbindung der Formel IX ist eine der zuvor genannten, beispielsweise eine der unter Formel I genannten, während der Reaktion stabilen und anschliessend leicht abspaltbaren Acylgruppen, insbesondere die Trifluoracetylgruppe.

Die Reaktion der $\alpha$-Ketosäure mit der Hydroxylaminverbindung $H_2N—O—R^o$ wird auf übliche Weise ausgeführt, z.B. indem man die beiden Reaktionspartner in einem Lösungsmittel, wie Wasser oder einem organischen Lösungsmittel, wie einem Alkohol, z.B. Methanol, bei leicht erhöhter oder erniedrigter Temperatur, gegebenenfalls in einer Inertgas-, wie Stickstoffatmosphäre, reagieren lässt. Die Hydroxylaminverbindung kann, auch in situ, aus einem ihrer Salze, beispielsweise einem Hydrohalogenid, wie Hydrochlorid, durch Behandeln mit einer anorganischen Base, wie einem Alkalimetallhydroxid, z.B. Natriumhydroxid, oder einer organischen Base, wie einem tertiären Amin, z.B. einem Triniederalkylamin, wie Triäthylamin oder Aethyl-diisopropylamin, oder einer heterocyclischen tertiären Base, wie Pyridin, in Freiheit gesetzt werden.

In einer erhaltenen Verbindung der Formel VIIIa kann die Schutzgruppe nach üblichen, vorher genannten Methoden abgespalten werden, eine Trifluoracetylgruppe beispielsweise durch alkalische Verseifung.

In einer erhaltenen Verbindung der Formel VIIIa kann die freie Aminogruppe nach üblichen, vorher genannten Methoden in eine der genannten geschützten Aminogruppen übergeführt werden, beispielsweise durch Behandeln mit einem Säureanhydrid, wie dem Anhydrid des Kohlensäure-mono-tert.butylesters in Gegenwart von Base, in die entsprechende N-Acyl-, z.B. N-tert.-Butoxycarbonylverbindung.

Verbindung der Formel VIIIa und ihre geschützten Derivate, fallen bei ihrer Bildung aus der $\alpha$-Ketosäure IX und der Hydroxylaminverbindung $H_2N—O—R^o$ gewöhnlich als Gemisch von syn- und anti-Isomeren an, wobei das syn-Isomere üblicherweise in grösserer Menge gebildet wird. Die beiden Isomeren können in die syn- und anti-Isomeren getrennt werden, beispielsweise durch Kristallisation, Chromatographie, Destillation und dergleichen.

Bezüglich der Carboxylgruppe reaktionsfähige, funktionelle Derivate von Verbindungen der Formel VIIIa, worin die Aminogruppe geschützt ist, sind die gleichen, wie die unter den Säuren der Formel III genannten, und sind insbesondere Anhydride, wie gemischte Anhydride mit den genannten anorganischen oder organischen Säuren, oder aktivierte Ester, wie diejenigen mit den genannten Alkoholen.

Die Ueberführung einer erhaltenen Verbindung der Formel VIIIa, worin die Aminogruppe geschützt ist, in ein entsprechendes reaktionsfähiges funktionelles Derivat bezüglich der Carboxylgruppe erfolgt auf an sich bekannte Weise, gegebenenfalls in situ, beispielsweise wie bei der Herstellung von reaktionsfähigen funktionellen Derivaten von Säuren der Formel III angegeben oder gemäss den bakannten Herstellungsmethoden für die reaktionsfähigen funktionellen Derivate der Formel III.

Säurehalogenide werden z.B. hergestellt, indem man eine Verbindung der Formel VIIIa, mit geschützter Aminogruppe, oder ein Salz davon mit einem Halogenierungsmittel, beispielsweise Phosphorpentachlorid, Thionylchlorid oder Oxalylchlorid, umsetzt. Die Umsetzung wird bevorzugt in einem nicht-wässrigen Lösungsmittel oder Lösungsmittelgemisch, wie einem Carbonsäureamid, z.B. Dimethylformamid, und/oder in Gegenwart einer Base, wie einem tertiären Amin, wie Triniederalkylamin, z.B. Triäthylamin, oder einem tertiären cyclischen Amin, wie N-Methyl-morpholin, durchgeführt.

Symmetrische Anhydride oder von Halogeniden verschiedene gemischte Anhydride von Verbindungen der Formel VIIIa mit geschützter Aminogruppe können z.B. hergestellt werden, indem man eine entsprechende Verbindung mit einer freien Carboxylgruppe, vorzugsweise ein Salz, insbesondere Alkalimetall-, z.B. Natrium-, oder Ammonium-, z.B. Triäthylammoniumsalz davon, mit einem reaktionsfähigen Derivat, wie einem Halogenid, z.B. dem Chlorid, einer der genannten Säuren, z.B. einem Niederalkylhalogenformiat, wie Isobutylchlorformiat, oder einem gegebenenfalls halogenierten Niederalkancarbonsäurechlorid, z.B. Trichloracetylchlorid, umsetzt.

Aktievierte Ester von Verbindungen der Formel VIIIa mit geschützter Aminogruppe können z.B. hergestellt werden, indem man eine entsprechende Verbindung mit freier Carboxylgruppe in Gegenwart eines Carbodiimids, z.B. eines der oben genannten Carbodiimide, wie N,N'-Dicyclohexylcarbodiimid, mit einem gegebenenfalls, z.B. durch Nitro oder Halogen, wie Chlor, substituierten Phenol, wie u.a. Nitrophenol, z.B. 4-Nitrophenol oder 2,4-Dinitrophenol, oder Polyhalogenphenol, z.B. 2,3,4,5,6-Pentachlorphenol, oder mit 1-Hydroxybenzotriazol umsetzt.

Die $\alpha$-Ketosäuren der Formel IX sind neu und ebenfalls Gegenstand der vorliegenden Erfindung. Sie können auf an sich bekannte Weise hergestellt werden, indem man in einer Verbindung der Formel

$$H_2N—(C_mH_{2m})—A—C(=O)—CH_3 \quad (X),$$

worin m und A die unter formel VIIIa genannten Bedeutungen haben und worin die Aminogruppe in geschützter Form vorliegt, die Methylgruppe zu einer Carboxylgruppe oxidiert, oder in einer $\alpha$-Hydroxysäure der Formel

$$H_2N—(C_mH_{2m})—A—CH(OH)—COOH \quad (XI),$$

worin m und A die unter Formel VIIIa genannten Bedeutungen haben und worin die Aminogruppe in geschützter Form vorliegt, die $\alpha$-Hydroxygruppe zu einer $\alpha$-Oxogruppe oxidiert.

In einer Ausgangsverbindung der Formel X oder XI, ist die Aminogruppe beispielsweise durch eine der genannten Acylschutzgruppen, insbesondere durch die Trifluoracetylgruppe, geschützt. Die Oxidation einer Verbindung X wird mit einem der für die Ueberführung von aromatischen Acetylverbindungen, d.h. Arylmethylketonen, in entsprechende $\alpha$-Ketosäuren, d.h. Arylglyoxylsäuren, geeigneten Oxidationsmittel auf übliche Weise durchgeführt. Geeignete Oxidationsmittel sind beispielsweise oxidierende Oxide oder sauerstoffhaltige Säuren, wie solche des Selens, Schwefels, Mangans, Chroms oder Stickstoff, oder Salze entsprechender Säuren, insbesondere Alkalimetall-, wie Kaliumsalze davon, wobei die Salze gegebenenfalls in Gegenwart von Mineralsäuren, wie Salzsäure oder Schwefelsäure, verwendet werden, Wasserstoffperoxid, oder auch Sauerstoff in Gegenwart eines Katalysators, wie Platin auf Kohle. Hervorzuhebende Oxidationsmittel sind Selendioxid oder die selenige Säure, permangansaure Salze, wie Kaliumpermanganat, dichromsaure Salze, wie Kaliumdichromat, und salpetrige Säure, die in situ aus einem anorganischen Nitritsalz, wie einem entsprechenden Alkalimetallsalz oder Erdalkalimetallsalz, z.B. Natriumsalz, und einer Säure, wie Salzsäure oder Schwefelsäure, gebildet wird.

Die Oxidation wird in Wasser oder einem gegebenenfalls mit Wasser mischbaren, gegebenenfalls wasserhaltigen organischen Lösungsmittel, wie Pyridin, Essigsäure, einem Aether, wie Tetrahydrofuran oder Dioxan, oder auch einem Alkohol, wie einem Niederalkanol, z.B. Methanol oder Aethanol, bei Temperaturen von etwa 0° bis etwa 100° durchgeführt, wobei gewöhnlich, bei erhöhten Temperaturen, d.h. bei etwa 60—90°, gearbeitet wird.

Bevorzugt wird eine Verbindung der Formel X mit Selendioxid in einem Lösungsmittel, wie Pyridin, bei etwa 80—90°C, oder auch gemäss der Deutschen Offenlegungsschrift Nr. 2 528 786, mit Natrium-; oder Kaliumnitrit in wässriger Salz-, Schwefel- oder Phosphorsäure oxidiert.

Die Oxidation von $\alpha$-Hydroxysäuren der Formel XI, worin die Aminogruppe in geschützter Form vorliegt, zu den entsprechenden $\alpha$-Ketosäuren der Formel- IX kann ebenfalls auf an sich bekannte, d.h. wie für die Oxidation von Hydroxy- zu Oxogruppen bekannte Weise durchgeführt werden. Als Oxidationsmittel kommen wiederum oxidierende Oxide, wie solche des Mangans, Chroms, Stickstoffs oder Schwefels, wie Mangandioxid, Chromtrioxid, z.B. Jones Reagens oder Chromtrioxid in Gegenwart von Essigsäure, Schwefelsäure oder Pyridin, Distickstofftetroxid, Dimethylsulfoxid gegebenenfalls in Gegenwart von Dicyclohexylcarbodiimid oder Sauerstoff, und Peroxide, wie Wasserstoffperoxid, sauerstoffhaltige Säuren, wie Permangansäure, Chromsäure oder Hypochlorsäure oder Salze davon, wie Kaliumpermanganat, Natrium- oder Kaliumdichromat oder Kaliumhypochlorit, in Frage. Die $\alpha$-Hydroxygruppe kann auch durch die Oppenauer-Oxidation in die $\alpha$-Oxogruppe übergeführt werden, d.h. durch Behandeln mit dem Salz eines sterisch gehinderten Alkohols, wie Aluminium- oder Kalium-tert.-butoxid-, -isopropoxid oder -phenoxid in Gegenwart eines Ketons, wie Aceton, Cyclohexanon oder Fluorenen. Eine weitere Möglichkeit die $\alpha$-Hydroxygruppe in die $\alpha$-Oxogruppe überzuführen besteht in der Dehydrierung, z.B. mit Raney-Nickel.

Die Oxidation wird je nach Oxidationsmittel in Wasser oder einem gegebenenfalls mit Wasser mischbaren, gegebenenfalls wasserhaltigen Lösungsmittel bei Temperaturen von etwa 0° bis etwa 100° durchgeführt.

Verbindungen der Formel XI sind bekannt oder können auf an sich bekannte Weise hergestellt werden.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit anorganischen oder organischen, festen oder flüssigen, pharmazeutisch verwendbaren Trägerstoffen enthalten, die sich vorzugsweise zur parenteralen Verabreichung eigenen.

Vorzugsweise verwendet man die pharmakologisch wirksamen Verbindungen der vorliegenden Erfindung in Form von injizierbaren, z.B. intravenös, verabreichbaren Präparaten oder von Infusionslösungen. Solche Lösungen sind vorzugsweise isotonische wässrige Lösungen oder Suspensionen, wobei diese z.B. aus lyophilisierten Präparaten, welche die Wiksubstanz allein oder zusammen mit

einem Trägermaterial, z.B. Mannit, enthalten, vor Gebrauch hergestellt werden können. Die pharmazeutischen Präparate können sterilisiert sein und/oder Hilfstoffe, z.B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Lösungs- oder Lyophilisierungsverfahren, hergestellt und enthalten von etwa 0,1% bis 100%, insbesondere von etwa 1% bis etwa 50%, Lyophilisate bis zu 100% des Aktivstoffes. Je nach Art der Infektion und Zustand des infizierten Organismus verwendet man tägliche Dosen von etwa 0,5 g bis etwa 5 g s.c. zur Behandlung von Warmblütern von etwa 70 kg Gewicht.

Die folgenden Beispiele dienen zur Illustration der Erfindung; Temperaturen werden in Celsiusgraden angegeben.

In der Dünnschichtchromatographie werden diefolgenden Systeme verwendet:

System 52A: n-Butanol-Eisessig-Wasser (67:10:23)
System 96: sec. Butanol-Eisessig-Wasser (67:10:23)
System 101: n-Butanol-Pyridin-Eisessig-Wasser (38:24:30)
System 101A: n-Butanol-Pyridin-Eisessig-Wasser (42:24:4:30)

Rf-Angaben für Dünnschichtchromatographie:
DS: Auf Silicagel-Fertigplatten SL 254 der Fa. Antec, Birsfelden.
DC: Auf Cellulose-Fertigplatten der Fa. Merck, Darmstadt.
In den Beispielen werden folgende Abkürzungen verwendet:
BOC tert.-Butyloxycarbonyl,
Z Carbobenzoxy.

## Beispiel 1

a) Ein Gemisch von 1,60 g 7$\beta$ - {2 - [4 - ((2R) - 2 - BOC - Amino - 2 - t.butoxycarbonyläthoxycarbonylamino)phenyl]acetylamino} - 3 - [(1 - methyl - 1H - tetrazol - 5 - yl) - thiomethyl] - 3-cephem - 4 - carbonsäure - diphenylmethylester, 1,0 g Anisol, 2,50 ml Methylenchlorid und 25 ml Trifluoressigsäure wird 1 Stunde bei Raumtemperatur unter Ausschluss von Luftfeuchtigkeit gerührt. Aus der anfänglich klaren Lösung fällt ein voluminöser Niederschlag aus. Am Ende der Reaktionszeit wird die Suspension auf ein eiskaltes Gemisch von Petroläther (300 ml) und Diäthyläther (150 ml) gegossen, das anfallende Trifluoracetat abgenutscht, mit Diäthyläther gewaschen und im Hochvakuum bei Raumtemperatur getrocknet.

Das rohe 7$\beta$ - {2 - [4 - ((2R) - 2 - Amino - 2 - carboxyäthoxycarbonylamino)phenyl]acetylamino}-3 - [(1 - methyl - 1H - tetrazol - 5 - yl) - thiomethyl] - 3 - cephem - 4 - carbonsäure - trifluoracetat wird in 25 ml Wasser aufgeschlämmt, durch Zusatz von 1N Natriumbicarbonatlösung ein pH-Wert von 6,0 eingestellt, die Suspension 15 Min. bei Raumtemperatur gerührt, dann das ungelöste Produkt (wenig) durch Behandlung mit Aktivkohle und Filtrieren durch Celite abgetrennt. Das klare Filtrat wird mit viel Aethanol versetzt, der pH-Wert auf 5,5 korrigiert und die Lösung am Rotationsverdampfer (Hochvakuum) bei 45° eingeengt. Die wässrige Lösung wird noch zweimal mit Aethanol eingedampft, schliesslich das 7$\beta$ - {2 - [4 - ((2R) - 2 - Amino - 2 - carboxyäthoxycarbonylamino)phenyl]acetylamino} - 3 - [(1 - methyl - 1H - tetrazol - 5 - yl) - thiomethyl] - 3 - cephem - 4 - carbonsäure - natriumsalz durch Zugabe von Aethanol gefällt, abgenutscht und sukzessive mit Aethanol, Aethanol-Diäthyläther (1:1) und Diäthyläther gewaschen. Das Produkt kann durch Lösen in Wasser und Eindampfen bzw. Fällen mit Aethanol weiter gereinigt werden. F. 185—190° (Zersetzung); $[\alpha]_D = + 26° \pm 1°$ (c = 3.409; in Wasser); DS: $Rf_{52A} = 0{,}05$; $Rf_{101} = 0{,}32$; $Rf_{101A} = 0{,}45$.

Das Ausgangsmaterial kann wie folgt erhalten werden:

b) (2R)-N-BOC-Serin-tert.-butylester wird aus (2R)-N-BOC-Serin (Smp. 89—90°, $\alpha_D = -2 \pm 1°$ (c = 3; in Methanol)) durch Umsetzung mit O-tert.-Butyl-isoharnstoff in Methylenchlorid [(E. Vowinkel, Chem. Ber. *100*, 16 (1967)] gewonnen. Smp. 75—76°. $[\alpha]_D = -8 \pm 1°$ (c = 5,2 in Chloroform).

c) Eine Lösung von 13,0 g (2R)-N-BOC-Serin-tert.-butylester in 125 ml Methylenchlorid wird auf 0° abgekühlt, mit 28 ml Phosgenlösung (20% in Toluol) versetzt und anschliessend unter Rühren und Kühlen bei + 5° bis + 10° ein Gemisch von 6 ml Pyridin und 45 ml Methylenchlorid innert 30 Minuten zugetropft. Die Suspension wird 90 Minuten im Eisbad gerührt und darauf eine Lösung von 12,0 g 4-Aminophenylessigsäure-benzylester in 65 ml Methylenchlorid und 6 ml Pyridin bei 0° bis + 5° innert 20 Minuten dazu getropft und das Reaktionsgemisch 3 Stunden bei Raumtemperatur gerührt. Die Suspension wird mit Methylenchlorid verdünnt, sukzessive mit Wasser, 10%-iger Zitronensäure, Wasser, 1N Natriumbicarbonatlösung und Wasser gewaschen. Die organische Phase wird über Natriumsulfat getrocknet und das Methylenchlorid am Rotationsverdampfer bei 50° abgedampft. Der erhaltene 4-((2R)-2-BOC-Amino-2-t.butoxycarbonyläthoxycarbonylamino)phenylessigsäure-benzylester an Kieselgel gereinigt und aus Petroläther-Aethylacetat (20:1) umkristallisiert. F. 74—76°; $[\alpha]_D = -22 \pm 1°$ (c = 2,85, in Chloroform).

d) Eine Lösung von 25,0 g 4-((2R)-2-BOC-Amino-2-t.butoxycarbonyläthoxycarbonylamino)phenylessigsäurebenzylester in 300 ml Aethylacetat wird in Gegenwart von 3,0 g Palladium auf Aktivkohle (10%) hydriert. Der Katalysator wird abfiltriert, mit Aethylacetat gewaschen, das Filtrat am Rotationsverdampfer bei 50° eingeengt und die 4-((2R)-2-BOC-Amino-2-t.butoxycarbonyläthoxycarbonylamino)phenylessigsäure durch Zusatz von Petroläther kristallisiert. F. 133—137°; $\alpha_D = -36 \pm 1°$ (c = 1,06, in Chloroform).

e) Eine Lösung von 5,0 g 7β - Amino - 3 - [(1 - methyl - 1H - tetrazol - 5 - yl) - thiomethyl] - 3-cephem - 4 - carbonsäure - diphenylmethylester und 4,50 g 4 - ((2R) - 2 - BOC - Amino - 2 - t.butoxycarbonyläthoxycarbonylamino)phenylessigsäure in einem Gemisch von 30 ml Tetrahydrofuran und 15 ml N,N-Dimethylformamid wird mit einem Eisbad auf +5° abgekühlt, eine Lösung von 2,0 g N,N'-Dicyclohexylcarbodiimid in 10 ml Tetrahydrofuran unter Rühren und Kühlen innert 20 Minuten zugetropft, dann das Eisbad entfernt und das Reaktionsgemisch bei Raumtemperatur gerührt. Nach 3 Stunden Reaktionszeit wird die Suspension mit weiteren 2,0 g festem N,N'-Dicyclohexylcarbodiimid versetzt. Nach insgesamt 6,5 Stunden Rühren wird die Suspension abgenutscht, das Nutschgut mit Tetrahydrofuran gewaschen, das Filtrat mit viel Aethylacetat versetzt und das Tetrahydrofuran durch Einengen am Rotationsverdampfer bei 45° entfernt. Die resultierende Lösung wird mit Aethylacetat verdünnt, sukzessive mit Wasser, 1N Natriumbicarbonatlösung und Wasser gewaschen, die organische Phase wird abgetrennt, über Natriumsulfat getrocknet und am Rotationsverdampfer bei 45° eingedampft. Der zurückbleibende Schaum wird durch Chromatographieren an der 20-fachen Menge Kieselgel gereinigt. Die Fraktionen mit Methylenchlorid-Methylacetat (7:1) und (5:1) als Eluiermittel werden vereinigt und der 7β - {2 - [4 - ((2R) - 2 - BOC - Amino - 2 - t.butoxycarbonyläthoxycarbonylamino)phenyl]acetylamino} - 3 - [(1 - methyl - 1H - tetrazol - 5 - yl) - thiomethyl]-3 - cephem - 4 - carbonsäure - diphenylmethylester aus wenig Aethylacetat kristallisiert. F. 155—160°. DS (Fliessmittel: Toluol-Chloroform-Aethylacetat-Aethanol-(16:16:16:1)): Rf = 0,51.

## Beispiel 2

a) Ein Gemisch von 7,20 g 7β - {2 - [4 - ((2S) - 2 - BOC - Amino - 2 - t.butoxycarbonyläthoxycarbonylamino)phenyl]acetylamino} - 3 - [(1 - methyl - 1H - tetrazol - 5 - yl) - thiomethyl] - 3 - cephem-4 - carbonsäure - diphenylmethylester, 3,0 ml Anisol, 7,0 ml Methylenchlorid und 70 ml Trifluoressigsäure wird 45 Minuten bei Raumtemperatur unter Ausschluss von Luftfeuchtigkeit gerührt. Aus der anfänglich klaren Lösung fällt ein voluminöser Niederschlag aus. Am Ende der Reaktionszeit wird die Suspension auf ein eiskaltes Gemisch von Petroläther (1000 ml) und Diäthyläther (500 ml) gegossen, das anfallende Trifluoracetat abgenutscht, mit Diäthyläther gewaschen und im Hochvakuum bei Raumtemperatur getrocknet.

Das rohe 7β - {2 - [4 - ((2S) - 2 - Amino - 2 - carboxyäthoxycarbonylamino)phenyl]acetylamino} - 3 - [(1 - methyl - 1H - tetrazol - 5 - yl) - thiomethyl] - 3 - cephem - 4 - carbonsäure - trifluoracetat wird in 40 ml Wasser aufgeschlämmt, durch Zusatz von 1N Natriumbicarbonatlösung ein pH-Wert von 6,0 eingestellt, die Suspension 15 Min. bei Raumtemperatur gerührt, dann das ungelöste Produkt (wenig) durch Behandlung mit Aktivkohle und Filtrieren durch Celit abgetrennt. Das klare Filtrat wird mit viel Aethanol versetzt, der pH-Wert auf 5,5 korrigiert und die Lösung am Rotationsverdampfer (Hochvakuum) bei 45° eingeengt. Die wässrige Lösung wird noch zweimal mit Aethanol eingedampft, schliesslich das 7β - {2 - [4 - ((2S) - 2 - Amino - 2 - carboxyäthoxycarbonylamino)phenyl]acetylamino} - 3 - [(1 - methyl - 1H - tetrazol - 5 - yl) - thiomethyl] - 3 - cephem-4 - carbonsäure - natriumsalz durch Zugabe von Aethanol gefällt, abgenutscht und sukzessive mit Aethanol, Aethanol-Diäthyläther (1:1) und Diäthyläther gewaschen. Das Produkt kann durch Lösen in Wasser und Eindampfen bzw. Fällen mit Aethanol weiter gereinigt werden. F. 218—225° (Zersetzung); $[\alpha]_D = + 23 \pm 1°$ (c = 3,002; in Wasser); DS: $Rf_{52A} = 0,05$; $Rf_{101} = 0,33$; $Rf_{101A} = 0,28$.

Das Ausgangsmaterial kann wie folgt erhalten werden:

b) (2S)-N-BOC-Serin-tert.-butylester wird aus (2S)-N-BOC-Serin (Smp. 85—95°, $\alpha_D = +3 \pm 1°$ (c = 3; in Methanol)) durch Umsetzung mit O-tert.-Butyl-isoharnstoff in Methylenchlorid [(E. Vowinkel, Chem. Ber. *100*, 16 (1967)] gewonnen. Smp. 95—103°. $[\alpha]_D = +7 \pm 1°$ (c = 4,96 in Chloroform).

c) Eine Lösung von 12,1 g (2S)-N-BOC-Serin-tert.-butylester in 125 ml Methylenchlorid wird auf 0° abgekühlt, mit 30 ml Phosgenlösung (20% in Toluol) versetzt und anschliessend unter Rühren und Kühlen bei + 5° bis +10° ein Gemisch von 5 ml Pyridin und 20 ml Methylenchlorid innert 30 Minuten zugetropft. Die Suspension wird 90 Minuten im Eisbad gerührt und darauf eine Lösung von 13,0 g 4-Aminophenylessigsäure-benzylester in 50 ml Methylenchlorid und 6 ml Pyridin bei 0° bis +5° innert 20 Minuten dazu getropft und das Reaktionsgemisch 3 Stunden bei Raumtemperatur gerührt. Die Suspension wird mit Methylenchlorid verdünnt, sukzessive mit Wasser, 10%-iger Zitronensäure, Wasser, 1N Natriumbicarbonatlösung und Wasser gewaschen. Die organische Phase wird über Natriumsulfat getrocknet und das Methylenchlorid am Rotationsverdampfer bei 50° abgedampft. Der erhaltene 4-((2S)-2-BOC-Amino-2-t.butoxycarbonyläthoxycarbonylamino)phenylessigsäure-benzylester an Kieselgel gereinigt und aus Petroläther-Aethylacetat (20:1) umkristallisiert. F. 74—75°; $[\alpha]_D = + 21 \pm 1°$ (c = 3,02, in Chloroform).

d) Eine Lösung von 13,0 g 4-((2S)-2-BOC-Amino-2-t.butoxycarbonyläthoxycarbonylamino)phenylessigsäure-benzylester in 200 ml Aethylacetat wird in Gegenwart von 1,50 g Palladium

auf Aktivkohle (10%) hydriert. Der Katalysator wird abfiltriert, mit Aethylacetat gewaschen, das Filtrat am Rotationsverdampfer bei 50° eingeengt und die 4-((2S)-2-BOC-Amino-2-t.butoxycarbonyläthoxycarbonylamino)phenylessigsäure durch Zusatz von Petroläther kristallisiert. F. 150—156°; $\alpha_D = +37 \pm 1°$ (c = 2,96, in Chloroform).

e) Eine Lösung von 7,50 g 7β - Amino - 3 - [(1 - methyl - 1H - tetrazol - 5 - yl) - thiomethyl]-3 - cephem - 4 - carbonsäure - diphenylmethylester und 7,00 g 4 - ((2S) - 2 - BOC - Amino - 2-t.butoxycarbonyläthoxycarbonylamino)phenylessigsäure in einem Gemisch von 50 ml Tetrahydrofuran and 15 ml N,N-Dimethylformamid wird mit einem Eisbad auf +5° abgekühlt, eine Lösung von 3,0 g N,N'-Dicyclohexylcarbodiimid in 20 ml Tetrahydrofuran unter Rühren und Kühlen innert 20 Minuten zugetropft, dann das Eisbad entfernt und das Reaktionsgemisch bei Raumtemperatur gerührt. Nach 3 Stunden Reaktionszeit wird die Suspension mit weiteren 2,0 g festem N,N'-Dicyclohexylcarbodiimid versetzt. Nach insgesamt 6,5 Stunden Rühren wird die Suspension abgenutscht, das Nutschgut mit Tetrahydrofuran gewaschen, das Filtrat mit viel Aethylacetat versetzt und das tetrahydrofuran durch Einengen am Rotationsverdampfer bei 45° entfernt. Die resultierende Lösung wird mit Aethylacetat verdünnt, sukzessive mit Wasser, 1N Natriumbicarbonatlösung und Wasser gewaschen, die organische Phase wird abgetrennt, über Natriumsulfat getrocknet und am Rotationsverdampfer bei 45° eingedampft. Der zurückbleibende Schaum wird durch Chromatographieren an der 20-fachen Menge Kieselgel gereinigt. Die Fraktionen mit Methylenchlorid-Methylacetat (7:1) und (5:1) als Eluiermittel werden vereinigt und der 7β - {2 - [4 - ((2S) - 2 - BOC - Amino - 2 - t.butoxycarbonyläthoxycarbonylamino)phenyl]acetylamino} - 3 - [(1 - methyl - 1H - tetrazol - 5 - yl) - thiomethyl] - 3 - cephem - 4 - carbonsäure - diphenylmethylester aus wenig Aethylacetat kristallisiert. F. 125—132°. DS (Fliessmittel: Toluol-Chloroform-Aethylacetat-Aethanol-(16:16:16:1)): Rf = 0,50.

Beispiel 3

a) Ein Gemisch von 3,7 g 7β - {2 - [4 - ((2R) - 2 - BOC - Amino - 2 - t.butoxycarbonyläthoxycarbonylamino)phenyl]acetylamino} - 3 - carbamoyloxymethyl - 3 - cephem - 4 - carbonsäure-diphenylmethylester, 3,0 ml Anisol, 4,0 ml Methylenchlorid und 40 ml Trifluoressigsäure wird 1 Stunde bei Raumtemperatur unter Ausschluss von Luftfeuchtigkeit gerührt. Darauf wird die Suspension auf ein eiskaltes Gemisch von Petroläther (800 ml) und Aether (400 ml) gegossen, das anfallende Trifluoracetat abgenutscht, mit Diäthyläther gewaschen und bei Raumtemperatur im Hochvakuum getrocknet.

Das 7β - {2 - [4 - ((2R) - 2 - Amino - 2 - carboxyäthoxycarbonylamino)phenyl]acetylamino}-3 - carbamoyloxymethyl - 3 - cephem - 4 - carbonsäure - natriumsalz wird aus dem obigen Trifluoracetat nach dem Verfahren von Beispiel 1 gewonnen. Smp. 237—242° (Zersetzung). DS:$Rf_{52A}$ = 0,07; $Rf_{101}$ = 0,30.

Das Ausgangsmaterial kann wie folgt hergestellt werden:

b) Eine Lösung von 5,0 g 7β - Amino - 3 - carbamoyloxy - 3 - cephem - 4 - carbonsäure-diphenylmethylester und 4,9 g 4 - ((2R) - 2 - BOC - Amino - 2 - t.butoxycarbonyläthoxycarbonylamino)phenylessigsäure in 50 ml Tetrahydrofuran wird mit einer Lösung von 2,8 g N,N'-Dicyclohexylcarbodiimid in 10 ml Tetrahydrofuran bzw. mit 1,5 g festem N,N'-Dicyclohexylcarbodiimid nach dem Verfahren von Beispiel 1 umgesetzt. Das Rohprodukt wird durch Chromatographieren an der 30-fachen Menge Kieselgel gereinigt. Die Fraktionen mit Methylenchlorid-Methylacetat (7:3) und (3:2)0 als Eluiermittel werden vereinigt und der 7β - {2[4 - ((2R) - 2 - BOC - Amino - 2 - t.butoxycarbonyläthoxycarbonylamino)phenyl]acetylamino} - 3 - carbamoyloxymethyl - 3 - cephem - 4 - carbonsäure - diphenylmethylester aus Aethylacetatlösung mit einem Gemisch von Petroläther und Aether gefällt. F. 95—114° (langsame Zersetzung); DS (Fliessmittel: Aethylacetat-Toluol-Aethanol-(47:47:5)): Rf = 0,34.

Die gemäss Beispiel 5a) erhaltene Verbindung kann auch wie folgt hergestellt werden:

ai) Eine Suspension von 24 g 7β - {2 - [4 - ((2R) - 2 - BOC - Amino - 2 - diphenylmethoxycarbonyläthoxycarbonylamino)phenyl]acetylamino} - 3 - carbamoyloxymethyl - 3 - cephem - 4 - carbonsäurediphenylmethylester in 130 ml Methylenchlorid und 6,6 ml Anisol wird bei 0° mit 130 ml Trifluoressigsäure versetzt und die resultierende klare Lösung 45 Minuten bei 0° gerührt.

Durch Zugabe von 740 ml Diäthyläther-Hexan 1:1 wird das Trifluoracetat der 7β - {2 - [4 - ((2R)-2 - Amino - 2 - carboxyäthoxycarbonylamino)phenyl]acetylamino} - 3 - carbamoyloxymethyl - 3-cephem - 4 - carbonsäure ausgefällt. Der Niederschlag wird abfiltriert, in 140 ml Wasser gelöst und die Lösung durch Zugabe von 1N Natriumbicarbonat auf pH 5,5 gestellt und filtriert. Das im Vakuum eingedampfte klare Filtrat gibt einen Rückstand, der mit Aethanol zu einem nahezu farblosen Pulver digeriert wird, das abgenutscht und getrocknet wird. Man erhält das Natriumsalz der 7β - {2 - [4 - ((2R) - 2 - Amino - 2 - carboxyäthoxycarbonylamino)phenyl]acetylamino} - 3-carbamoyloxymethyl - 3 - cephem - 4 - carbonsäure; $[\alpha]_D^{20} = +110° \pm 1°$ (c = 3, $H_2O$); $[\alpha]_D^{20} = +90° \pm 1°$ (c = 3, 0,1 N Natriumbicarbonat). DC: $Rf_{101}$ = 0,38.

Das Ausgangsmaterial kann wie folgt erhalten werden:

c) Eine Suspension von 100 g (2R)-Serin in 1 l Wasser wird durch langsame Zugabe von 100 g festem, wasserfreiem Natriumcarbonat in Lösung gebracht, dann versetzt man mit 2 l Dioxan sowie 346 g Di-tert.-butyl-pyrocarbonat und rührt 30 Minuten bei +20°. Das Dioxan wird im Vakuum abgedampft und die wässrige Phase bei 0° und pH 2,0 mit Essigester extrahiert. Man erhält 236,7 g

amorphes, rohes (2R)-N-BOC-Serin, das ca. 195 g reines Produkt enthält. Beim Verreiben mit Petroläther und Kühlen kann daraus die reine Verbindung erhalten werden; F. 78—83° (Zersetzung).

d) Zu einer Lösung von 236,7 g des erhaltenen rohen (2R)-BOC-Serins in 700 ml Methylenchlorid tropft man bei 22° unter Kühlen eine Lösung von ca. 214 g Diphenyldiazomethan in 1 Liter Methylenchlorid und rührt $1\frac{1}{2}$ Stunden bei 20°. Das Gemisch wird im Vakuum eingedampft, der Rückstand in Aethylacetat aufgenommen und bei 0° mit Phosphat-Puffer von pH 2,0 dann von pH 7,0 gewaschen. Die getrocknete, organische Phase gibt beim Eindampfen einen kristallinen Rückstand, der in Hexan digeriert wird und man erhält nach Filtrieren den (2R)-N-BOC-serin-diphenylmethylester; F. 116—117° (korr.). $[\alpha]_D^{20} = +6° \pm 1°$ (c = 1, Chloroform).

e) Eine Suspension von 46,4 g (2R)-N-BOC-serindiphenylmethylester in 340 ml absolutem Methylenchlorid wird bei 0° zu einer Mischung von 62 ml 20%-igem Phosgen in Toluol und 10 ml Pyridin getropft und 1 Stunde bei 0° gerührt (Lösung A).

Eine Suspension von 18,9 g p-Aminophenylessigsäure in 75 ml Pyridin wird mit 37,5 ml Trimethylchlorsilan versetzt, wobei unter Selbsterwärmung auf 33° eine klare Lösung entsteht. Nach 15 Minuten Rühren verdünnt man mit 70 ml Methylenchlorid und rührt weitere 15 Minuten (Lösung B).

Bei 0° wird die Lösung B innerhalb 3 Minuten zur Lösung A getropft und anschliessend 30 Minuten bei 0° sowie 30 Minuten bei +20° gerührt. Das Gemisch wird auf 1,3 Liter 0,3-m. Phosphatpuffer pH 7 gerührt und bei pH 6 mit Essigester extrahiert. Die getrocknete und eingedampfte organische Phase wird mit Toluol digeriert und ergibt nach Filtration die 4-((2R)-2-BOC-Amino-2-diphenylmethoxycarbonyläthoxycarbonylamino)phenylessigsäure; F. 165—166° (korr.), $[\alpha]_D^{20} = +2° \pm 1°$ (c = 1, Chloroform).

f) Eine Suspension von 38,4 g 4-((2R)-2-BOC-Amino-2-diphenylmethoxycarbonyläthoxycarbonylamino)phenylessigsäure und 7,1 g N-Methylmorpholin in 500 ml absolutem Methylenchlorid wird bei 0° mit 8,8 g Chlorameisensäureisobutylester versetzt und anschliessend 90 Minuten bei 0° gerührt. Diese Lösung des Anhydrids tropft man bei 0° zueiner Lösung von 35,9 g 3-Carbamoyloxymethyl-7-ammonium-3-cephem-4-carbonsäure-diphenylmethylester-tosylat und 7,1 N-Methylmorpholin in 300 ml Methylenchlorid und rührt 45 Minuten bei 22°. Der Ansatz wird zu 600 ml 0,5 m. Dikaliumhydrogenphosphat-Puffer gerührt. Nach Abdestillieren des Methylenchlorids im Vakuum extrahiert man in der Kälte mit Aethylacetat. Die organische Phase wird mit Phosphat-Puffer von pH 2,0 gewaschen, getrocknet, im Vakuum eingeengt und ergibt den 7β-{2-[4 - ((2R) - 2 - BOC - Amino - 2 - diphenylmethoxycarbonyläthoxycarbonylamino)phenyl]acetylamino}-3 - carbamoyloxymethyl - 3 - cephem - 4 - carbonsäure - diphenylmethylester; F. 167—169°; $[\alpha]_D^{20} = +46° \pm 1°$ (c = 3, Dimethylsulfoxyd).

Beispiel 4

a) Ein Gemisch von 2,2 g 7β-{2-[4-((5R,S)-5-BOC-Amino-5-t.butoxycarbonylphenylaminocarbonylamino)phenyl]acetylamino}-cephalosporansäure-diphenylmethylester, 1,9 g Anisol, 15 ml Methylenchlorid und 120 ml Trifluoressigsäure wird 1 Stunde bei Raumtemperatur unter Ausschluss von Luftfeuchtigkeit gerührt. Darauf wird die Suspension auf ein eiskaltes Gemisch von Petroläther (600 ml) und Aether (300 ml) gegossen, das anfallende Trifluoracetat abgenutscht, mit Aether gewaschen und bei Raumtemperatur im Hochvakuum getrocknet.

Das 7β - {2 - [4 - ((5R,S) - 5 - Amino - 5 - carboxypentylaminocarbonylamino)phenyl]acetylamino} - cephalosporansäure-natriumsalz wird aus dem obigem Trifluoracetat nach dem Verfahren von Beispiel 1 erhalten. F. 248—255° (Zersetzung); DS: $Rf_{52A} = 0{,}05$; $Rf_{101} = 0{,}32$; $Rf_{101A} = 0{,}47$.

Das Ausgangsmaterial kann wie folgt hergestellt werden:

b) Der ölige (2R,S)-$N^2$-BOC-$N^6$-Z-Lysin-tert.-butylester wird aus (2R,S)-$N^2$-BOC-$N^6$-Z-Lysin durch Umsetzung mit O-tert.-Butyl-isoharnstoff in Methylenchlorid (E. Vowinkel, Chem. Ber. *100*, 16 (1967)) gewonnen. Das Rohprodukt wird durch Chromatographieren an der 20-fachen Kieselgel gereinigt und die Fraktionen mit Toluol-Aethylacetat (5:1) als Eluiermittel vereinigt. DS (Fliessmittel: Toluol-Aethylacetat (2:1)). Rf = 0,56.

c) Eine Lösung von 30,0 g (2R,S)-$N^2$-BOC-$N^6$-Z-Lysin-tert.-butylester in 300 ml Methanol wird in Gegenwart von 4,0 g Palladium auf Aktivkohle (10%) hydriert. Nach ca. 4 Stunden kommt die Wasserstoffaufnahme zum Stillstand. Der Katalysator wird abfiltriert, mit Methanol gewaschen und das Filtrat am Rotationsverdampfer bei 50° eingedampft. Der (2R,S)-$N^2$-BOC-Lysin-tert.-butylester ist ölig.

d) Ein Gemisch von 66 ml Phosgenlösung (20% in Toluol) und 450 ml Methylenchlorid wird mit einem Eisbad auf 0° abgekühlt und unter Rühren und Kühlen bei + 5 bis + 8° eine Lösung von 36,0 g (2R,S)-$N^2$-BOC-Lysin-tert.-butylester in 180 ml Methylenchlorid und 10 ml Pyridin innert 1 Stunde zugetropft. Die Suspension wird 1 Stunde im Eisbad gerührt und darauf eine Lösung von 26,1 g 4-Aminophenylessigsäure-benzylester in 280 ml Methylenchlorid und 22 ml Pyridin bei 0° bis +5° dazu getropft. Das Reaktionsgemisch wird 2 Stunden bei Raumtemperatur gerührt. Die Suspension wird mit Methylenchlorid verdünnt und sukzessive mit Wasser, 10%-iger Zitronensäure, Wasser, 1N Natriumbicarbonatlösung und Wasser gewaschen. Die organische Phase wird über Natriumsulfat getrocknet und das Lösungsmittel am Rotationsverdampfer bei 50° abgedampft. Das Rohprodukt wird durch Chromatographieren an der 25-fachen Menge Kieselgel gereinigt und die mit Methylenchlorid-Methylacetat (4:1) als Eluiermittel erhaltenen Fraktionen vereinigt. Der erhaltene 4-((5R,S)-5-BOC-Amino-5-t.butoxycarbonylpentylaminocarbonylamino)phenylessigsäure-benzylester ist ölig. DS

(Fliessmittel: Toluol-Chloroform-Aethylacetat-Aethanol-(3:3:3:0,3)): Rf: 0,32.

e) Eine Lösung von 7,60 g 4-((5R,S)-5-BOC-Amino-5-t.butoxycarbonylpentylaminocarbonylamino)phenylessigsäurebenzylester in 200 ml Aethylacetat wird in Gegenwart von 1,0 g Palladium auf Aktivkohle (10%) hydriert. Nach ca. 2 Stunden ist die berechnete Wasserstoffmenge aufgenommen. Der Katalysator wird abfiltriert, mit Aethylacetat gewaschen, das Filtrat am Rotationsverdampfer bei 45° eingeengt und die 4-((5R,S)-5-t.butoxycarbonylpentylaminocarbonylamino)phenylessigsäure durch Zugabe von Petroläther kristallisiert. F: 121—124°.

f) Eine Lösung von 4,40 g 7$\beta$ - Amino - cephalosporansäure - diphenylmethylester und 4,80 g 4 - ((5R,S) - 5 - BOC - Amino - 5 - t.butoxycarbonylpentylaminocarbonylamino)phenylessigsäure in 40 ml Tetrahydrofuran wird mit einer Lösung von 2,0 g N,N' - Dicyclohexylcarbodiimid in 10 ml Tetrahydrofuran bzw. mit 1,0 g festem N,N' - Dicyclohexylcarbodiimid nach dem Verfahren von Beispiel 1 umgesetzt. Das Rohprodukt wird durch Chromatographieren an der 30-fachen Menge Kieselgel gereinigt. Die Fraktionen mit Methylenchlorid-Methylacetat (1:1) werden vereinigt und der 7$\beta$ - {2 - [4 - ((5R,S) - 5 - BOC - Amino - 5 - t.butoxycarbonylpentylaminocarbonylamino)phenyl]-acetylamino} - cephalosporansäure - diphenylmethylester aus Aethylacetatlösung mit einem Gemisch von Petroläther und Aether gefällt. F. 120—128° (Zersetzung). DS (Fliessmittel: Toluol-Aethanol-(12:1)) Rf = 0,17.

Beispiel 5

a) Ein Gemisch von 1,60 g 7$\beta$ - {2 - [4 - ((3R) - 3 - BOC - Amino - 3 - t.butoxycarbonylpropionylamino)phenyl]acetylamino} - 3 - [(1 - methyl - 1H - tetrazol - 5 - yl) - thiomethyl] - 3 - cephem - 4 - carbonsäurediphenylmethylester, 1,0 g Anisol, 2,50 ml Methylenchlorid und 25 ml Trifluoressigsäure wird 40 Minuten bei Raumtemperatur unter Ausschluss von Luftfeuchtigkeit gerührt. Die erhaltene Suspension wird auf ein eiskaltes Gemisch von Petroläther (400 ml) und Diäthyläther (200 ml) gegossen, das anfallende Trifluoracetat abgenutscht, mit Diäthyläther gewaschen und bei Raumtemperatur im Hochvakuum getrocknet.

Das 7$\beta$ - {2 - [4 - ((3R) - 3 - Amino - 3 - carboxypropionylamino) - phenyl]acetylamino} - 3 -[(1-methyl - 1H - tetrazol - 5 - yl) - thiomethyl] - 3 - cephem - 4 - carbonsäure - natriumsalz wird aus dem Trifluoracetat nach den Verfahren von Beispiel 1 erhalten. F. 248—252° (Zersetzung). DS: $Rf_{52A}$ = 0,09, $Rf_{101}$ = 0,33, $Rf_{101A}$ = 0,28.

Das Ausgangsmaterial kann wie folgt hergestellt werden:

b) (2R) - N - BOC - Asparaginsäure - $\alpha$ - tert. - butylester wird durch Verseifen von (2R) - N - BOC-Asparaginsäure - ($\beta$ - methylester) - $\alpha$ - tert.-butylester [F. 50—51° (Zersetzung); $[\alpha]_D = -17 \pm 1°$ (c = 2,93 in Chloroform)] mit 1 N Natriumhydroxid (P. M. Hardy et al., J. Chem. Soc., Perkin. Trans. 1, 605 (1972)) in Aceton erhalten. F. 96—100° (Zersetzung); $[\alpha]_D = -15 \pm 1°$ (c = 2,88 in Chloroform).

c) Eine Lösung von 10,0 g (2R) - N - BOC - Asparaginsäure - $\alpha$ - tert.-butylester und 8,35 g 4 - Aminophenylessigsäurebenzylester in 125 ml Tetrahydrofuran wird mit einem Eisbad auf +5° abgekühlt, eine Lösung von 7,15 g N,N' - Dicyclohexylcarbodiimid in 30 ml Tetrahydrofuran unter Rühren und Kühlen innert 20 Minuten zugetropft, dann das Eisbad entfernt und das Reaktionsgemisch bei Raumtemperatur gerührt. Nach 3 Stunden Reaktionszeit wird das Reaktionsgemisch mit 4,0 g festem N,N'-Dicyclohexylcarbodiimid versetzt. Nach insgesamt 7 Stunden Rühren wird die Suspension abgenutscht, das Nutschgut mit Aethylacetat gewaschen, das Filtrat mit viel Aethylacetat versetzt und das Tetrahydrofuran durch Einengen am Rotationsverdampfer bei 45° entfernt. Die erhaltene Lösung wird mit Aethylacetat verdünnt und sukzessive mit Wasser, 1N Natriumbicarbonatlösung und Wasser gewaschen. Die organische Phase wird über Natriumsulfat getrocknet und am Rotationsverdampfer bei 45° eingedampft. Der erhaltene 4-((3R)-3-BOC-Amino-3-t.butoxycarbonylpropionylamino)-phenylessigsäure-benzylester wird aus einem Gemisch von Benzol und Petroläther umkristallisiert. F. 130—133° (Zersetzung); $[\alpha]_D = -15 \pm 1°$ (c = 1,51 in Chloroform).

d) Eine Lösung von 12,2 g 4-((3R)-3-BOC-Amino-3-t.butoxycarbonylpropionylamino)phenylessigsäure-benzylester in einem Gemisch von Aethylacetat (360 ml) und Aethanol (40 ml) wird in Gegenwart von 4,0 g Palladium auf Aktivkohle (10%) bei Raumtemperatur hydriert. Nach ca. 1 Stunde ist die berechnete Wasserstoffmenge aufgenommen. Der Katalysator wird abfiltriert, mit einem Gemisch von Aethylacetat-Aethanol-(9:1) gewaschen und das Filtrat am Rotationsverdampfer bei 45° eingedampft. Die 4-((3R)-3-BOC-Amino-3-t.butoxycarbonylpropionylamino)-phenylessigsäure wird durch Vermischen mit Petroläther gefällt. F. 160—166° (Zersetzung); $[\alpha]_D = +2 \pm 1°$ (c = 1,92, in Methanol).

e) Eine Lösung von 5,0 g 7$\beta$ - Amino - 3 - [(1 - methyl - 1H - tetrazol - 5 - yl) - thiomethyl] - 3-cephem - 4 - carbonsäure - diphenylmethylester und 4,40 g 4 - ((3R) - 3 - BOC - Amino - 3 - t.butoxycarbonylpropionylamino)phenylessigsäure in einem Gemisch von 30 ml Tetrahydrofuran und 10 ml N,N - Dimethylformamid wird mit einer Lösung von 2,5 g N,N' - Dicyclohexylcarbodiimid in 10 ml Tetrahydrofuran bzw. 2,0 g festem N,N' - Dicyclohexylcarbodiimid nach dem Verfahren von Beispiel 1 umgesetzt. Das Rohprodukt wird durch Chromatographieren an der 20-fachen Menge Kieselgel gereinigt. Die Fraktionen mit Methylenchlorid - Methylacetat (4:1) werden vereinigt und der 7$\beta$ - {2 - [4 - ((3R) - 3 - BOC - Amino - 3 - t.butoxycarbonylpropionylamino)phenyl]acetylamino} - 3 - [(1 - methyl - 1H - tetrazol - 5 - yl) - thiomethyl] - 3 - cephem - 4 - carbonsäure - diphenylmethylester aus Aethylacetat kristallisiert. F. 155—160° (Zersetzung); DS (Fliessmittel: Toluol-Aethylacetat - Chloroform - Aethanol - (16:16:16:1)): Rf = 0,26.

Beispiel 6

a) Eine Aufschlämmung von 1,07 g (1 mMol) 7β - {2 - [5 - ((2R) - 2 - BOC - Amino - 2 - diphenylmethoxycarbonyl - äthoxycarbonylaminomethyl) - 2 - furyl] - 2 - syn - methoximinoacetylamino} - 3 - [(1 - methyl - 1H - tetrazol - 5 - yl) - thiomethyl] - 3 - cephem - 4 - carbonsäure - diphenylmethylester in 3 ml Anisol wird auf 0° gekühlt, mit 12 ml Trifluoressigsäure versetzt und während 20 Minuten bei 0° gerührt. Anschliessend wird zweimal unter Zusatz von Toluol im Vakuum eingedampft. Der Rückstand, enthaltend das Trifluoracetat des Endproduktes, wird in Aethylacetat/Wasser aufgenommen und die organische Phase abgetrennt. Die wässrige Phase wird mit 1N Natronlauge auf pH 6,1 eingestellt, im Vakuum auf ca. 1,5 ml eingeengt und mit 60 ml Aethanol versetzt, worauf das Natriumsalz der 7β - 2 - [5 - ((2R) - 2 - Amino - 2 - carboxyäthoxycarbonylaminomethyl) - 2 - furyl] - 2 - syn - methoximinoacetylamino} - 3 - [(1 - methyl - 1H-tetrazol - 5 - yl) - thiomethyl] - 3 - cephem - 4 - carbonsäure ausfällt. DS: $Rf_{52A}$ = 0,05.

Das Ausgangsmaterial kann wie folgt hergestellt werden:

b) Zu 50 g (0,51 Mol) Furfurylamin werden unter Eiskühlung 150 ml Trifluoressigsäure getropft. Die Lösung wird auf 45° erwärmt, tropfenweise mit 150 ml Acetanhydrid versetzt und anschliessend 3 Stunden bei 50°—55° und über Nacht bei Raumtemperatur gerührt. Die Mischung wird im Vakuum eingeengt und noch zweimal unter Zusatz von Toluol im Vakuum eingedampft. Das kristallisierende 2-Acetyl-5-trifluoracetylaminomethyl-furan wird abfiltriert und mit Diäthyläther gewaschen. F. 96°; IR-Spektrum (in $CHCl_3$): Absorptionsbanden bei 2,90; 3,03; 5,78; 5,97; 6,57 μ.

c) Eine Lösung von 100 g (0,42 Mol) 2-Acetyl-5-trifluoracetylaminomethylaminomethylfuran und 72 g (0,65 Mol) Selendioxid in 300 ml Pyridin wird vorsichtig unter gutem Rühren auf 85° erwärmt und bei 90° während 3 Stunden gerührt. Dann wird durch Celite filtriert, das Filtrat im Vakuum eingeengt, der Rückstand in Aethylacetat aufgenommen und mit verdünnter Schwefelsäure und gesättigter Natriumchloridlösung gewaschen. Nach Trocknen der organischen Phase über Natriumsulfat wird zur Trockene eingeengt und die erhaltene 2-(5-Trifluoracetylaminomethyl-2-furyl)-2-oxoessigsäure durch Chromatographie an Silicagel (System Methylenchlorid/Aethylacetat 9:1) gereinigt. IR-Spektrum (Dioxan): Absorptionsbanden bei 3,08; 5,67; 5,81; 5,98; 6,45 μ; DS: $Rf_{52A}$ = 0,35; F. 126—128°.

d) Eine Suspension von 27 g (102 mMol) 2-(5-Trifluoracetylaminomethyl-2-furyl)-2-oxoessigsäure in 250 ml Methanol wird mit 8,07 ml Pyridin (102 mMol) und 8,5 g O-Methylhydroxylamin-hydrochlorid (102 mMol) versetzt. Die erhaltene Lösung wird 1½ Stunden bei Raumtemperatur unter Stickstoff gerührt, anschliessend im Vakuum zur Trockene eingeengt, der Rückstand in Aethylacetat aufgenommen und die Lösung mit verdünnter Schwefelsäure und gesättigter Natriumchloridlösung gewaschen. Die organische Phase wird über Natriumsulfat getrocknet und eingeengt. Die 2-(5-Trifluoracetylaminomethyl-2-furyl)-2-syn-methoximinoessigsäure wird als teilweise kristallisierender Schaum isoliert (über 85% syn-Isomeres). Nach Umkristallisation aus Aethylacetat/Hexan erhält man das reine syn-Isomere. F. 115—117°; IR-Spektrum (Nujol): Absorptionsbanden bei 3,2; 5,67; 5,82; 6,36 μ; DS: Rf = 0,41 (n-Butanol:Essigsäure:Wasser = 67:10:23).

e) Eine Lösung von 31 g (102 mMol) 2 - (5 - Trifluoracetylaminomethyl - 2 - furyl) - 2 - syn-methoximinoessigsäure in 150 ml Wasser und 200 ml Dioxan wird mit 27 ml (202 mMol) 7,5 N Natronlauge bei Raumtemperatur während 1 Stunde gerührt. Die Lösung wird auf pH 6 gestellt und im Vakuum auf 30 ml eingeengt. Das kristallisierte Zwitterion der 2 - (5 - Aminomethyl - 2 - furyl)-2 - syn - methoximinoessigsäure wird abfiltriert und mit Aceton gewaschen. F. 230° (Zersetzung) IR-Spektrum (Nujol): Absorptionsbanden bei 3,25; 6,12; 6,29; 6,52 μ; DS: $Rf_{52A}$ = 0,1.

f) Eine Lösung von 560 mg (1,5 mMol) (2R)-N-BOC-Serin-diphenylmethylester (hergestellt aus (2R)-N-BOC-Serin und Diphenyldiazomethan) in 10 ml Methylenchlorid wird auf 0° gekühlt und nacheinander mit 0,12 ml (1,5 mMol) Pyridin und 0,72 ml Phosgen (20%-ig in Toluol) versetzt. Die Lösung lässt man bei 0° unter Stickstoff während 1 Stunde Rühren (Lösung I).

Eine Suspension von 250 mg (1,25 mMol) 2-(5-Aminomethyl-2-furyl)-2-syn-methoximinoessigsäure in 1 ml Acetonitril wird mit 0,93 ml Bis-trimethylsilylacetamid (3,75 mMol) versetzt und 3/4 Stunden bei Raumtemperatur unter Stickstoff gerührt (Lösung II).

Lösung II wird nun zu Lösung I pipettiert und nach Zufügen von 0,2 ml (1,4 mMol) Triäthylamin 1½ Stunden gerührt. Nach Schütteln mit 2 N Schwefelsäure und Kochsalzlösung wird die organische Phase über Natriumsulfat getrocknet und im Vakuum eingeengt. Der ölige Rückstand wird an Silicagel (System Methylenchlorid/Aethylacetat 9:1) chromatographiert.

Die 2-[5-((2R)-2-BOC-Amino-2-diphenylmethoxycarbonyläthoxycarbonylaminomethyl)-2-furyl]-2-syn-methoximinoessigsäure wird als Schaum isoliert. IR-Spektrum (in $CHCl_3$): Absorptionsbanden bei 2,94; 5,78; 5,84; 5,95; 6,15; 6,66 μ. DS:$Rf_{52A}$ = 0,53.

g) Eine Lösung von 10 g (16,8 mMol) 2-[5-((2R)-2-BOC-Amino-2-diphenylmethoxycarbonyläthoxycarbonylaminomethyl)-2-furyl]-2-syn-methoximinoessigsäure in 50 ml Methylenchlorid wird auf

—20° gekühlt, mit 1,87 ml (16,8 mMol) N-Methylmorpholin und 1,9 ml (14 mMol) Chlorameisensäureisobutylester versetzt. Nach $1\frac{1}{2}$ stündiger Reaktionsdauer bei —15° werden 6,92 g 7β - Amino - 3[(1 - methyl - 1H - tetrazol - 5 - yl) - thiomethyl] - 3 - cephem - 4 - carbonsäure - diphenylmethylester zugefügt und die Lösung auf 0° gebracht Nach 3 Stunden wird die Reaktionsmischung nacheinander mit 2N Schwefelsäure, verdünnter wässriger Natriumbicarbonatlösung und konzentrierter, wässriger Natriumchloridlösung gewaschen, über Natriumsulfat getrocknet, filtriert und eingedampft. Durch Chromatographie des Rückstandes an Silicagel (System Toluol/Aethylacetat 3:1) wird der 7β - {2 - [5 - ((2R) - 2 - BOC - Amino - 2 - diphenylmethoxycarbonyläthoxycarbonylaminomethyl) - 2 - furyl - 2 - syn - methoximinoacetylamino} - 3 - [(1 - methyl - 1H - tetrazol - 5 - yl) - thiomethyl] - 3 - cephem - 4 - carbonsäure - diphenylmethylester rein isoliert. IR - Spektrum ($CHCl_3$): Absorptionsbanden bei 2,92; 5,61, 5,74; 5,84; 6,66 μ; DS:Rf = 0,41 (Toluol/Aethylacetat 1:1).

Beispiel 7

a) Eine Aufschlämmung von 1,06 g (1 mMol) 7β - {2 - [5 - ((2R) - 2 - BOC - Amino - 2 - diphenylmethoxycarbonyl - äthoxycarbonylaminomethyl) - 2 - thienyl] - 2 - syn - methoximinoacetylamino}-3 - acetoxymethyl - 3 - cephem - 4 - carbonsäure - diphenylmethylester in 3 ml Anisol wird auf 0° gekühlt, mit 12 ml Trifluoressigsäure versetzt und während 20 Minuten bei 0° gerührt. Anschliessend wird zweimal unter Zusatz von Toluol im Vakuum eingedampft. Der Rückstand, enthaltend das Trifluoracetat des Endproduktes, wird in Aethylacetat/Wasser aufgenommen und die organische Phase abgetrennt. Die wässrige Phase wird mit 1N Natronlauge auf pH 6,1 eingestellt, im Vakuum auf ca. 1,5 ml eingeengt und mit 60 ml Aethanol versetzt, worauf das Natriumsalz der 7β - {2 - [5 - ((2R) - 2-Amino - 2 - carboxyäthoxycarbonylaminomethyl) - 2 - thienyl] - 2 - syn - methoximino - acetylamino}-3 - acetoxymethyl - 3 - cephem - 4 - carbonsäure ausfällt. DS: $Rf_{52A}$ = 0,05.

Das Ausgangsmaterial kann wie folgt hergestellt werden:

b) Eine Lösung von 100 g (0,40 Mol) 2 - Acetyl - 5 - trifluoracetylaminomethylthiophen (herstellbar analog Beispiel 9b) aus 2 - Aminomethylthiophen, Trifluoressigsäure und Acetanhydrid) und 72 g (0,65 mMol) Selendioxid in 300 ml Pyridin wird vorsichtig unter gutem Rühren auf 85° erwärmt und bei 90° während 3 Stunden gerührt. Dann wird durch Celite filtriert, das Filtrat im Vakuum eingeengt, der Rückstand in Aethylacetat aufgenommen und mit verdünnter Schwefelsäure und gesättigter Natriumchloridlösung gewaschen. Nach Trocknen der organischen Phase über Natriumsulfat wird zur Trockne eingeengt und die erhaltene 2 - (5 - Trifluoracetylaminomethyl - 2-thienyl) - 2 - oxoessigsäure durch Chromatographie an Silicagel (System Methylenchlorid/Aethylacetat 9:1) gereinigt. IR-Spektrum (Dioxan): Absorptionsbanden bei 3,08; 5,67; 5,81; 5,90; 6,45 μ; DS: $Rf_{52A}$ = 0,35.

c) Eine Suspension von 28 g (100 mMol) 2 - (5 - Trifluoracetylaminomethyl - 2 - thienyl) - 2-oxoessigsäure in 250 ml Methanol wird mit 8,07 ml Pyridin (102 mMol) und 8,5 g O-Methylhydroxylamin-hydrochlorid (102 mMol) versetzt. Die erhaltene Lösung wird $1\frac{1}{2}$ Stunden bei Raumtemperatur unter Stickstoff gerührt, anschliessend im Vakuum zur Trockene eingeengt, der Rückstand in Aethylacetat aufgenommen und die Lösung mit verdünnter Schwefelsäure und gesättigter Natriumchloridlösung gewaschen. Die organische Phase wird über Natriumsulfat getrocknet und eingeengt. Die 2 - (5 - Trifluoracetylaminomethyl - 2 - thienyl) - 2 - syn - methoximinoessigsäure wird als Schaum isoliert (ca. 70% syn-Isomeres). IR-Spektrum (Nujol): Absorptionsbanden bei 3,2; 5,67; 5,82; 6,36 μ; DS: $Rf_{52A}$ = 0,41.

d) Eine Lösung von 31 g (100 mMol) 2 - (5 - Trifluoracetylaminomethyl - 2 - thienyl) - 2 - syn-methoximinoessigsäure in 150 ml Wasser und 200 ml Dioxan wird mit 27 ml (202 mMol) 7,5 N Natronlauge bei Raumtemperatur während 1 Stunde gerührt. Die Lösung wird auf pH 6 gestellt und im Vakuum auf 30 ml eingeengt. Das ausfallende Zwitterion der 2 - (5 - Aminomethyl - 2 - thienyl)-2 - syn - methoximinoessigsäure wird abfiltriert und mit Aceton gewaschen. IR-Spektrum (Nujol): Absorptionsbanden bei 3,25; 6,12; 6,29; 6,52 μ; DS: $Rf_{52A}$ = 0,1.

e) Eine Lösung von 560 mg (1,5 mMol) (2R) - N - BOC - Serin - diphenylmethylester (hergestellt aus (2R) - N - BOC - Serin und Diphenyldiazomethan) in 10 ml Methylenchlorid wird auf 0° gekühlt und nacheinander mit 0,12 ml (1,5 mMol) Pyridin und 0,72 ml Phosgen (20%-ig in Toluol) versetzt. Die Lösung lässt man bei 0° unter Stickstoff während 1 Stunde Rühren (Lösung I).

Eine Suspension von 250 mg (1,25 mMol) 2 - (5 - Aminomethyl - 2 - thienyl) - 2 - syn-methoximinoessigsäure wird mit 0,93 ml Bis - trimethylsilylacetamid (3,75 mMol) versetzt und 3/4 Stunden bei Raumtemperatur unter Stickstoff gerührt (Lösung II).

Lösung II wird nun zu Lösung I pipettiert und nach Zufügen von 0,2 ml (1,4 mMol) Triäthylamin $1\frac{1}{2}$ Stunden gerührt. Nach Schütteln mit 2 N Schwefelsäure und Kochsalzlösung wird die organische Phase über Natriumsulfat getrocknet und im Vakuum eingeengt. Der ölige Rückstand wird an Silicagel (System Methylenchlorid/Aethylacetat 9:1) chromatographiert.

Die 2 - [5 - ((2R) - 2 - BOC - Amino - 2 - diphenylmethoxycarbonyläthoxycarbonylaminomethyl) - 2 - thienyl] - 2 - syn - methoximinoessigsäure wird als Schaum isoliert. IR-Spektrum (in $CHCl_3$): Absorptionsbanden bei 2,94; 5,78; 5,84; 5,95; 6,15; 6,66 μ. DS:$Rf_{52A}$ = 0,53.

f) Eine Lösung von 10 g (16,0 mMol) 2 - [5 - ((2R) - 2 - BOC - Amino - 2 - diphenylmethoxycarbonyläthoxycarbonylaminomethyl) - 2 - thienyl] - 2 - syn - methoximinoessigsäure in 50 ml Methylenchlorid wird auf —20° gekühlt, mit 1,87 ml (16,8 mMol) N - Methylmorpholin und 1,9 ml (14 mMol) Chlorameisensäureisobutylester versetzt. Nach 1½ stündiger Reaktionsdauer bei —15° werden 6,92 g 7β - Amino - 3 - acetylmethyl - 3 - cephem - 4 - carbonsäure - diphenylmethylester zugefügt und die Lösung auf 0° gebracht. Nach 3 Stunden wird die Reaktionsmischung nacheinander mit 2N Schwefelsäure, verdünnter wässriger Natriumbicarbonatlösung und konzentrierter, wässriger Natriumchloridlösung gewaschen, über Natriumsulfat getrocknet, filtriert und eingedampft. Durch Chromatographie des Rückstandes an Silicagel (System Toluol/Aethylacetat 3:1) wird der 7β - {2 - [5-((2R) - 2 - BOC - Amino - 2 - diphenylmethoxycarbonyläthoxycarbonylaminomethyl) - 2 - thienyl]-2 - syn - methoximinoacetylamino} - 3 - acetoxymethyl - 3 - cephem - 4 - carbonsäure - diphenylmethylester rein isoliert. IR - Spektrum ($CHCl_3$): Absorptionsbanden bei 2,92; 5,61; 5,74; 5,84; 6,66 μ; DS:Rf = 0,41 (Toluol/Aethylacetat 1:1).

Beispiel 8

a) Eine Suspension von 0,58 g 7β - {2 - [4 - ((2R) - 2 - BOC - Amino - 2 - diphenylmethoxycarbonyläthoxycarbonylaminosulfonylamino)phenyl]acetylamino} - 3 - carbamoyloxymethyl-3 - cephem - 4 - carbonsäurediphenylmethylester und 0,15 ml Anisol in 3 ml Methylenchlorid wird bei 0° mit 3 ml Trifluoressigsäure versetzt und 45 Minuten bei 0° gerührt. Durch Zugabe von 17 ml Diäthyläther-Hexan 1:1 wird das Trifluoracetat des Endproduktes gefällt, das nach 10 Minuten bei 0° abfiltriert und in 15 ml Wasser gelöst wird. Die wässrige Lösung wird dreimal mit je 20 ml Aethylacetat gewaschen, mit 1 N Natronlauge auf pH 4,6 gestellt, mit Aceton verdünnt und im Vakuum eingeengt. Das ausfallende 7β - {2 - [4 - ((2R) - 2 - Amino - 2- carboxyäthoxycarbonylaminosulfonylamino)-phenyl]acetylamino} - 3 - carbamoyloxymethyl - 3 - cephem - 4 - carbonsäure - natriumsalz wird abfiltriert und im Vakuum getrocknet; $[\alpha]_D^{20} = +79° + 1°$ (c = 3; Wasser).

Das Ausgangsmaterial wird wie folgt hergestellt:

b) Zu einer Lösung von 0,69 g Chlorsulfonylisocyanat in 1,3 ml Methylenchlorid wird bei 20° eine Lösung von 1,94 g (2R) - N - BOC - Serin - diphenylmethylester und 0,69 g Pyridin in 10 ml Methylenchlorid getropft. Nach 20 Minuten Rühren bei 40° wird die klare Lösung auf 0° abgekühlt (Lösung A).

Eine Suspension von 0,75 g Aminophenylessigsäure in 2,91 g Pyridin wird mit 1,29 g Trimethylchlorsilan versetzt, wobei eine Klare Lösung entsteht und die Temperatur auf 38° ansteigt. Nach 20 Minuten Rühren bei 40—45° wird auf 0° gekühlt (Lösung B).

Bei 0° wird lösung B zu Lösung A getropft und anschliessend 30 Minuten bei 0° und 1 Stunde bei +22° weitergerührt. Das Gemisch wird unter Rühren auf 80 ml 0,2-m Dikaliumhydrogenphosphatlösung gegossen, im Vakuum vom Methylenchlorid befreit und die Verbleibende wässrige Phase bei pH2 in der Kälte mit Aethylacetat extrahiert. Durch Eindampfen der organischen Phase wird die 2 - [4 - ((2R)-2 - BOC - Amino - 2 - diphenylmethoxycarbonyläthoxycarbonylaminosulfonylamino)phenyl]-essigsäure in Form eines Schaumes erhalten.

c) Eine Lösung von 2,08 g 2-[4 - ((2R) - 2 - BOC - Amino - 2 - diphenylmethoxycarbonyläthoxycarbonylaminosulfonylamino)phenyl]essigsäure und 0,32 g N-Methylmorpholin in 28 ml Methylenchlorid wird bei 0° mit 0,43 g Chlorameisensäureisobutylester versetzt und anschliessend 1 Stunde bei 0° gerührt. Diese Lösung des entstehenden gemischten Anhydrids wird bei 0° zu einer Lösung von 1,62 g 7β-Amonio-3-carbamoyloxymethyl-3-cephem-4-carbonsäure-di-phenylmethylester-p-tosylat und 0,34 g N-Methylmorpholin in 14 ml Methylenchlorid getropft. Nach 1½ Stunden Rühren bei 0° und 1 Stunde bei +22° wird das Gemisch auf 100 ml 0,3-m Kaliumdihydrogenphosphatlösung gerührt und im Vakuum von organischen Lösungsmitteln befreit. Die verbleibende wässrige Phase wird bei 0° und pH 2,0 mit Aethylacetat extrahiert. Die organische Phase wird mit 0,5-m Dikaliumhydrogenphosphatlösung gewaschen, mit Natriumsulfat getrocknet, im Vakuum eingedampft und ergibt den 7β - {2 - [4 - ((2R) - 2 - BOC - Amino - 2 - diphenylmethoxycarbonyläthoxycarbonylaminosulfonylamino)phenyl]acetylamino} - 3 - carbamoyloxymethyl - 3 - cephem - 4 - carbonsäurediphenylmethylester.

Beispiel 9

a) Eine Lösung von 10,5 g 7β - {(2R,S) - 2 - [4 - ((2R) - 2 - BOC - Amino - 2 - tert. - butoxycarbonyläthoxycarbonylamino)phenyl] - 2 - (2,2,2 - trichloräthoxycarbonyloxy)acetylamino} - 3 - carbamoyloxymethyl-3-cephem-4-carbonsäure - diphenylmethylester in 100 ml Acetonitril und 100 ml Eisessig wird im Eisbad abgekühlt und unter starkem Rühren bei +3° während 2 Stunden portionenweise mit insgesamt 20,5 g Zinkpulver in gleichen Zeitabständen versetzt. Anschliessend wird die Suspension weitere 3 Stunden im Eisbad gerührt, dann durch Celit abgenutscht und das Nutschgut mit viel Aethylacetat gewaschen. Das Filtrat wird sukzessive mit viel Wasser, 1N Natriumhydrogencarbonat und Wasser gewaschen. Die Aethylacetatlösung wird über Natriumsulfat getrocknet, das Lösungsmittel am Rotationsverdampfer bei 45° abgedampft und der Rückstand durch Chromatographieren an der 25-fachen Menge Kieselgel gereinigt. Die Fraktionen mit Methylenchlorid-Methylacetat-(7:3) als Eluiermittel werden vereinigt und der 7β - {(2,R,S) - 2 - [4 - ((2R) - 2 - BOC - Amino - 2 - tert. - butoxycarbonyläthoxycarbonylamino)phenyl] - 2 - hydroxyacetylamino} - 3 - carbamoyloxymethyl - 3 - cephem - 4 - carbonsäure - diphenylmethylester aus

Aethylacetatlösung mit einem Gemisch von Petroläther und Diäthyläther gefällt. DS (Fliessmittel: Chloroform-Aethylacetat-Aethanol-(5:5:0,1)): Rf = 0,20 und 0,32 (Diastereomerengemisch).

Ein Gemisch von 2,70 g 7$\beta$ - {(2R,S) - 2 - [4 - ((2R) - 2 - BOC - Amino - 2 - tert. - butoxycarbonyläthoxycarbonylamino)phenyl] - 2 - hydroxyacetylamino} - 3 - carbamoyloxymethyl - 3 - cephem - 4 - carbonsäure - diphenylmethylester, 2,70 ml Methylenchlorid, 1,70 ml Anisol und 27,0 ml Trifluoressigsäure wird 1 Stunde bei Raumtemperatur unter Ausschluss von Luftfeuchtigkeit gerührt. Darauf wird die Suspension auf ein eiskaltes Gemisch von Petroläther (500 ml) und Diäthyläther (250 ml) gegossen, das anfallende Trifluoracetat abgenutscht, mit Diäthyläther gewaschen und im Hochvakuum bei Raumtemperatur gerocknet. Das erhaltene Trifluoracetat der 7$\beta$ - {(2R,S) - 2 - [4 - ((2R) - 2 - Amino - 2 - carboxyäthoxycarbonylamino)phenyl] - 2 - hydroxyacetylamino} - 3 - carbamoyloxymethyl - 3 - cephem - 4 - carbonsäure wird analog Beispiel 1a) in das entsprechende Natriumsalz übergeführt. F. ab 220° (Zersetzung). DS: $Rf_{101}$ = 0,24; $Rf_{101A}$ = 0,17.

Das Ausgangsmaterial kann wie folgt hergestellt werden:

b) 16 g (R,S)-4-Amino-mandelsäure werden in 80 ml Wasser suspendiert, unter Rühren durch Zutropfen von 2N Natronlauge ein pH-Wert von 7,5 eingestellt und die Lösung mittels Eisbad auf +3 bis +5° abgekühlt. Darauf wird eine Lösung von 22,2 g Chlorameisensäure-2,2,2-trichloräthylester in 50 ml Tetrahydrofuran unter starkem Rühren und Kühlen innert 20 Minuten bei +5°zugetropft und durch Zutropfen von 2N Natronlauge der pH-Wert des Reaktionsgemisches zwischen 7—8 gehalten. Anschliessend wird das Gemisch 1 Stunde bei +5° gerührt, dann mit 100 ml Wasser verdünnt und zweimal mit Aethylacetat ausgezogen. Die wässrige Phase wird abgetrennt, mit Aethylacetat überschichtet, im Eisbad abgekühlt, unter Rühren durch Zugabe von verdünnter Salzsäure-(1:1) sauer gestellt (pH 2) und zweimal mit Aethylacetat ausgezogen. Die organischen Extrakte werden vereinigt, einmal mit Solelösung gewaschen, über Natriumsulfat getrocknet und das Lösungsmittel am Rotationsverdampfer bei 50° abgedampft. Die (R,S)-4-(2,2,2-Trichloräthoxycarbonylamino)-mandelsäure wird aus Aethylacetat-Petroläther umkristallisiert. F. 202—205° (Zersetzung).

c) In ein Lösung von 64 g (R,S)-4-(2,2,2-Trichloräthoxycarbonylamino)mandelsäure in 140 ml N,N-Dimethylformamid und 38,4 ml Triäthylamin werden innert 2 Stunden unter Rühren bei Raumtemperatur 27 ml Benzylbromid getropft. Das Reaktionsgemisch wird 18 Stunden bei Raumtemperatur gerührt, die Suspension auf viel Wasser gegossen und zweimal mit einem Gemisch von Aethylacetat-Diäthyläther-(1:1) ausgezogen. Die organischen Extrakte werden vereingt, zweimal mit Solelösung gewaschen und über Natriumsulfat getrocknet. Nach Abdampfen des Lösungsmittels wird das Produkt mit Petroläther digeriert und der (R,S)-4-(2,2,2-Trichloräthoxycarbonylamino)-mandelsäurebenzylester kristallin erhalten. F. 72—76° (Zersetzung).

d) Eine Lösung von 20 g (R,S)-4-(2,2,2-Trichloräthoxycarbonylamino)mandelsäure-benzylester in 200 ml Eisessig wird unter Rühren bei Raumtemperatur während 3 Stunden portionenweise mit insgesamt 21 g Zinkpulver in gleichen Zeitabständen versetzt. Anschliessend wird die Suspension weitere 2 Stunden bei Raumtemperatur gerührt, dann durch Celit abgenutscht, das Nutschgut mit Aethylacetat gewaschen und das Filtrat am Rotationsverdampfer bei 50° stark eingeengt. Der Rückstand wird auf Wasser gegossen, mit Aethylacetat überschichtet, durch Zugabe von festem Natriumhydrogencarbonat leicht alkalisch gestellt (pH 7,5—8,0) und zweimal mit Aethylacetat ausgezogen. Die organischen Extrakte werden vereinigt, sukzessive mit Solelösung, 1N Natriumhydrogencarbonat und Solelösung gewaschen, über Natriumsulfat getrocknet und das Lösungsmittel am Rotationsverdampfer bei 45° abgedampft. Der erhaltene (R,S)-4-Amino-mandelsäure-benzylester hat den Schmelzpunkt 88—92° (Zersetzung).

e) Eine Lösung von 2,50 ml Pyridin in 10 ml Methylenchlorid wird unter Rühren und Kühlen innert 4 Minuten bei 0° bis +5° in ein Gemisch von 16 ml Phosgenlösung (20% in Toluol) und 10 ml Methylenchlorid getropft. Darauf wird eine Lösung von 8,0 g N-BOC-(R)-Serin-tert.-butylester in 40 ml Methylenchlorid innert 15 Minuten in die obige Suspension getropft und anschliessend das Reaktionsgemisch 1 Stunde bei 0° bis +5° gerührt. Nach Zugabe einer Lösung von 7,8 g (R,S)-4-Amino-mandelsäure-benzylester in 80 ml Methylenchlorid und 2,7 ml Pyridin innert 3 Minuten bei 0° wird die Suspension 30 Minuten bei 0° und 30 Minuten bei Raumtemperatur gerührt, darauf mit 400 ml Diäthyläther verdünnt und sukzessive mit Wasser, Zitronensäurelösung (5%), Wasser, 0,1 N Salzsäure und Wasser gewaschen. Nach Trocken und Abdampfen des Lösungsmittels wird der ölige (R,S)-4-((2R)-2-BOC-Amino-2-tert.-butoxycarbonyläthoxycarbonylamino)mandelsäure-benzylester durch Verreiben mit Petroläther zum Kristallisieren gebracht. F. 98—100°; $[\alpha]_D = -17 \pm 1°$ (c = 2,83 in Chloroform); DS (Fliessmittel:Toluol-Chloroform-Aethylacetat-(1:1:1)): Rf = 0,46.

f) Eine Lösung von 20,0 g (R,S)-4-((2R)-2-BOC-Amino-2-tert.-butoxycarbonyläthoxycarbonylamino)mandelsäure-benzylester in 40 ml Tetrahydrofuran und 40 ml Pyridin wird im Eisbad abgekühlt und unter Rühren innert 20 Minuten bei 0 bis +5° eine Lösung von 6,90 ml Chlorameisensäure-2,2,2-trichloräthylester in 40 ml Tetrahydrofuran zugetropft. Das Reaktionsgemisch wird 3 Stunden bei 0° gerührt, mit Diäthyläther verdünnt und die organische Phase sukzessive mit Wasser, Zitronensäurelösung (15%), Wasser, 1N Natriumhydrogencarbonat und Wasser gewaschen. Die Lösung wird über Natriumsulfat getrocknet und das Lösungsmittel am Rotationsverdampfer bei 45° abgedampft. Durch Vermischen mit Petroläther wird der ($\alpha$R,S) - 4 - ((2R) - 2 - BOC - Amino - 2 - tert. - butoxycarbonyläthoxycarbonylamino) - $\alpha$ - (2,2,2 - trichloräthoxycarbonyloxy)phenylessigsäure - benzylester fest. F. 85—90° (Zersetzung). $[\alpha] = -15° \pm 1°$ (c = 3,02 in Chloroform); DS (Fliessmittel: Toluol-Chloroform-Aethylacetat-(4,5:4,5:1)): Rf = 0,42.

g) Eine Lösung von 18,0 g (αR,S) - 4 - ((2R) - 2 - BOC - Amino - 2 - tert. - butoxycarbonyläthoxycarbonylamino) - α - (2,2,2 - trichloräthoxycarbonyloxy) - phenylessigsäure - benzylester in 250 ml Aethylacetat wird in Gegenwart von 3,0 g Palladium auf Aktivkohle (10%) hydriert. Nach ca. 2 Stunden ist die berechnete Wasserstoffmenge aufgenommen. Der Katalysator wird abfiltriert, mit Aethylacetat gewaschen, das Filtrate am Rotationsverdampfer bei 45° eingeengt und die (αR,S) - 4 - ((2R) - 2 - BOC - Amino - 2 - tert. - butoxycarbonyläthoxycarbonylamino) - α - (2,2,2-trichloräthoxycarbonyloxy)phenylessigsäure durch Zugabe von Petroläther gefällt. F. 94—100° (Zersetzung); $[\alpha]_D = -18° \pm 1°$ (c = 0,93 in Chloroform).

h) Eine Lösung von 7,25 g 7β-Amino-3-carbamoyloxymethyl-3-cephem-4-carbonsäure-diphenylmethylester und 10,0 g (αR,S) - 4 - ((2R) - 2 - BOC - Amino - 2 - tert. - butoxycarbonyläthoxycarbonylamino) - α - (2,2,2 - trichloräthoxycarbonyloxy)phenylessigsäure in 125 ml Tetrahydrofuran wird mit einer Lösung von 3,75 g N,N'-Dicyclohexylcarbodiimid in 25 ml Tetrahydrofuran und später mit einer zweiten Portion von 1,75 g festem N,N'-Dicyclohexylcarbodiimid nach dem Verfahren von Beispiel 1 umgesetzt. Das Rohprokukt wird durch Chromatographieren an der 25-fachen Menge Kieselgel gereinigt. Die Fraktionen mit Methylenchlorid-Methylacetat-(17:3) als Eluiermittel werden vereinigt und der 7β - {(2R,S) - 2 - [4 - ((2R) - 2 - BOC - Amino - 2 - tert. - butoxycarbonyläthoxycarbonylamino)phenyl] - 2 - (2,2,2 - trichloräthoxycarbonyloxy)acetylamino} - 3 - carbamoyloxymethyl - 3 - cephem - 4 - carbonsäure - diphenylmethylester aus Aethylacetatlösung mit einem Gemisch von Petroläther und Diäthyläther gefällt. F. 138—145° (Zersetzung). DS (Fliessmittel: Toluol-Aethylacetat-Aethanol-(5:5:0,1): Rf = 0,54.

## Beispiel 10

a) Ein Gemisch von 2,0 g 7β - {(2R) - 2 - [4 - ((2R) - 2 - BOC - Amino - 2 - diphenylmethoxycarbonyläthoxycarbonylamino)phenyl] - 2 - hydroxyacetylamino} - 3 - [(1 - methyl - 1H - tetrazol - 5 - yl) - thiomethyl] - 3 - cephem - 4 - carbonsäure - diphenylmethylester, 2,0 ml Methylenchlorid, 1,2 ml Anisol und 16 ml eiskalter Trifluoressigsäure wird 15 Minuten bei Raumtemperatur unter Ausschluss der Luftfeuchtigkeit gerührt. Aus der anfänglich klaren Lösung fällt ein voluminöser Niederschlag aus. Anschliessend wird die Suspension auf ein eiskaltes Gemisch von Petroläther (400 ml) und Diäthyläther (200 ml) gegossen, das anfallende Trifluoracetat der 7β - {(2R) - 2 - [4 - ((2R) - 2 - Amino - 2 - carboxyäthoxycarbonylamino)phenyl] - 2 - hydroxyacetylamino} - 3 - [(1 - methyl - 1H - tetrazol - 5 - yl) - thiomethyl] - 3 - cephem - 4 - carbonsäure abgenutscht, mit Diäthyläther gewaschen und im Hockvakuum bei Raumtemperatur getrocknet. Das entprechende Natriumsalz wird aus dem Trifluoracetat nach dem Verfahren von Beispiel 1a) erhalten. f. ab 165° (Zersetzung); $[\alpha]_D = -3° \pm 1°$ (c = 1,83 in 0,1 N Natriumhydrogencarbonat); DS: $Rf_{101} = 0{,}30$; $Rf_{101A} = 0{,}23$; $Rf_{52A} = 0{,}07$.

Das Ausgangsmaterial kann wie folgt hergestellt werden:

b) Eine Lösung von 5,70 g Pyridin in 10 ml Methylenchlorid wird unter Rühren und Kühlen bei 0° bis +3° in 105 ml Phosgenlösung (20% in Toluol) getropft und das Reaktionsgemisch 10 Minuten bei 0° gerührt. Nach Zutrpfen einer Lösung von 25,0 g (R)-N-BOC-Serin-diphenylmethylester innert 20 Minuten bei 0°, wird das Gemisch 1 Stunde im Eisbad gerührt und darauf mit Methylenchlorid verdünnt. Die Methylenchloridlösung wird dreimal mit eiskaltem Wasser gewaschen, über Natriumsulfat getrocknet und das Lösungsmittel am Rotationsverdampfer bei 45° abgedampft. Beim Verreiben mit Petroläther wird das erhaltene (2R)-2-BOC-Amino-2-diphenylmethoxycarbonyläthoxycarbonylchlorid fest. F. 79—80° (Zersetzung).

c) 8,86 g (R)-4-Amino-mandelsäure werden in einem Gemisch von 90 ml Acetonitril und 20 ml Tetrahydrofuran aufgeschlämmt, auf 0° abgekühlt und innert 75 Minuten unter Rühren bei 0° portionenweise mit insgesamt 25 ml N,O-Bis-(trimethylsilyl)-acetamid versetzt, wobei eine klare Lösung entsteht. Diese Lösung wird auf —15° abgekühlt und unter Rühren mit 4,2 ml Pyridin anschliessend mit einer Lösung von 23 g (2R)-2-BOC-Amino-2-diphenylmethoxycarbonyläthoxycarbonylchlorid in einem Gemisch von 90 ml Acetonitril und 90 ml Tetrahydrofuran versetzt. Nach 2,5 Stunden Rühren im Eisbad wird das Gemisch mit Aethylacetat verdünnt, dreimal mit Wasser gewaschen, über Natriumsulfat getrocknet und das Lösungsmittel am Rotationsverdampfer bei 45° abdestilliert. Bei Zugabe von Petroläther wird die (R)-4-((2R)-2-BOC-Amino-2-diphenylmethoxycarbonyläthoxycarbonylamino)mandelsäure fest. F. 65—71°; $[\alpha]_D = -36° \pm 1°$ (c = 2,93 in Dimethylsulfoxid >; DS (Fliessmittel: Chloroform-Aethylacetat-Eisessig-(5:5:0,2)): Rf = 0,23.

d) Eine Lösung von 7,55 g 7β - Amino - 3 - [(1 - methyl - 1H - tetrazol - 5 - yl) - thiomethyl] - 3 - cephem - 4 - carbonsäurediphenylmethylester, 8.50 g (R) - 4 - ((2R) - 2 - BOC - Amino - 2 - diphenylmethoxycarbonyläthoxycarbonylamino)mandelsäure und 2,85 g 1-Hydroxybenzotriazol (angefeuchtet mit 10% Wasser) in einem Gemisch von 30 ml N,N-Dimethylformamid und 45 ml Tetrahydrofuran wird mit einem Eisbad auf 0° bis +3° abgekühlt, unter Kühlen und Rühren innert 30 Minuten eine Lösung von 1,70 g N,N'-Dicyclohexylcarbodiimid in 20 ml Tetrahydrofuran zugetropft und das Reaktionsgemisch im Eisbad wietergerührt. Nach 35 Stunden wird eine weitere Lösung von 1,50 g N,N'-Dicyclohexylcarbodiimid in 20 ml Tetrahydrofuran zugetropft. Nach insgesamt 7,5 Stunden bei 0° wird die Suspension abgenutscht, das Nutschgut mit Aethylacetat gewaschen, das Filtrat mit viel Aethylacetat verdünnt, die Aethylacetlösung sukzessive mit Wasser, 1N Natriumhydrogencarbonat und Wasser gewaschen. Das Rohprodukt wird durch Chromatographieren an der 25-fachen Menge Kieselgel gereinigt. Die Fraktionen mit Chloroform-Methylacetat-(14:1) als Eluiermittel werden vereinigt und der 7$\beta$ - {(2R) - 2 - [4 - ((2R) - 2 - BOC - Amino - 2 - diphenylmethoxycarbonyläthoxycarbonylamino)phenyl] - 2 - hydroxyacetylamino} - 3 - [(1 - methyl - 1H - tetrazol - 5 - yl) - thiomethyl] - 3 - cephem - 4 - carbonsäure - diphenylmethylester aus Aethylacetat kristallisiert. F. 169—172° (Zersetzung). DS (Fliessmittel: Chloroform-Aethylacetat-Aethanol-(5:5:0,2)): Rf = 0,57. $[\alpha]_D = -52 \pm 1°$ (c = 1,75 in Dimethylsulfoxid).

Beispiel 11

a) Ein Gemisch von 2,0 g 7$\beta$ - {(2R) - 2 - [4 - ((2R) - 2 - BOC - Amino - 2 - diphenylmethoxycarbonyläthoxycarbonylamino)phenyl] - 2 - hydroxyacetylamino} - 3 - carbamoyloxymethyl - 3 - cephem - 4 - carbonsäure - diphenylmethylester, 2,0 ml Methyllenchlorid, 1,20 ml Anisol und 16,0 ml eiskalter Trifluoressigsäure wird 15 Minuten bei Raumtemperatur unter Ausschluss der Luftfeuchtigkeit gerührt. Darauf wird die Suspension auf ein eiskaltes Gemisch von Petroläther (400 ml) und Diäthyläther (200 ml) gegossen, das anfallende Trifluoracetat der 7$\beta$ - {(2R) - 2 - [4 - ((2R) - 2 - Amino - 2 - carboxyäthoxycarbonylamino)phenyl] - 2 - hydroxyacetylamino} - 3 - carbamoyloxymethyl - 3 - cephem - 4 - carbonsäure abgenutscht, mit Diäthyläther gewaschen und im Hochvakuum bei Raumtemperatur getrocknet. Das entsprechende Natriumsalz wird aus dem Trifluoracetat nach dem Verfahren von Beispiel 1a) erhalten. F. ab 230° (Zersetzung); $[\alpha]_D = +62 \pm 1°$ (c = 1,93 in 0,1 N Natriumhydrogencarbonat); DS: $Rf_{52A}$ = 0,06; $R_{101}$ = 0,32; $Rf_{101A}$ = 0,24.

Das Ausgangsmaterial kann wie folgt hergestellt werden:

b) Eine Lösung von 8,0 g 7$\beta$-Amino-3-carbamoyloxymethyl-3-cephem-4-carbonsäure-diphenylmethylester, 10,0 g (R)-4-((2R)-2-BOC-Amino-2-diphenylmethoxycarbonyläthoxycarbonylamino)-mandelsäure und 3,40 g 1-Hydroxybenzotriazol in 80 ml Tetrahydrofuran wird auf +3° abgekühlt, dann unter Rühren und Kühlen innert 30 Minuten eine Lösung von 4,0 g 'N,N-Dicyclohexylcarbodiimid in 20 ml Tetrahydrofuran zugetropft und das Reaktionsgemisch 7 Stunden im Eisbad gerührt. Anschliessend wird die Suspension nach dem Verfahren von Beispiel 17d aufgearbeitet. Das Rohprodukt wird durch Chromatographieren and der 30-fachen Menge Kieselgel gereinigt. Die Fraktionen mit Chloroform-Methylacetat-(4:1) als Eluiermittel werden vereinigt und das Produkt aus Chloroform kristallisiert. Der 7$\beta$ - {(2R) - 2 - [4 - ((2R) - 2 - BOC - Amino - 2 - diphenylmethoxycarbonyläthoxycarbonylamino)phenyl] - 2 - hydroxyacetylamino} - 3 - carbamoyloxymethyl - 3 - cephem - 4 - carbonsäure - diphenylmethylester schmilzt bei 129—133° unter Zersetzung; $[\alpha]_D = +22 \pm 1°$ (c = 3,41 in Dimethylsulfoxid); DS (Fliessmittel: Chloroform-Aethylacetat-Aethanol-(5:5:0,1)): Rf = 0,32.

Beispiel 12

a) Eine Suspension von 3,0 g 7$\beta$ - {(2R,S) - 2 - [4 - ((2R) - 2 - Amino - 2 - diphenylmethoxycarbonyläthoxycarbonylamino)phenyl] - 2 - sulfoacetylamino} - 3 - [(1 - methyl - 1H - tetrazol - 5 - yl) - thiomethyl] - 3 - cephem - 4 - carbonsäure - diphenylmethylester in 25 ml Methylenchlorid und 1,0 ml Anisol wird auf 0° abgekühlt, mit 4,65 ml eiskalter Trifluoressigsäure versetzt und 40 Minuten im Eisbad unter Ausschluss der Luftfeuchtigkeit gerührt. Aus der Anfänglich klaren Lösung fällt ein Schmieriger Niederschlag aus. Nach dem Ablauf der Reaktionszeit wird die Schmiere durch Zugabe von 10 ml eiskalter Trifluoressigsäure rasch in Lösung gebracht, dann auf ein eiskaltes Gemisch von Petroläther (500 ml) und Diäthyläther (250 ml) gegossen. Das Anfallende Trifluoracetat wird abgenutscht, mit Diäthyläther gewaschen und im Hochvakuum bei Raumtemperatur getrocknet. Das 7$\beta$ - {(2R,S) - 2 - [4 - ((2R) - 2 - Amino - 2 - carboxyäthoxycarbonylamino)phenyl] - 2 - sulfoacetylamino} - 3 - [(1 - methyl - 1H - tetrazol - 5 - yl) - thiomethyl] - 3 - cephem - 4 - carbonsäure - dinatriumsalz wird aus dem Trifluoracetat nach dem Verfahren von Beispiel 1a) erhalten. F. ab 205° (Zersetzung). DS: $Rf_{101}$ = 0,23; $Rf_{101A}$ = 0,14.

Das Ausgangsmaterial kann wie folgt hergestellt werden:

b) Eine Lösung von 88 g ($\alpha$R,S)-4-Nitro-$\alpha$-sulfophenylessigsäure in 430 ml Wasser wird in Gegenwart von 12,5 g Palladium auf Aktivkohle (10%) hydriert. Nach ca. 12 Stunden kommt die Wasserstoffaufnahme zum Stillstand. Der Katalysator wird abfiltriert, mit wenig Wasser gewaschen, das Filtrat im Eisbad abgekühlt, unter Rühren und Kühlen durch Zutropfen von konzentriertfer Salzsäure sauer gestellt (pH 1,2) und die Suspension einige Stunden im Eisbad stehengelassen. Die anfallende ($\alpha$R,S)-4-Amino-$\alpha$-sulfophenylessigsäure wird abgenutscht und mit eiskaltem Wasser gewaschen. F. über 300° (Zersetzung). DS: $Rf_{52A}$ = 0,03.

c) 17,5 g ($\alpha$R,S)-4-Amino-$\alpha$-sulfo-phenylessigsäure werden in einem Gemisch von 120 ml Methylenchlorid und 40 ml Acetonitril aufgeschlämmt und unter Rühren innert 75 Minuten portionenweise mit insgesamt 60 ml N,O-Bis-(trimethylsilyl)-acetamid versetzt. Die erhaltene klare Lösung wird noch 1 Stunde bei Raumtemperatur gerührt, auf 0° abgekühlt und nacheinander mit 6,10 ml Pyridin und einer Lösung von 34 g (2R)-2-BOC-Amino-2-diphenylmethoxycarbonyläthoxycarbonylchlorid in 200 ml Methylenchlorid versetzt. Nach 1 Stunde Rühren im Eisbad und 1,5 Stunden bei Raumtemperatur wird das Reaktionsgemisch in 4 Liter Aethylacetat gegossen, sukzessive mit 0,1 N Salzsäure, Wasser und Solelösung gewaschen, über Natriumsulfat getrocknet und das Lösungsmittel am Rotationsverdampfer bei 45° abgedampft. Beim Vermischen mit Petroläther wird die (αR,S) - 4 - ((2R) - 2 - BOC - Amino - 2 - diphenylmethoxycarbonyläthoxycarbonylamino) - α - sulfo - phenylessigsäure fest.F. ab 160° (Zersetzung). DS: $Rf_{52A} = 0,42$; $Rf_{67} = 0,31$.

Eine Suspension von 26,5 g (αR,S) - 4 - ((2R) - 2 - BOC - Amino - 2 - diphenylmethoxycarbonyläthoxycarbonylamino) - α - sulfo - phenylssigsäure und 40 g Toluol-4-sulfonsäuremonohydrat in 430 ml Acetonitril wird 5 Stunden bei Raumtemperatur gerührt, dann mit 250 ml Wasser versetzt und das Acetonitril am Rotationsverdampfer bei 50° abgedampft. Das angefallene Produkt wird abgenutscht und mit Wasser gewaschen. Die (αR,S) - 4 - ((2R) - 2 - Amino - 2 - diphenylmethoxycarbonyläthoxycarbonylamino) - α - sulfo - phenylessigsäure wird durch Lösen in Wasser bei pH 6,8 (Zugabe von 2N Natronlauge), Filtrieren und Ansäuern der klaren Lösung mit 2N Salzsäure (pH 1,5) gereinigt. F. ab 190° (Zersetzung): $[\alpha]_D = +11 \pm 1°$ (c = 2,55 in Dimethylsulfoxid); DS: $Rf_{52A} = 0,23$.

d) Die folgende Reaktion wird in einer Stickstoffatmosphäre ausgeführt. Eine Lösung von 5,1 g 7β - Amino - 3 - [(1 - methyl - 1H - tetrazol - 5 - yl) - thiomethyl] - 3 - cephem - 4 - carbonsäure - diphenylmethylester und 5,0 g (αR,S) - 4 - ((2R) - 2 - Amino - 2 - diphenylmethoxycarbonyläthoxycarbonylamino) - α - sulfo - phenylessigsäure in einem Gemisch von 270 ml Tetrahydrofuran und 30 ml Wasser wird mit 2,6 g N,N'-Dicyclohexylcarbodiimid versetzt und das Reaktionsgemisch 8 Stunden bei Raumtemperatur gerührt. Darauf wird die Suspension abgenutscht, das Filtrat mit einem Gemisch von 1,4 Liter Petroläther und 0,7 Liter Diäthyläther verrührt und das anfallende Produkt abgenuscht. Das Nutschgut wird in einem Gemisch von Aethanol (150 ml)-Diäthyläther (150 ml)-Petroläther (150 ml) aufgeschlämmt, abgenutscht und im Hockvakuum bei Raumtemperatur getrocknet. Das Rohprodukt wird mit 250 ml Methylenchlorid gerührt, der unlösliche Teil abgetrennt, die klare Lösung am Rotationsverdampfer bei 45° abgedampft und der zurückbleibende Schaum an der 25-fachen Menge Kieselgel chromatographiert. Die Fraktionen mit Methylenchlorid-Trifluoräthanol-Wasser-7:4:0,2) werden vereinigt. Man erhält daraus den 7β - {(2R,S) - 2 - [4 - ((2R) - 2 - Amino - 2 - diphenylmethoxycarbonylamino)phenyl] - 2 - sulfoacetylamino} - 3 - [(1-methyl - 1H - tetrazol - 5 - yl) - thiomethyl] - 3 - cephem - 4 - carbonsäure - diphenylmethylester. F. 173—175° unter Zersetzung. DS (Fliessmittel: Methylacetat-Aethanol-(4:1)): Rf = 0,42.

Beispiel 13

a) Eine auf 0° gekühlte Lösung von 5,4 g (5,3 mMol) 7β - {(2R) - 2 - BOC - Amino - 2 - [3 - ((2R) - 2 - BOC - amino - 2 - diphenylmethoxycarbonyläthoxycarbonylamino)phenyl] - acetylamino} - 3 - methoxy - 3 - cephem - 4 - carbonsäure - diphenylmethylester und 5,4 ml Anisol in 30 ml absolutem Methylenchlorid wird mit 25 ml Trifluoressigsäure versetzt und 30 Minuten unter Feuchtigkeitsausschluss gerührt. Nach Zugabe von 250 ml Petroläther-Diäthyläther 1:1 wird der gebildete Niederschlag abfiltriert, mit Diäthyläther gewaschen und getrocknet. Die wässrige Lösung (35 ml) des rohen Ditrifluoracetates wird mit Aethylacetat (3 x 20 ml) extrahiert, mit 2N-Natronlauge auf pH 5 gestellt und tropfenweise mit 130 ml Isopropanol versetzt. Die erhaltene 7β - {(2R) - 2 - Amino - 2 - [3 - (2R) - 2 - amino - 2 - carboxyäthoxycarbonylamino)phenyl]acetylamino} - 3 - methoxy - 3 - cephem - 4 - carbonsäure wird aus 140 ml Wasser-Isopropanol 1:3 umkristallisiert, zur Entfernung von Isopropanol zweimal mit wenig Wasser abgezogen und am Hochvakuum getrocknet. F. 170° Zersetzung. DS ($Rf_{96} = 0,1$; $[\alpha]_D = 12 \pm 1°$ (c = 1,17 in 0,1 N HCl).

Das Ausgangsmaterial kann wie folgt hergestellt werden:

b) Eine auf 0° gekühlte Lösung von 4,45 g (12 mMol) (2R) - N - BOC - Serin - diphenylmethylester und 1,0 ml absolutem Pyridin in 100 ml absolutem Methylenchlorid wird unter Rühren und Feuchtigkeitsausschluss mit 6,2 ml 20%-igem Phosgen in Toluol versetzt und 3 Stunden bei Raumtemperatur gerührt (Lösung A).

3,2 g (12 mMol) (2R)-2-BOC-Amino-2-(3-amino)phenyl-essigsäure gelöst in 100 ml absolutem Methylenchlorid werden unter Stickstoff mit 3,2 ml Bis(trimethylsilyl)-acetamid versetzt und eine Stunde bei Raumtemperatur gerührt (Lösung B).

Bei 0° wird die Lösung B innert 5 Minuten zur Lösung A getropft, anschliessend mit 1,3 ml N-Methylmorpholin versetzt und 30 Minuten bei 0° und 3 Stunden bei 25° gerührt. Die Lösung wird mit Eiswasser und gesättigter Natriumchloridlösung gewaschen, über Natriumsulfat getrocknet und am Rotationsverdampfer eingeengt. Das Rohprodukt wird an Kieselgel mit Diäthyläther als Eluiermittel gereinigt, woraus (2R) - 2 - BOC - Amino - 2 - [3 - ((2R) - 2 - BOC - amino - 2 - diphenylmethoxycarbonyläthoxycarbonylamino)phenyl] - essigsäure als farbloses amorphes Pulver isoliert wird DS: $Rf_{96} = 0,9$.

c) Ein auf —20° gekühlte Lösung von 3,4 g (5,1 mMol) (2R) - 2 - BOC - Amino - 2 - [3 - ((2R) - 2 - BOC - amino - 2 - diphenylmethoxycarbonyläthoxy-carbonylamino)phenyl]essigsäure und 0,59 ml (5,1 m mol) N-Methylmorpholin in 150 ml absolutem Methylenchlorid wird unter Feuchtigkeitsausschluss und Rühren mit 0,67 ml (5,1 mMol) Chlorameisensäure-isobutylester versetzt. Zum so erhältlichen gemischten Anhydrid werden in einer Portion 2,21 g (5, 1 mMol) 7β - Amino - 3 - methoxy - 3 - cephem - 4 - carbonsäure - diphenylmethylester - hydrochlorid, dann 0,57 ml N-Methylmorpholin bei —10° gegeben. Nach 2 Stunden Reaktionszeit bei 0° wird die Lösung mit Aethylacetat (500 ml) verdünnt, mit Eiswasser und gesättigter Natriumchloridlösung gewaschen, über Natriumsulfat getrocknet und am Rotationsverdampfer eingeengt. Der Rückstand wird an Kieselgel mit Diäthyläther als Eluiermittel gereinigt, woraus der dünnschichtchromatographisch einheitliche 7$\beta$ - {(2R) - 2 - BOC - Amino - 2 - [3 - ((2R) - 2 - BOC - amino - 2 - diphenylmethoxycarbonyläthoxycarbonylamino)phenyl]acetylamino} - 3 - methoxy - 3 - cephem - 4 - carbonsäure - diphenylmethylester als amorphes Produkt isoliert wird. DS (Fliessmittel: Aethylacetat): Rf = 0,60 Infrarotspektrum in Methylenchlorid: characteristische Banden bei 3380, 1778, 1720, 1680, 1490 und 1160 $cm^{-1}$.

Beispiel 14

a) Ein Gemisch von 8,0 g 7$\beta$ - {2 - [4 - ((4R) - 4 - BOC - Amino - 4 - t.butoxycarbonylbutyrylamino)phenyl]acetylamino} - 3 - [(1 - methyl - 1H - tetrazol - 5 - yl) - thiomethyl] - 3 - cephem - 4 - carbonsäure - diphenylmethylester, 3,0 ml Anisol, 8,0 ml Methylenchlorid und 80 ml Trifluoressigsäure wird 35 Minuten bei Raumtemperatur unter Ausschluss von Luftfeuchtigkeit gerührt. Die erhaltene Suspension wird auf ein eiskaltes Gemisch von Petroläther (1 Liter) und Diäthyläther (500 ml) gegossen, das anfallende Trifluoracetat abgenutscht, mit Diäthyläther gewaschen und bei Raumtemperatur im Hochvakuum getrocknet.

Das 7$\beta$ - {2 - [4 - ((4R) - 4 - Amino - 4 - carboxybutylrylamino) - phenyl]acetylamino} - 3 - [(1 - methyl - 1H - tetrazol - 5 - yl) - thiomethyl] - 3 - cephem - 4 - carbonsäure - natriumsalz wird aus dem Trifluoracetat nach dem Verfahren von Beispiel 1 erhalten. F. ab 200° (Zersetzung). DS: $Rf_{52A} = 0,06$, $RF_{101} = 0,28$, $Rf_{101A} = 0,28$.

Das Ausgangsmaterial kann wie folgt hergestellt werden:

b) Ein Gemisch von 10,5 g (2R)-Glutaminsäure-$\gamma$-benzylester (hergestellt nach dem Verfahren von R.L. Prestidge et al, J.O.C. ***40***, 3287 (1975)), 90 ml Dioxan, 30 ml Wasser und 13 ml Di-tert.-butyldicarbonat wird bei Raumtemperatur gerührt und der pH-Wert des Reaktionsgemisches durch Zutropfen von 1N Natronlauge zwischen 7 und 7.6 gehalten. Nach 2 Stunden wird die klare Lösung am Rotationsverdampfer bei 50° auf ca. 50 ml eingeengt und mit Aethylacetat ausgezogen. Die wässrige Phase wird abgetrennt mit 20%-iger Zitronensäurelösung angesäuert (pH 3) und dreimal mit Aethylacetat ausgezogen. Die organischen Extrakte werden vereinigt, einmal mit Solelösung gewaschen, über Natriumsulfat getrocknet und das Lösungsmittel am Rotationsverdampfer bei 45° abgedampft. Beim Vermischen mit Petroläther und Kühlen wird der (2R)-N-BOC-Glutaminsäure-$\gamma$-benzylester fest. F. 50—58° (Zersetzung). $[\alpha]_D = -13 \pm 1°$ (c = 3,11 in Chloroform).

Der ölige (2R)-N-BOC-Glutaminsäure-($\gamma$-benzylester)-$\alpha$-tert.-butylester wird aus (2R)-N-BOC-Glutaminsäure-$\gamma$-benzylester durch Umsetzung mit O-tert.-Butylisoharnstoff in Methylenchlorid (E. Vowinkel, Chem. Ber. *100*, 16 (1967)) gewonnen. Das Rohprodukt wird durch Chromatographie an der 20-fachen Menge Kieselgel gereinigt. DS (Fliessmittel: Toluol-Aethylacetat-Chloroform-(3:1:1)): Rf = 0,58 (mit Ninhydrin entwickelt); $[\alpha]_D = 8 \pm 1°$ (3,17 in Chloroform).

c) Eine Lösung von 28 g (2R)-N-BOC-Glutaminsäure-($\gamma$-benzylester)-$\alpha$-tert.-butylester in 250 ml Aethylacetat wird in Gegenwart von 4,0 g Palladium auf Aktivkohle (10%) hydriert. Nach ca 4 Stunden ist die berechnete Wasserstoffmenge aufgenommen. Der Katalysator wird abfiltriert, mit Aethylacetat gewaschen, das Filtrat am Rotationsverdampfer bei 45° eingeengt und der (2R)-N-BOC-Glutaminsäure-$\alpha$-tert.-butylester durch Zugabe von Petroläther kristallisiert. F. 109—112° (Zersetzung). $[\alpha]_D = -5 + 1°$ (c = 2,66 in Chloroform).

d) Eine Lösung von 9,0 g (2R)-N-BOC-Glutaminsäure-$\alpha$-tert.-butylester und 7,50 g 4-Aminophenylessigsäurebenzylester in 80 ml Tetrahydrofuran wird mit einem Eisbad auf +5° abgekühlt, eine Lösung von 6,50 g N,N'-Dicyclohexylcarbodiimid in 20 ml Tetrahydrofuran unter Rühren und Kühlen innert 20 Minuten zugetropft, dann das Eisbad entfernt und das Reaktionsgemisch bei Raumtemperatur gerührt. Nach 3 Stunden Reaktionszeit wird das Reaktionsgemisch mit 3,0 g festem N,N'-Dicyclohexylcarbodiimid versetzt. Nach insgesamt 7 Stunden Rühren wird die Suspension abgenutscht, das Nutschgut mit Aethylacetat gewaschen, das Filtrat mit viel Aethylacetat versetzt und das Tetrahydrofuran durch Einengen am Rotationsverdampfer bei 45° entfernt. Die erhaltene Lösung wird mit Aethylacetat verdünnt und sukzessive mit Wasser, 1N Natriumbicarbonatlösung und Wasser gewaschen. Die organische Phase wird über Natriumsulfat getrocknet und am Rotationsverdampfer bei 45° eingedampft. Das Rohprodukt wird durch Filtieren an der 10-fachen Menge Kieselgel gereinigt. Die Fraktionen mit Methylenchlorid-Methylacetat-(10:0,5) als Eluiermittel werden vereinigt. Der erhaltene 4 - ((4R) - 4 - BOC - Amino - 4 - t. - butoxycarbonylbutyrylamino)phenylessigsäure - benzylester wird aus einem Gemisch von Aethylacetat-Petroläther umkristallisiert F. 101—104° (Zersetzung).

e) Eine Lösung von 20,0 g 4 - ((4R) - 4 - BOC - Amino - 4 - t.butoxycarbonylbutyrylamino)-phenylessigsäure-benzylester in einem Gemisch von Aethylacetat (150 ml) und Aethanol (150 ml) wird in Gegenwart von 5,0 g Palladium auf Aktivkohle (10%) bei Raumtemperatur hydriert. Nach ca. 5 Stunden ist die berechnete Wasserstoffmenge aufgenommen. Der Katalysator wird abfiltriert, mit einem Gemisch von Aethylacetat-Aethanol-(1:1) gewaschen und das Filtrat am Rotationsverdampfer bei 45° eingedampft. Die 4-((4R)-4-BOC-Amino-4-t.butoxycarbonylbutyrylamino)-phenylessigsäure wird durch

Vermischen mit Petroläther gefällt. F. 129—136° (Zersetzung); $[\alpha]_D = +10 \pm 1°$ (c = 3,04, in Methanol).

f) Eine Lösung von 9,5 g 7β - Amino - 3 - [(1-methyl - 1H - tetrazol - 5 - yl) - thiomethyl] - 3 - cephem - 4 - carbonsäurediphenylmethylester und 8,0 g 4 - ((4R) - 4 - BOC - Amino - 4 - t.butoxycarbonylbutyrylamino)phenylessigsäure in einem Gemisch von 50 ml Tetrahydrofuran und 15 ml N,N-Dimethylformamid wird mit einer Lösung von 4,0 g N,N'-Dicyclohexylcarbodiimid in 20 ml Tetrahydrofuran bzw. 2,0 g festem N,N'-Dicyclohexylcarbodiimid nach dem Verfahren von Beispiel 1 umgesetzt und das Reaktionsgemisch aufgearbeitet. Beim Einengen der Aethylacetatlösung fällt der kristalline 7β - {2 - [4 - ((4R) - 4 - BOC - Amino - 4 - t. - butoxycarbonylbutyrylamino)-phenyl]acetamido} - 3 - [(1-methyl - 1H - tetrazol - 5 - yl) - thiomethyl] - 3 - cephem - 4 - carbonsäurediphenylmethylester aus. F. 172—178° (Zersetzung). DS (Fliessmittel: Toluol-Aethylacetat-Methylenchlorid-Aethanol-(16:16:16:1)). Rf = 0,32.

Beispiel 15

Analog den vorstehenden Beispielen können ausgehend von den entsprechenden Verbindungen, worin die Carboxylgruppen und die Aminogruppen in geschützter Form vorliegen, die folgenden Verbindungen hergestellt werden:
(2R,S)-2-[4-((2R)-2-Amino-2-carboxyäthoxycarbonylamino)phenyl]-2-formyloxy-essigsäure;
2-[4-((2R)-2-Amino-2-carboxyäthoxycarbonylamino)phenyl]-2-syn-methoxyimino-essigsäure;
und ihre geschützten Derivate.

**Patentansprüche**

1. Verbindungen der Formel

$$HOOC-\underset{\displaystyle NH_2}{\underset{|}{C}H}-(C_nH_{2n})-X-W-NH-(C_mH_{2m})-A-\overset{\displaystyle Y}{\overset{|}{\underset{\displaystyle Z}{\underset{|}{C}}}}-\overset{\displaystyle O}{\overset{\|}{C}}-OH \qquad (III),$$

worin der Index n eine ganze Zahl von 1 bis 4, der Index m 0 oder 1, X Sauerstoff, Schwefel oder die Gruppe —NH—, W eine Gruppe —CO—, —CO—$NHSO_2$— oder —$SO_2NH$—CO—, der X—W zusammen eine Gruppe —CO— oder —CO—$NHSO_2$—, A gegebenenfalls durch Niederalkyl, Hydroxy, Niederalkoxy und/oder Halogen substituiertes Phenylen, gegebenenfalls durch Niederalkyl, Niederalkoxy und/oder Halogen substituiertes Thienylen oder Furylen, Y Wasserstoff, Hydroxyl, Formyloxy, Amino oder gegebenenfalls in Salzform vorliegendes Sulfo und Z Wasserstoff, oder Y und Z zusammen eine Oxogruppe oder eine Gruppe =N—O—R°, worin R° Wasserstoff oder gegebenenfalls durch Niederalkoxy, Halogen, Hydroxy, acyliertes Hydroxy, Sulfo, Carboxy oder durch Niederalkyl verestertes Carboxy substituiertes Niederalkyl darstellt, und worin die Aminocarbonsäuregruppierung HOOC—CH($NH_2$) und gegebenenfalls in der Gruppierung A—C(Y)(Z)— vorhandene weitere funktionelle Gruppen in geschützter Form vorliegen, und ihre zur Acylierung geeigneten reaktionsfähigen Derivate oder Salze davon.

2. Verbindungen der Formel III, worin die Gruppe —($C_nH_{2n}$)— unverzweigt ist und die Indices n und m die angegebene Bedeutung haben, X Sauerstoff oder die Gruppe —NH—, W eine Gruppe —CO— oder —CO—$NHSO_2$—, oder X—W zusammen eine Gruppe —CO— oder —CO—$NHSO_2$— bedeuten, A p- oder m-Phenylen, 2,5-Thienylen oder 2,5-Furylen darstellt, Y Wasserstoff, Hydroxyl, Amino oder Sulfo und Z Wasserstoff, oder Y und Z zusammen eine Gruppe =N—O—R° bedeuten, worin R° Wasserstoff oder Methyl ist, und worin die Aminocarbonsäuregruppierung HOOC—CH($NH_2$)— und gegebenenfalls in der Gruppierung —A—C(Y)—(Z)— vorhandene weitere funktionelle Gruppen in geschützter Form vorliegen, und ihre zur Acylierung geeigneten reaktionsfähigen Derivate, oder Salze davon, nach Patentanspruch 1.

3. Verbindungen der Formel III, worin die Gruppe —($C_nH_{2n}$)— unverzweigt ist und die Indices n und m die angegebene Bedeutung haben, X Sauerstoff oder die Gruppe —NH— bedeutet, W eine Gruppe —CO— oder —$CONHSO_2$—, oder X—W zusammen eine Gruppe —CO— oder —$CONHSO_2$— bedeuten, A p- oder m-Phenylen, oder, wenn m 1 ist, auch 2,5-Thienylen oder 2,5-Furylen darstellt, Y Wasserstoff, Hydroxyl, Amino oder Sulfo und Z Wasserstoff, oder Y und Z zusammen eine Gruppe =N—O—R° bedeuten, worin R° Wasserstoff oder Methyl ist, und worin die Aminocarbonsäuregruppierung HOOC—CH($NH_2$)— und gegebenenfalls in der Gruppierung —A—C(Y)(Z)— vorhandene weitere funktionelle Gruppen in geschützter Form vorliegen, und ihre zur Acylierung geeigneten reaktionsfähigen Derivate, oder Salze davon, nach Patentanspruch 1.

4. 4-((2R)-2-BOC-Amino-2-t.butoxycarbonyläthoxycarbonylamino)phenylessigsäure, nach Patentanspruch 1.

5. 4-((2S)-2-BOC-Amino-2-t.-butoxycarbonyläthoxycarbonylamino)phenylessigsäure, nach Patentanspruch 1.

6. 4-((2R)-2-BOC-Amino-2-diphenylmethoxycarbonyläthoxycarbonylamino)phenylessigsäure, nach Patentanspruch 1.

7. 4-((5R,S)-5-t.butoxycarbonylpentylaminocarbonylamino)phenylessigsäure, nach Patentanspruch 1.

8. 4-((3R)-3-BOC-Amino-3-t.butoxycarbonylpropionylamino)phenylessigsäure, nach Patentanspruch 1.

9. 2-[5-((2R)-2-BOC-Amino-2-diphenylmethoxycarbonyläthoxycarbonylaminomethyl)-2-furyl]-2-syn-methoxyiminoessigsäure, nach Patentanspruch 1.

10. 2-[5-((2R)-2-BOC-Amino-2-diphenylmethoxycarbonyläthoxycarbonylaminomethyl)-2-thienyl]-2-syn-methoxyiminoessigsäure, nach Patentanspruch 1.

11. 2-[4-((2R)-2-BOC-Amino-2-diphenylmethoxycarbonyläthoxycarbonylaminosulfonylamino)-phenyl]essigsäure, nach Patentanspruch 1.

12. ($\alpha$R,S)-4-((2R)-2-BOC-Amino-2-tert.-butoxycarbonyläthoxycarbonylamino)-$\alpha$-(2,2,2-trichloräthoxycarbonyloxy)phenylessigsäure, nach Patentanspruch 1.

13. (R)-4-((2R)-2-BOC-Amino-2-diphenylmethoxycarbonyläthoxycarbonylamino)mandelsäure, nach Patentanspruch 1.

14. ($\alpha$R,S)-4-((2R)-2-BOC-Amino-2-diphenylmethoxycarbonyläthoxycarbonylamino)-$\alpha$-sulfophenylessigsäure, nach Patentanspruch 1.

15. (2R)-2-BOC-Amino-2-[3-((2R)-2-BOC-amino-2-diphenylmethoxycarbonyläthoxycarbonylamino)phenyl]essigsäure, nach Patentanspruch 1.

16. 4-((4R)-4-BOC-Amino-4-t.-butoxycarbonylbutyrylamino)phenylessigsäure, nach Patentanspruch 1.

17. Verwendung von Verbindungen der Formel III, worin die Aminocarbonsäuregruppierung HOOC—CH($NH_2$)— und gegebenenfalls in der Gruppierung —A—C(Y)(Z)— vorhandene weitere funktionelle Gruppen in geschützter Form vorliegen, und ihrer zur Acylierung geeigneten reaktionsfähigen Derivate, oder Salzen davon, zur Herstellung von antibiotisch wirksamen Acylamido-3-cephem-4-carbonsäure-Verbindungen der Formel

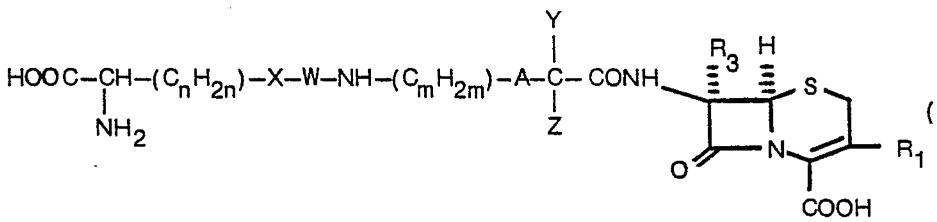

(I)

worin der Index n eine ganze Zahl von 1 bis 4, der Index m 0 oder 1, X Sauerstoff, Schwefel oder die Gruppe —NH—, W eine Gruppe —CO—, —CO—$NHSO_2$— oder —$SO_2NH$—CO—, oder X—W zusammen eine Gruppe —CO— oder —CO—$NHSO_2$—, A gegebenenfalls durch Niederalkyl, Hydroxy, Niederalkoxy und/oder Halogen substituiertes Phenylen, gegebenenfalls durch Niederalkyl, Niederalkoxy und/oder Halogen substituiertes Thienylen oder Furylen, Y Wasserstoff, Hydroxyl, Formyloxy, Amino oder gegebenenfalls in Salzform vorliegendes Sulfo und Z Wasserstoff, oder Y und Z zusammen eine Oxogruppe oder eine Gruppe =N—O—$R^o$, worin $R^o$ Wasserstoff oder gegebenenfalls durch Niederalkoxy, Halogen, Hydroxy, acyliertes Hydroxy, Sulfo, Carboxy oder durch Niederalkyl verestertes Carboxy substituiertes Niederalkyl darstellt, $R_1$ Wasserstoff, $C_1$—$C_4$-Niederalkyl, $C_1$—$C_4$-Niederalkoxy, Halogen oder eine Gruppe der Formel —$CH_2$—$R_2$, worin $R_2$ eine Hydroxygruppe, eine durch eine niederaliphatische Carbonsäure, die Carbaminsäure oder eine am N-Atom durch Niederalkyl, Halogenniederalkyl oder Niederalkanoylniederalkyl substituierte Carbaminsäure veresterte Hydroxy- oder Mercaptogruppe, Niederalkoxy, Niederalkylthio, oder eine durch einen über eion Ringkohlenstoffatom an die Mercaptogruppe gebundenen heterocyclischen Rest mit 1—4 Ringstickstoffatomen und gegebenenfalls einem weiteren Ringheteroatom der Gruppe Sauerstoff und Schwefel, der durch Niederalkyl, Hydroxyniederalkyl, Niederalkanoyloxyniederalkyl, Halogenniederalkyl, Carboxyniederalkyl, Niederalkoxycarbonylniederalkyl, Sulfoniederalkyl, amidiertes Sulfoniederalkyl, Aminoniederalkyl, Mono- oder Diniederalkylaminoniederalkyl, Acylaminoniederalkyl, Cycloalkyl, Aryl, Arylniederalkyl, Heterocyclyl, Halogen, Amino, Mono- oder Diniederalkylamino, Niederalkanoylamino, Halogenniederalkanoylamino, Carboxyniederalkanoylamino, Nitro, Hydroxy, Niederalkoxy, Carboxy, Niederalkoxycarbonyl, Carbamoyl, N-mono- oder N,N-diniederalkyliertes Carbamoyl, Cyan, Oxo oder Oxido ein- oder mehrfach substituiert sein kann, verätherte Mercaptogruppe oder eine quaternäre Ammoniumgruppe darstellt, wobei unter niederen Resten, sofern nicht ausdrücklich anders definiert, solche mit 1 bis 7 C-Atomen zu verstehen sind, und $R_3$ Wasserstoff oder Methoxy bedeuten, worin die Carboxylgruppen gegebenenfalls in physiologisch spaltbarer Form verestert sind, und Salzen davon.

18. Verfahren zur Herstellung der im Anspruch 1 definierten Verbindungen der Formel (III) und ihrer zur Acylierung geeigneten reaktionsfähigen Derivate, oder Salzen davon, dadurch gekennzeichnet, dass man eine Verbindung der Formel

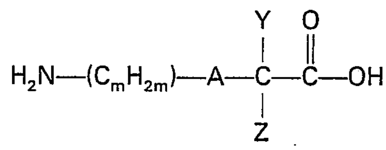

(VIII),

worin die Aminogruppe gegebenenfalls durch eine die Acylierung erlaubende Gruppe substituiert sein kann, und gegebenenfalls in der Gruppierung —A—C(Y)(Z)— vorhandene funktionelle Gruppen geschützt sind, unter intermediärem Schutz der Carboxylgruppe, mit einem reaktionsfähigen funktionellen Derivat einer Säure der Formel

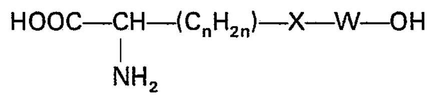

(V),

worin die Aminocarbonsäuregruppierung HOOC—CH($NH_2$)— in geschützter Form vorliegt, oder, wenn X—W zusammen eine Gruppe —CO— bedeuten, auch mit einer entsprechenden freien Säure oder mit einem Salz davon, acyliert und wenn erwünscht, eine erhaltene Verbindung in eine andere Verbindung der Formel III mit entsprechend geschützten funktionellen Gruppen überführt.

**Revendications**

1. Composés de formule:

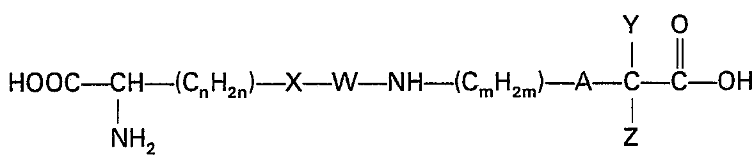

(III)

dans laquelle l'indice n représente un nombre entier de 1 à 4, l'indice m est égal à 0 ou 1, X représente l'oxygène, le soufre ou le groupe —NH—, W représente un groupe —CO, —CO—$NHSO_2$— ou —$SO_2$NH—CO—, ou bien X—W représentent ensemble un groupe —CO— ou —CO—$NHSO_2$—, A représente un groupe phénylène éventuellement substitué par des groupes alkyles inférieurs, hydroxy, alcoxy inférieurs et/ou des halogènes, un groupe thiénylène ou furylène éventuellement substitué par des groupes alkyles inférieurs, alcoxy inférieurs et/ou des halogènes, Y représente l'hydrogène, un groupe hydroxy, formyloxy, amino ou sulfo éventuellement à l'état de sel et Z représente l'hydrogène, ou bien Y et Z forment ensemble un groupe oxo ou un groupe =N—O—R°, dans lequel R° représente l'hydrogène ou un groupe alkyle inférieur éventuellement substitué par un groupe alcoxy inférieur, des halogènes, des groupes hydroxy, hydroxy acylés, sulfo, carboxy ou carboxy estérifié par un groupe alkyle inférieur, et dans laquelle le groupement acide aminocarboxylique HOOC—CH($NH_2$)— et éventuellement les autres groupes fonctionnels présents dans le groupement A—C(Y)(Z)— sont à l'état protégé, et leurs dérivés réactifs appropriés à l'acylation, ou leurs sels.

2. Composés de formule (III), dans laquelle le groupe —($C_nH_{2n}$)— est non ramifié et les indices n est m ont les significations indiquées, X représente l'oxygène ou le groupe —NH—, W représente un groupe —CO— ou —CO—$NHSO_2$—, ou bien X—W forment ensemble un groupe —CO— ou —CO—$NHSO_2$—, A représente un groupe p- ou m-phénylène, 2,5-thiénylène ou 2,5-furylène, Y représente l'hydrogène, un groupe hydroxy, amino ou sulfo et Z représente l'hydrogène, ou bien Y et Z forment ensemble un groupe =N—O—R° dans lequel R° représente l'hydrogène ou le groupe méthyle, et dans lesquels le groupement acide aminocarboxylique HOOC—CH($NH_2$)— et les autres groupes fonctionnels éventuellement présents dans le groupement —A—C(Y)—(Z)— sont à l'état protégé, et leurs dérivés réactifs appropriés à l'acylation, ou leurs sels, selon la revendication 1.

3. Composés de formule III, dans laquelle le groupe —($C_nH_{2n}$)— est non ramifié et les indices n et m ont les significations indiquées, X représente l'oxygène ou le groupe —NH—, W représente un groupe —CO— ou —$CONHSO_2$—, ou bien X—W forment ensemble un groupe —CO— ou —$CONHSO_2$—, A représente un groupe p- ou m-phénylène, ou bien également, lorsque m est égal à 1, un groupe 2,5-thiénylène ou 2,5-furylène, Y représente l'hydrogène, un groupe hydroxy, amino ou sulfo et Z représente l'hydrogène, ou bien Y et Z forment ensemble un groupe =N—O—R° dans lequel R° représente l'hydrogène ou le groupe méthyle, et dans lesquels le groupement acide aminocarboxylique HOOC—CH($NH_2$)— et les autres groupes fonctionnels éventuellement présents dans le groupement —A—C(Y)—(Z)— sont à l'état protégé, et leurs dérivés réactifs appropriés à l'acylation, ou leurs sels, selon la revendication 1.

4. L'acide 4-((2R)-2-BOC-amino-2-tert.-butoxycarbonyléthoxycarbonylamino)-phénylacétique selon la revendication 1.

5. L'acide 4-((2S)-2-BOC-amino-2-tert.-butoxycarbonyléthoxycarbonylamino)-phénylacétique selon la revendication 1.

6. L'acide 4-((2R)-2-BOC-amino-2-diphénylméthoxycarbonyléthoxycarbonylamino)-phénylacétique selon la revendiction 1.

7. L'acide 4-((5R,S)5-tert.-butoxycarbonylpentylaminocarbonylamino)-phénylacétique selon la revendication 1.

8. L'acide 4-((3R)-3-BOC-amino-3-tert.-butoxycarbonylpropionylamino)-phénylacétique selon la revendication 1.

9. L'acide 2-[5-((2R)-2-BOC-amino-2-diphénylméthoxycarbonyléthoxycarbonylaminométhyl)-2-furyl]-2-syn-méthoxyiminoacétique selon la revendication 1.

10. L'acide 2-[5-((2R)-2-BOC-amino-2-diphénylméthoxycarbonyléthoxycarbonylaminométhyl)-2-thiényl]-2-syn-méthoxyiminoacétique selon la revendication 1.

11. L'acide 2-[4-((2R)-2-BOC-amino-2-diphénylméthoxycarbonyléthoxycarbonylaminosulfonylamino)phényl]-acétique selon la revendication 1.

12. L'acide ($\alpha$ R,S)-4)((2R)-2-BOC-amino-2-tert.-butoxycarbonyléthoxycarbonylamino)-$\alpha$-(2,2,2-trichloréthoxycarbonyloxy)-phénylacétique selon la revendication 1.

13. L'acide (R)-4-((2R)-2-BOC-amino-2-diphénylméthoxycarbonyléthoxycarbonylamino)-mandélique selon la revendication 1.

14. L'acide ($\alpha$ R,S)-4-((2R)-2-BOC-amino-2-diphénylméthoxycarbonyléthoxycarbonylamino)-$\alpha$-sulfophénylacétique selon la revendication 1.

15. L'acide (2R)-2-BOC-amino-2-[3-((2R)-2-BOC-amino-2-diphénylméthoxycarbonyléthoxycarbonylamino)-phényl]-acétique selon la revendication 1.

16. L'acide 4-((4R)-4-BOC-amino-4-tert.-butoxycarbonylbutyrylamino)-phénylacétique selon la revendication 1.

17. Utilisation des composés de formule (III), dans laquelle le groupement acide aminocarboxylique HOOC—CH($NH_2$)— et les autres groupes fonctionnels éventuellement présents dans le groupement —A—C(Y)—(Z)— sont à l'état protégé, et de leurs dérivés réactifs appropriés à l'acylation, ou de leurs sels, pour le préparation de dérivés d'acide acylamido-3-céphème-4-carboxyliques possédant une activité antibiotique, de formule:

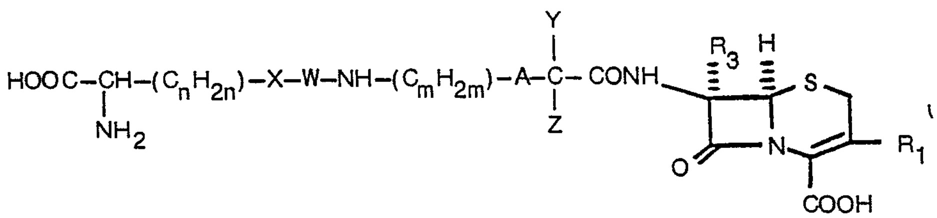

(I)

dans laquelle l'indice n représente un nombre entier de 1 à 4, l'indice m est égal à 0 ou 1, X représente l'oxygène, le soufre ou le groupe —NH—, W représente un groupe —CO—, —CO—$NHSO_2$— ou —$SO_2NH$—CO— ou bien X—W forment ensemble un groupe —CO— ou —CO—$NHSO_2$, A représente un groupe phénylène éventuellement substitué par un groupe alkyle, inférieur, hydroxy, alcoxy inférieur et/ou des halogènes, un groupe thiénylène ou furylène éventuellement substitué par un groupe alkyle inférieur, alcoxy inférieur et/ou des halogènes, Y représente l'hydrogène, un groupe hydroxy, formyloxy, amino ou sulfo éventuellement à l'état de sel et Z représente l'hydrogène ou bien Y et Z forment ensemble un groupe oxo ou un groupe =N—O—$R^o$ dans lequel $R^o$ représente l'hydrogène ou un groupe alkyle inférieur, des halogènes, un groupe hydroxy, hydroxy acylé, sulfo, carboxy ou carboxy estérifié par un groupe alkyle inférieur, $R_1$ représente l'hydrogène, un groupe alkyle inférieur en $C_1$—$C_4$, alcoxy inférieur en $C_1$—$C_4$, un halogène, ou un groupe de formule —$CH_2$—$R_2$ dans lequel $R_2$ représente un groupe hydroxy, un groupe hydroxy ou mercapto estérifié par un acide carboxylique aliphatique inférieur, l'acide carbamique ou un acide carbamique substitué sur l'atome d'azote par un groupe alkyle inférieur, halogénoalkyle inférieur ou (alcanoyle inférieur) alkyle inférieur, un groupe alcoxy inférieur, alkylthio inférieur ou un reste hétérocyclique relié au groupe mercapto par l'intermédiaire d'un atome de carbone cyclique, contenant de 1 à 4 atomes d'azote cyclique et éventuellement un autre hétéroatome cyclique du groupe formé par l'oxygène ou le soufre, qui peut être substitué une ou plusieurs fois par des groupes alkyles inférieurs, hydroxyalkyles inférieurs, (alcanoyl inférieur)-oxyalkyles inférieurs, halogénoalkyles inférieurs, carboxyalkyles inférieurs, (alcoxy inférieur)-carbonyl-(alkyles inférieurs) sulfoalkyles inférieurs, sulfoalkyles inférieurs amidés, aminoalkyles inférieurs, mono- ou di-(alkyle inférieur)-aminoalkyles inférieurs, acylaminoalkyles inférieurs, cycloalkyles, aryles, arylalkyles, inférieurs, hétérocyclyles, des halogènes, des groupes amino, mono- ou di-(alkyle inférieur)-amino, alcanoylamino inférieurs, halogénoalcanoylamino inférieurs carboxyalcanoylamino inférieurs, nitro, hydroxy, alcoxy inférieurs, carboxy, alcoxycarbonyles inférieurs, carbamoyles, N-mono- ou N,N-di-(alkyle inférieur)-carbamoyles cyano, oxo ou oxydo, un groupe mercapto éthérifié ou un groupe ammonium quaternaire, étant précisé que lorsqu'on parle de restes inférieurs et sauf

indication contraire, il s'agit de restes contenant de 1 à 17 atomes de carbone, et $R_3$ représente l'hydrogène ou un groupe méthoxy, les groupes carboxyles pouvant éventuellement être estérifiés sous une forme hydrolysable dans l'organisme, et leurs sels.

18. Procédé de préparation des composés de formule (III) définis dans la revendication 1 et de leurs dérivés réactifs appropriés à l'acylation, ou de leurs sels, caractérisé en ce que l'on acyle un composé de formule:

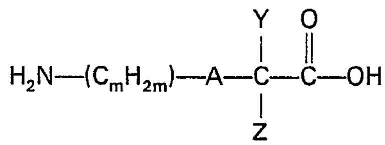

(VIII)

dans laquelle le groupe amino peut éventuellement être substitué par un groupe autorisant l'acylation, et les groupes fonctionnels éventuellement présents dans le groupement —A—C(Y)—(Z) sont protégés, avec protection intermédiaire du groupe carboxyle, à l'aide d'un dérivé fonctionnel réactif d'un acide de formule:

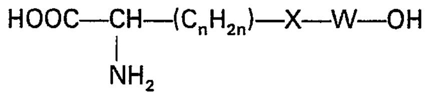

(V)

dans laquelle le groupement acide aminocarboxylique HOOC—CH($NH_2$)— est à l'état protégé, ou bien également, lorsque X—W forment ensemble un groupe —CO—, à l'aide d'un acide libre correspondant ou d'un sel d'un tel acide après quoi, si on le désire, on convertit un composé obtenu en un autre composé de formule (III) dont les groupes fonctionnels correspondants sont protégés.

## Claims

1. Compounds of the formula

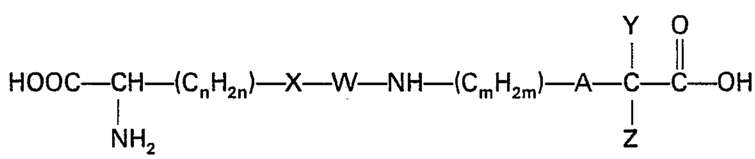

(III),

in which

the index *n* represents an integer of from 1 to 4,

the index *m* represents 0 or 1,

X represents oxygen, sulfur or an —NH— group,

W represents a —CO—, —CO—$NHSO_2$— or —$SO_2$—NH—CO— group, or

X—W together represent a —CO— or —CO—$NHSO_2$— group

A represents phenylene which is unsubstituted or substituted by lower alkyl, hydroxyl, lower alkoxy, and/or halogen, or thienylene or furylene each of which is unsubstituted or substituted by lower alkyl, lower alkoxy and/or halogen,

Y represents hydrogen, hydroxyl, formyloxy, amino or sulfo optionally present in salt form, and

Z represents hydrogen, or

Y and Z together represent an oxo group or an =N—O—R° group in which R° represents hydrogen, or lower alkyl which is unsubstituted or substituted by lower alkoxy, halogen, hydroxyl, acylated hydroxyl, sulfo, carboxyl, or carboxyl esterified by lower alkyl,

and in which the aminocarboxylic acid grouping HOOC—CH($NH_2$)— and further functional groups optionally present in the grouping —A—C(Y)(Z)— are present in protected form, and their reactive derivatives suitable for acylation; or salts thereof.

2. Compounds of the formula (III), wherein the group —($C_2H_{2n}$)— is unbranched, the indices *n* and *m* have the given meanings, X represents oxygen or an —NH— group, W represents a —CO— or —CO—$NHSO_2$— group, or X—W together represent a —CO— or —CO—$NHSO_2$— group, A represents *p*- or *m*-phenylene, 2,5-thienylene or 2,5-furylene, Y represents hydrogen, hydroxyl, amino or sulfo, and Z represents hydrogen, or Y and Z together represent an =N—O—R° group, in which R° is hydrogen or methyl, and in which the aminocarboxylic acid grouping HOOC—CH($NH_2$)— and further functional groups optionally present in the grouping —A—C(Y)(Z)— are present in protected form, and their reactive derivatives suitable for acylation, or salts thereof, according to claim 1.

3. Compounds of the formula (III), in which the —($C_2H_{2n}$)— group is unbranched and the indices *n* and *m* have the given meanings, X represents oxygen or an —NH— group, W represents a —CO— or

—CO—$NHSO_2$ group, or X—W together represent a —CO— or —CO—$NHSO_2$— group, A represents *p* or *m*-phenylene or, when *m* is 1, also 2,5-thienylene or 2,5-furylene, Y represents hydrogen, hydroxyl, amino or sulfo, and Z represents hydrogen, or Y and Z together represent an =N—O—R° group, in which R° is hydrogen or methyl, and in which the aminocarboxylic acid grouping HOOC—CH($NH_2$)— and further functional groups optionally present in the grouping —A—C(Y)(Z)— are present in protected form, and their reactive derivatives suitable for acylation, or salts thereof according to claim 1.

4. 4-((2R)-2-BOC-amino-2-tert-butoxycarbonylethoxycarbonylamino)phenylacetic acid, according to claim 1.

5. 4-((2S)-2-BOC-amino-2-tert-butoxycarbonylethoxycarbonylamino)phenylacetic acid, according to claim 1.

6. 4-((2R)-2-BOC-amino-2-diphenylmethoxycarbonylethoxycarbonylamino)phenylacetic acid, according to claim 1.

7. 4-((5R,S)-5-tert-butoxycarbonylpentylaminocarbonylamino)phenylacetic acid, according to claim 1.

8. 4-((3R)-3-BOC-amino-3-tert-butoxycarbonylpropionylamino)phenylacetic acid, according to claim 1.

9. 2-[5-((2R)-2-BOC-amino-2-diphenylmethoxycarbonylethoxycarbonylaminomethyl)-2-furyl]-2-syn-methoxyiminoacetic acid, according to claim 1.

10. 2-[5-((2R)-2-BOC-amino-2-diphenylmethoxycarbonylethoxycarbonylaminomethyl)-2-thienyl]-2-syn-methoxyiminoacetic acid, according to claim 1.

11. 2-[4-((2R)-2-BOC-amino-2-diphenylmethoxycarbonylethoxycarbonylaminosulfonylamino)-phenyl]acetic acid, according to claim 1.

12. (αR,S)-4-((2R)-2-BOC-amino-2-tert-butoxycarbonylethoxycarbonylamino)-α-(2,2,2-trichloroethoxycarbonyloxy)phenylacetic acid, according to claim 1.

13. (R)-4-((2R)-2-BOC-amino-2-diphenylmethoxycarbonylethoxycarbonylamino)mandelic acid, according to claim 1.

14. (αR,S)-4-((2R)-2-BOC-amino-2-diphenylmethoxycarbonylethoxycarbonylamino)-α-sulfophenylacetic acid, according to claim 1.

15. (2R)-2-BOC-amino-2-[3-((2R)-2-BOC-amino-2-diphenylmethoxycarbonylethoxycarbonylamino)phenyl]acetic acid, according to claim 1.

16. 4-((4R)-4-BOC-amino-4-tert-butoxycarbonylbutyrylamino)-phenylacetic acid, according to claim 1.

17. The use of compounds of the formula III wherein the aminocarboxylic acid grouping HOOC—CH($NH_2$)— and further functional groups optionally present in the grouping —A—C(Y)(Z) are present in protected form, and of their reactive derivatives suitable for acylation, or of salts thereof, for the manufacture of antibiotically active acylamido-3-cephem-4-carboxylic acid compounds of the formula

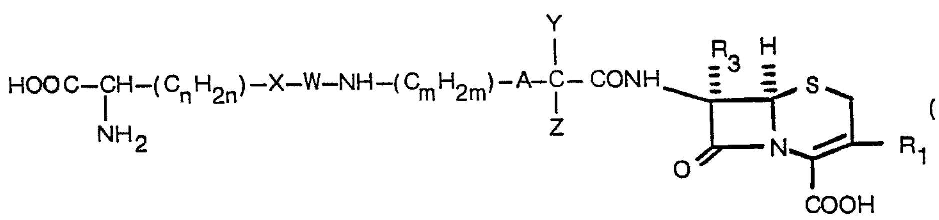

(I)

in which

the index *n* represents an integer of from 1 to 4,

the index *m* represents 0 or 1,

X represents oxygen, sulfur or an —NH— group,

W represents a —CO—, —CO—$NHSO_2$— or —$SO_2NH$—CO— group, or

X—W together represent a —CO— or —CO—$NHSO_2$— group

A represents phenylene which is unsubstituted or substituted by lower alkyl, hydroxyl, lower alkoxy and or halogen, or thienylene or furylene each of which is unsubstituted or substituted by lower alkyl, lower alkoxy and/or halogen,

Y represents hydrogen, hydroxyl, formyloxy, amino or sulfo optionally present in salt form, and

Z represents hydrogen, or

Y and Z together represent an oxo group or an =N—O—R° group in which R° represents hydrogen, or lower alkyl which is unsubstituted or substituted by lower alkoxy, halogen, hydroxyl, acylated hydroxyl, sulfo, carboxyl or carboxyl esterified by lower alkyl,

$R_1$ is hydrogen, $C_1$—$C_4$-lower alkyl, $C_1$—$C_4$-lower alkoxy, halogen or a group of the formula —$CH_2$—$R_2$, wherein

$R_2$ is a hydroxyl group, a hydroxyl or mercapto group esterified by a lower aliphatic carboxylic acid,

carbamic acid or a carbamic acid substituted on the N atom by lower alkyl, halo-lower-alkyl or lower-alkanoyl-lower-alkyl, or it is lower alkoxy, lower alkylthio, or a mercapto group etherified by a heterocyclic radical which is bound by way of a ring carbon atom to the mercapto group and which has 1—4 ring carbon atoms and optionally a further ring hetero atom of the group oxygen and sulfur, which can be mono- or polysubstituted by lower alkyl, hydroxy-lower-alkyl, lower-alkanoyl-lower-alkyl, halo-lower-alkyl, carboxy-lower-alkyl, lower-alkoxy-carbonyl-lower-alkyl, sulfo-lower-alkyl, amidated sulfo-lower-alkyl, amino-lower-alkyl, mono- or di-lower-alkylamino-lower-alkyl, acylamino-lower-alkyl, cycloalkyl, aryl, aryl-lower-alkyl, heterocyclyl, halogen, amino, mono- or di-lower-alkylamino, lower-alkanoylamino, halo-lower-alkanoylamino, carboxy-lower-alkanoylamino, nitro, hydroxyl, lower alkoxy, carboxyl, lower-alkoxy-carbonyl, carbamoyl, N-mono- or N,N-di-lower-alkylated carbamoyl, cyano, oxo or oxido, or a quaternary ammonium group, and by the term "lower" radicals are meant, where otherwise not expressly defined, those having 1—7 C atoms, and

$R_3$ is hydroxy or methoxy,

wherein the carboxyl groups are optionally esterified in a physiologically detachable form, and salts thereof.

18. A process for the manufacture of the compounds of the formula III defined in claim 1, and of their reactive derivatives suitable for acylation, or of salts thereof, characterised in that a compound of the formula

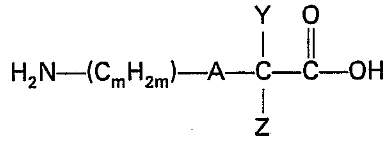

(VIII),

in which the amino group can optionally be substituted by a group allowing acylation, and functional groups optionally present in the grouping —A—C(Y)(Z)— are protected, is, with intermediate protection of the carboxyl group, acylated with a reactive functional derivative of an acid of the formula

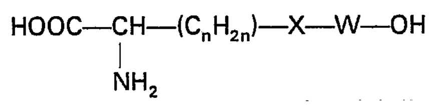

(V),

in which the aminocarboxylic acid grouping HOOC—CH($NH_2$)— is present in protected form; or, if X—W together represent a —CO— group, also with a corresponding free acid or with a salt thereof; and, if desired, a compound obtained is converted into a different compound of the formula III with correspondingly protected functional groups.